# EUROPEAN PATENT SPECIFICATION

(11) **EP 1 986 694 B1**
(45) Date of publication and mention of the grant of the patent: **10.06.2015**
(21) Application number: 07704560.7
(22) Date of filing: 13.02.2007
(51) Int. Cl.: A61K 47/48, C07K 14/61

(54) **COUPLING OF POLYPEPTIDES AT THE C-TERMINUS**
KOPPLUNG VON POLYPEPTIDEN AN DEN C-TERMINUS
COUPLAGE DE POLYPEPTIDES À L'EXTRÉMITÉ C

(30) Priority: 14.02.2006 EP 06101627
(43) Date of publication of application: 05.11.2008
(73) Proprietor: Novo Nordisk Health Care AG, Thurgauerstrasse 36/38 8050 Zürich (CH)
(72) Inventor: PESCHKE, Bernd, DK-2760 Måløv (DK); ZUNDEL, Magali, DK-2870 Dyssegaard (DK)
(74) Representative: Winther, Kamilla
(86) International application number: PCT/EP2007/051388
(87) International publication number: WO 2007/093594

(56) References cited:
- EP-A2- 0 243 929
- WO-A-99/03887
- US-A- 4 468 383
- US-A- 5 225 532
- STEVEN E CWIRLA: "Peptide Agonist of the Thrombopoietin Receptor as Potent as the Natural Cytokine" SCIENCE, vol. 276, 13 June 1997 (1997-06-13), pages 1696-1699, XP002426795
- NICHOLAS C WRIGHTON ET AL.: "Increased potency of an erythropoietin peptide mimetic through covalent dimerization" NATURE BIOTECHNOLOGY, vol. 15, November 1997 (1997-11), pages 1261-1265, XP002426796
- GAO Y ET AL: "Structure-activity relationship of the novel bivalent and C-terminal modified analogues of endomorphin-2" BIOORGANIC & MEDICINAL CHEMISTRY LETTERS, OXFORD, GB, vol. 15, no. 7, 1 April 2005 (2005-04-01), pages 1847-1850, XP004789394 ISSN: 0960-894X cited in the application
- JASON D HUBER ET AL.: "Conjugation of Low Molecular Weight Poly(ethylene glycol) to Biphalin Enhances Antinociceptive Profile" JOURNAL OF PHARMACEUTICAL SCIENCES, vol. 92, no. 7, July 2003 (2003-07), pages 1377-1385, XP002426797
- YOSHIO OKADA ET AL.: "Unique High-Affinity Synthetic mu-Opioid Receptor Agonists with Central- and Systemic-Mediated Analgesia" JOURNAL OF MEDICINAL CHEMISTRY, vol. 46, 17 July 2003 (2003-07-17), pages 3201-3209, XP002426798

## Description

### FIELD OF THE INVENTION

The present invention relates to the field of protein chemistry, in particular to therapeutic polypeptide conjugates.

### BACKGROUND OF THE INVENTION

A range of polypeptides of human origin have been used as therapeutic agents for the treatment of various diseases over the past 80 years. Different modifications of natural polypeptides have also been tested in order to increase potency or to remedy different shortcomings of the natural polypeptide when used as a therapeutic agent, e.g. due to a too high clearance rate. Within the field of therapeutic polypeptides, it has inter alia been investigated whether coupling of two polypeptides to each other may confer beneficial pharmacodynamic or pharmacokinetic effects. Usually these polypeptide conjugates have been dimerized compounds or fusion polypeptides connected by a peptide bond between the C-terminal of the first polypeptide and the N-terminal of the second polypeptide.

Examples of modified or enhanced biological effects of dimerized polypeptides compared to the monomer polypeptides can be found in the literature.

Albrecht et al. (Bioconjugate Chem. 15, 16-26 (2004)) dimerized ScFvs, which were dimerized via a dithio-linkage.

Gao et al. (Bioorg. Med. Chem. Lett. 15, 1847-1850 (2005)) discusses the structure-activity relationship of the novel bivalent and C-terminal modified analogues of endomorphin-2. Allegedly a tenfold higher potency was obtained by C-terminal dimerization of this polypeptide. The C-terminal dimerization was achieved by use of symmetrical diamines to which the polypeptides were coupled. This strategy may be used for synthetically accessible peptides of limited size. However, for larger polypeptides produced in a biological system this strategy would not be useful since a selective coupling of a diamine to the C-terminus would be expected to be very difficult as it would require selective activation of the C-terminal acid.

Stewart (Peptides 25, 527-532 (2004)) reported a pronounced effect of dimerization at the N-terminus of a polypeptide on the biological activity. Whereas the peptide itself did not show any effect on growth inhibition in vivo of a PC-3 cancer cell line, the dimerized version showed a 78% inhibition.

Bifunctional molecules which have been used as spacers for covalent conjugation of two biomolecules have been described by Li et al. (Bioorg. Med. Chem. Lett. 15, 5558-5561 (2005)).

EP 243 929 discloses certain uses of carboxypeptidase to incorporate polypeptides, reporter groups or cytotoxic agents into the C-terminus of polypeptides.

Covalent modification, e.g. by PEGylation or lipid attachment, has been successfully applied on several polypeptide-based pharmaceutics to improve their pharmacokinetic and pharmacodynamic profiles. In practice, however, this approach does always warrant polypeptide compounds with the optimal match of pharmacokinetic and pharmacodynamic profiles, i.e. high activity, prolonged circulatory half-life etc. Also, the methods available for coupling two polypeptides limit the number of polypeptide conjugates which have been synthesized and subjected to preclinical and clinical testing. For instance, many polypeptides which may be useful as therapeutic agents, may exhibit a biological activity which is influenced by modifications in the N-terminus. For such polypeptides it may be difficult to produce polypeptide conjugates as coupling via the N-terminal compromises the desired biological activity. Safety aspects, regulatory legislation and manufacturing costs all demand that the methods for coupling two polypeptides to be used as a pharmaceutical; and in particular as a therapeutic compound, can produce the polypeptide conjugate in a reasonable pure form from the constituent polypeptides.

Thus, there is a need for improved and alternative methods for coupling polypeptides to produce polypeptide conjugates which may be obtained in commercially relevant yields, exhibit high specific activity, prolonged plasma half-life, and/or acceptable physio-chemical properties in relation to manufacturing, handling and formulation. Such methods are provided by, and additional aspects, features, and advantages of the invention described in, the description of the invention provided herein. These aspects are more fully described in, and additional aspects, features, and advantages of the invention will be apparent from, the description of the invention provided herein.

### SUMMARY OF THE INVENTION

It may be advantageous to couple polypeptides together in order to e.g. enhance their biological activity when two polypeptides of the same kind are in close vicinity of each other, to combine two different biological effects when two different polypeptides are coupled together, and/or to enhance pharmacokinetic parameters of at least one of the coupled polypeptides.

The present invention provides, inter alia, a method for coupling two polypeptides together at their respective *C*-terminus comprising modification of the C-termini and reacting the C-termini to form a covalent bond between the two polypeptides.

This method is shown to be useful for producing polypeptide conjugates in high yields and purity, as well as allowing both of the constituent polypeptides to retain intact N-termini.

In one particular the present invention provides a method for coupling two polypeptides together at their respective *C*-terminus comprising
(a) an enzyme catalyzed modification of the C-termini of the polypeptides to be coupled by which chemical groups comprising reactive groups are introduced to the C-termini of the polypeptides to be coupled, and
(b) reacting
   (i) the reactive groups of the two chemical groups with each other or
   (ii) reacting the reactive group of the first chemical group with a first reactive group of a spacer-molecule and reacting the reactive group of the second chemical group with a second reactive group of the spacer-molecule, wherein the spacer between the two polypeptides comprises at least one ethylene glycol diradical.

More specifically, the present invention provides a method comprising
(a) an enzyme catalyzed modification of the C-termini of the polypeptides to be coupled by which a chemical group comprising reactive group W is introduced to the C-terminus of the first polypeptide to be coupled and a chemical group comprising reactive group Z is introduced to the C-terminus of the second polypeptide to be coupled, and
(b) reacting the reactive group Z with a reactive group Y of a spacer-molecule to form a chemical group X and
   reacting the reactive group W with a reactive group V of the spacer-molecule to form a chemical group U.

In one embodiment the enzyme catalyzed modification is performed by the enzyme carboxypeptidase Y.

### DETAILED DESCRIPTION OF THE INVENTION

As mentioned above, the present invention provides a method for coupling two polypeptides together at their respective *C*-terminus comprising
(a) an enzyme catalyzed modification of the C-termini of the polypeptides to be coupled by which chemical groups comprising reactive groups are introduced to the C-termini of the polypeptides to be coupled, and
(b) reacting
   (i) the reactive groups of the two chemical groups with each other or
   (ii) reacting the reactive group of the first chemical group with a first reactive group of a spacer-molecule and reacting the reactive group of the second chemical group with a second reactive group of the spacer-molecule, wherein the spacer between the two polypeptides comprises at least one ethyleneglycol diradical.

More specifically, the present invention provides a method comprising
(a) an enzyme catalyzed modification of the C-termini of the polypeptides to be coupled by which a chemical group comprising reactive group W is introduced to the C-terminus of the first polypeptide to be coupled and a chemical group comprising reactive group Z is introduced to the C-terminus of the second polypeptide to be coupled, and
(b) reacting the reactive group Z with a reactive group Y of a spacer-molecule to form a chemical group X and
   reacting the reactive group W with a reactive group V of the spacer-molecule to form a chemical group U.

The enzyme catalyzed modification of the C-termini of the polypeptides may be performed by a variety of enzymes. In one embodiment the enzyme is carboxypeptidase Y (CPY). In one embodiment the enzyme is a variant or a fragment of carboxypeptidase Y, which variant or fragment retains the ability to catalyse a reaction, by which the C-terminal amino acid of a protein or peptide is replaced by a different chemical moiety. Several variants of carboxypeptidase Y are known in the art; see e.g. WO 98/38285.

The term "polypeptide" as used herein means a compound comprising at least five constituent amino acid residues covalently connected by peptide bonds. In one embodiment, at least one of the polypeptides to be coupled comprises at least one chain comprising at least about 10, such as at least about 15, for instance at least about 20, such as at least about 30, for instance at least about 40, such as at least about 50, for instance at least about 75, such at least about 100 amino acid residues covalently connected to each other by peptide bonds. In one embodiment, both of the polypeptides to be coupled comprise at least one chain comprising at least about 10, such as at least about 15, for instance at least about 20, such as at least about 30, for instance at least about 40, such as at least about 50, for instance at least about 75, such at least about 100 amino acid residues covalently connected to each other by peptide bonds.

The constituent amino acids may be from the group of the amino acids encoded by the genetic code and they may be natural amino acids which are not encoded by the genetic code, as well as synthetic amino acids. Natural amino acids which are not encoded by the genetic code are e.g. hydroxyproline, γ-carboxy-glutamic acid, ornithine, phophoserine, D-alanine, D-glutamic acid. Synthetic amino acids comprise amino acids manufactured by organic synthesis, e.g. D-isomers of the amino acids encoded by the genetic code and Aib (α-aminoisobutyric acid), Abu (α-aminobutyric acid), Tle (tert-butylglycine), and β-alanine. A polypeptide may comprise a single peptide chain or it may comprise more than one polypeptide chain, such as human growth hormone being a single chain and human insulin being two chains connected by disulphide bonds. The choice of which of the polypeptides is to be seen as the "first polypeptide" (or polypeptide A) and which is to be seen as the "second polypeptide" (or polypeptide B) is of course arbitrary, and does therefore not propose any limitation on the scope of the present invention.

A reactive group Z may be introduced at the C-terminus of polypeptide A via a CPY-mediated reaction. This reactive group may be reacted with a reactive group Y of the spacer-molecule to form a chemical group X, coupling spacer-molecule LL covalently to a polypeptide A. A reactive group W may be introduced at the C-terminus of polypeptide B via a CPY-mediated reaction. This reactive group may be reacted with a second reactive group V on the spacer-molecule to form a chemical group U, coupling spacer-molecule LL covalently to a polypeptide B.

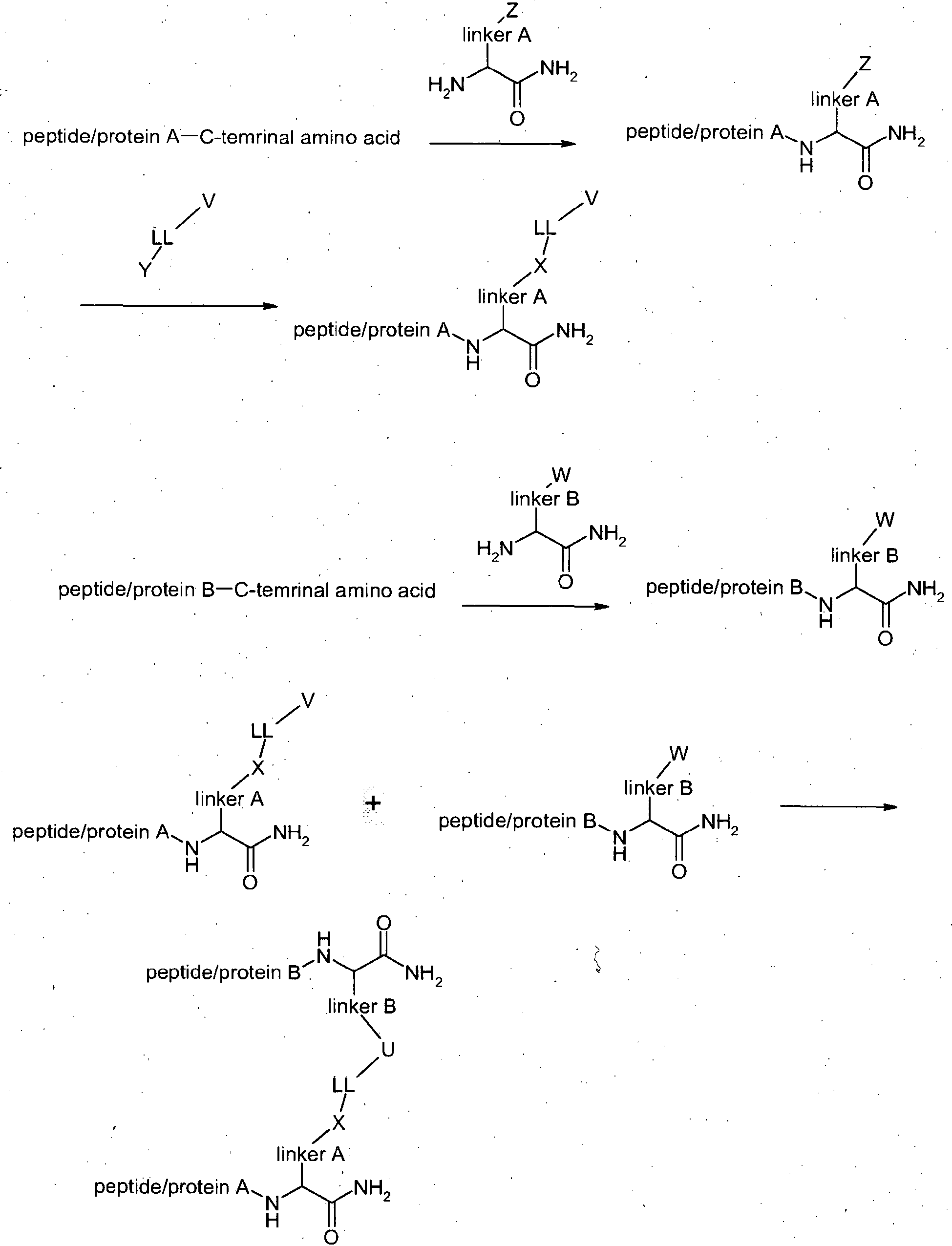

X and U are independently selected from a group consisting of diradicals of oxime and triazole,

The spacer between the two polypeptides comprises at least one ethyleneglycol diradical.

V, W, Y and Z are independently selected from the group consisting of alkoxylamino, oxo, azido and alkynyl

According to the present invention, the two polypeptides being coupled are from each other.

In this invention, the two polypeptides are different from each other. This opens a whole new field of therapeutic polypeptides since the tissue distribution of the two coupled polypeptides will be the same. Hereby, one of the polypeptides comprised by the coupled polypeptide compound can serve to determine the tissue distribution, whereas the other polypeptide can exhibit a certain biological activity which is desirable in the relevant tissue. Also, one of the polypeptides may serve as a protractor molecule enhancing the plasma half-life of the coupled polypeptide compound. Another possibility is that one of the polypeptides serves to protect another labile polypeptide so as to prevent proteolytic degradation of the labile polypeptide.

In one embodiment at least one of the polypeptides is human growth hormone (hGH), a derivative of hGH, a variant of hGH, or a derivative of a hGH variant.

The term "variant" as used herein refers either to a naturally occurring variation of a given polypeptide or a recombinantly prepared or otherwise modified variation of a given peptide or protein in which one or more amino acid residues have been modified by amino acid substitution, addition, deletion, insertion or invertion.

The term "derivative" as used herein refers to a polypeptide or variant or fragment thereof which is modified, i. e., by covalent attachment of any type of molecule, preferably having bioactivity, to the parent polypeptide. Typical modifications are amides, carbohydrates, alkyl groups, acyl groups, esters, PEGylations and the like.

In one embodiment at least one of the polypeptides is hGH-Leu-Ala.

In one embodiment the chemical group Z is introduced to the polypeptide via a linker A. In one embodiment the linker A is selected from the group consisting of bi-radicals of straight, branched and/or cyclic C₁₋₁₀alkane, C₂₋₁₀alkene, C₂₋₁₀alkyne, C₁₋₁₀heteroalkane, C₂₋₁₀heteroalkene, and C₂₋₁₀heteroalkyne, which is optionally substituted with oxo, wherein one or more homocyclic aromatic compound biradical(s) or heterocyclic aromatic compound biradical(s) may be inserted.

For the purpose of this specification, the term "cyclic" in relations to radicals and other chemical groups or molecules encompasses mixed cyclic/linear molecules, that is molecules with both a linear part and a cyclic part.

In one embodiment the chemical group W is introduced to the polypeptide via a linker B. In one embodiment the linker B are selected from the group consisting of bi-radicals of straight, branched and/or cyclic C₁₋₁₀alkane, C₂₋₁₀alkene, C₂₋₁₀alkyne, C₁₋₁₀heteroalkane, C₂₋₁₀heteroalkenb, C₂₋₁₀heteroalkyne, which is optionally substituted with oxo, wherein one or more homocyclic aromatic compound biradical(s) or heterocyclic aromatic compound biradical(s) may be inserted.

The term "C₁₋₁₀alkane" is intended to encompass linear or branched saturated hydrocarbon residues having from 1 to 10 carbon atoms. Particular examples are methane, ethane, n-propane, isopropane, n-butane, isobutane, sec-butane, tert-butane, n-pentane, isopentane, n-octane, etc.

The term "C₁₋₁₀alkyl" is intended to encompass a monoradical of a C₁₋₁₀alkane.

The term"C₂₋₁₀alkene" is intended to encompass olefinically unsaturated branched or straight hydrocarbon groups having from 2 to 10 carbon atoms and at least one double bond. Examples of such groups include, but are not limited to, ethylene, 1-propene, 2-propene, isopropene, 1, 3-butadiene, 1-butene, hexene, pentene and the like.

The term "C₂₋₁₀alkenyl" is intended to encompass a monoradical of a C₂₋₁₀alkene.

The term "C₂₋₁₀alkyne" is intended to encompass unsaturated branched or straight hydrocarbon groups having from 2 to 10 carbon atoms and at least one triple bond. Examples of such groups include, but are not limited to, 1-propyne, 2-propyne 1-butyn, 2-butyne, 1-pentyne, 2-pentyne and the like.

The term "C₂₋₁₀alkynyl" is intended to encompass a monoradical of a C₂₋₁₀alkyne.

The term "heteroarene" is intended to encompass a compound comprising a 5-6 membered monocyclic aromatic system or a 9-10 membered bicyclic aromatic system containing one or more heteroatoms (such as for instance nitrogen, oxygen, phosphorous or sulfur), such as furan, thiophene, pyrrole, imidazole, pyrazole, triazole, pyridine, pyrazine, pyrimidine, pyridazine, isothiazole, isoxazole, oxazole, oxadiazole, thiadiazole, quinoline, isoquinoline, quinazoline, quinoxaline, indole, benzimidazole, benzofuran, pteridine and purine and the like.

The term "heteroaryl" is intended to encompass a monoradical of a heteroarene.

The term "C₁₋₁₀heteroalkane" is intended to encompass straight or branched saturated carbon chains containing from 1 to 10 carbon atoms wherein one or more carbon atoms are substituted by a heteroatom (such as for instance nitrogen, oxygen, phosphorous or sulfur). Examples of such heteroalkanes are for instance

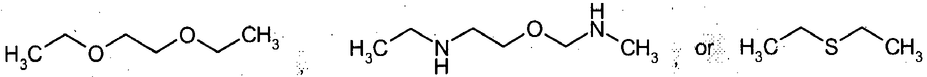

The term "C₁₋₁₀heteroalkyl" is intended to encompass a monoradical of a C₁₋₁₀heteroalkane.

The term "C₂₋₁₀heteroalkene" is intended to encompass olefinically unsaturated branched or straight hydrocarbon groups having from 2 to 10 carbon atoms and at least one double bond, wherein one or more carbon atoms are substituted by a heteroatom (such as for instance nitrogen, oxygen, phosphorous or sulfur), such as

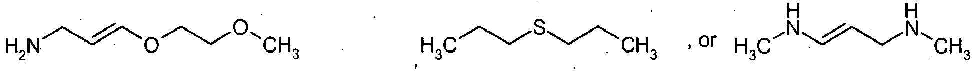

The term "C₂₋₁₀heteroalkenyl" is intended to encompass a monoradical of a C₂₋₁₀heteroalkene.

The terms "C₂₋₁₀heteroalkyne" is intended to encompass unsaturated branched or straight hydrocarbon groups having from 2 to 10 carbon atoms and at least one triple bond, wherein one or more carbon atoms are substituted by a heteroatom (such as for instance nitrogen, oxygen, phosphorous or sulfur), such as

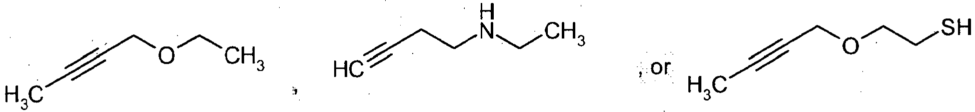

The term "C₂₋₁₀heteroalkynyl" is intended to encompass a monoradical of a C₂₋₁₀heteroalkyne.

In one embodiment of the present invention the linker A and the linker B are independently selected from the group consisting of 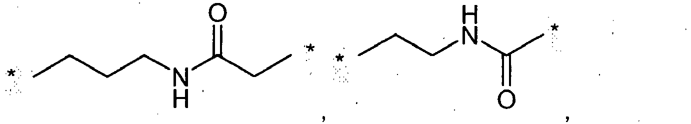 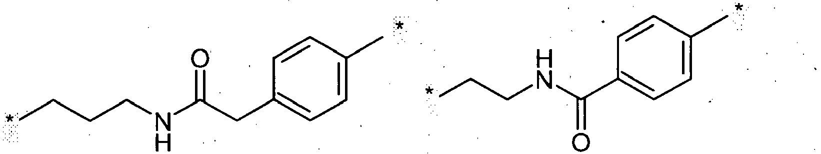 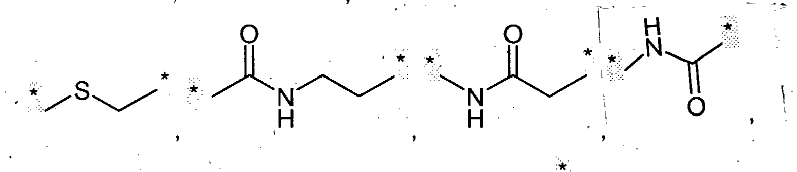 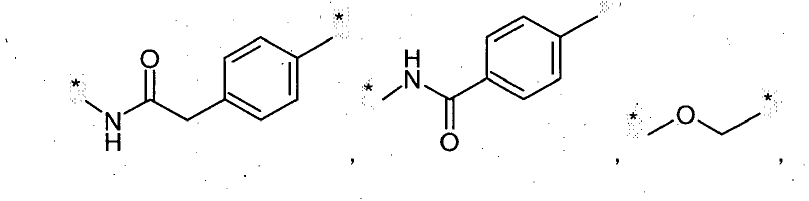 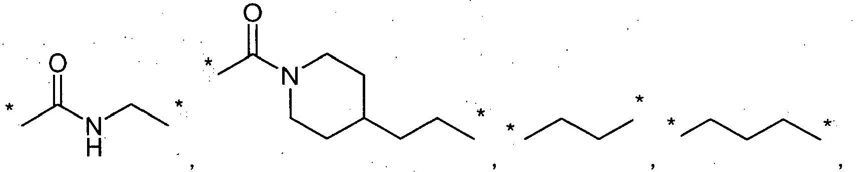 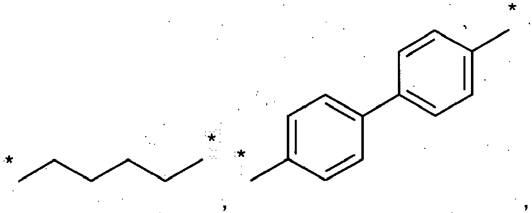 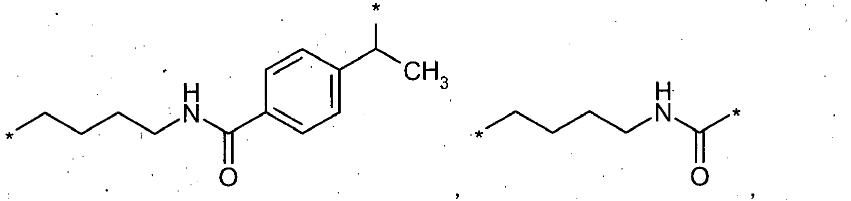 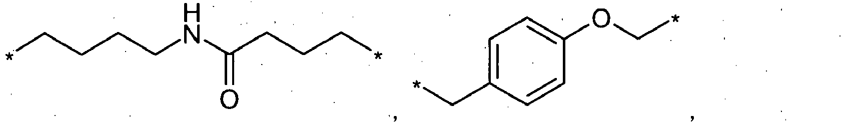 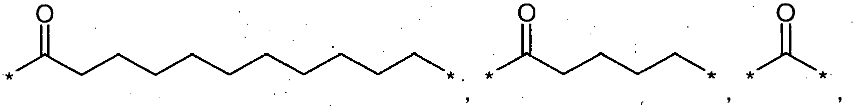 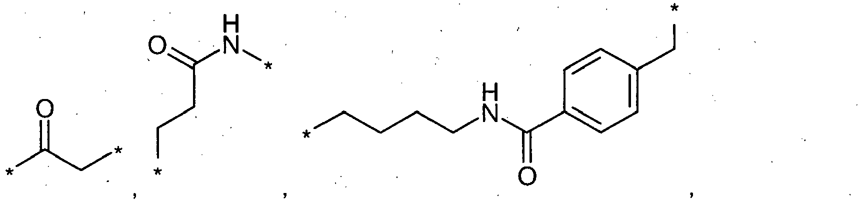 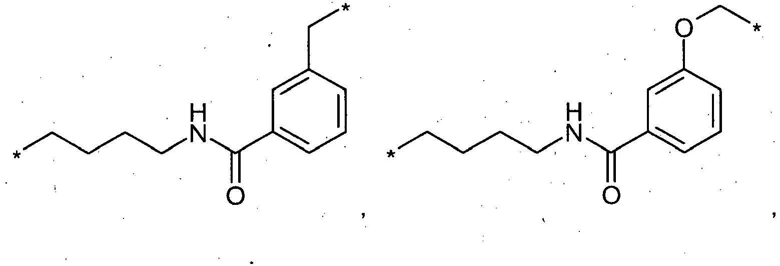 and

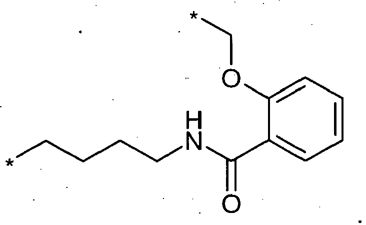

In one embodiment the present invention the linker A or the linker B contains one or more additional reactive groups, which are not used for the coupling of the two polypeptides.

In one embodiment the present invention relates to the use of a compound for the introduction of two reactive groups to the C-terminus of a peptide or protein, which compound has the structure

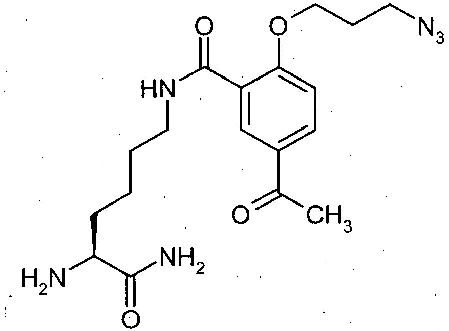

Herein described is the compound N,N'-bis(2-(2-(2-(2-(4-((3-(N-((S)-5-(carbamoyl)-5-(hGHylleucinylamino)pentyl)carbamoyl)benzyloxy)imino)-butoxy)ethoxy)ethoxy)ethoxy)ethyl)omega-carbamoyl3.4 kDa PEG-carboxylic acid amide.

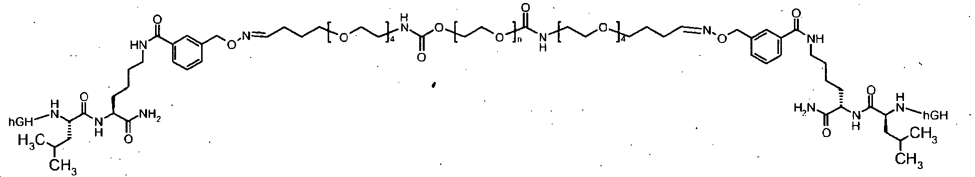

Also described are coupled polypeptide compounds prepared according to the methods of the present invention.

Also described is a polypeptide compound comprising two polypeptides coupled via their C-termini, wherein said polypeptide compounds has the structure as defined in any one of the above methods.

Also described is a polypeptide compound comprising two polypeptides coupled via their C-termini, wherein the linking-moiety comprises a 1,2,3-triazole moiety.

The term "linking-moiety" or "moiety linking the two polypeptides in the end-product" as used herein means the moiety linking the two polypeptides, that is, the moiety comprising all the atoms not present in the original polypeptides.

The term "1,2,3-triazole moiety" as used herein means a diradical with the structure of:

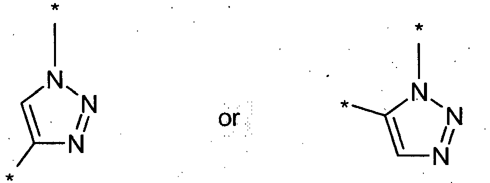

Examples of linking moieties comprising a 1,2,3-triazole moiety are 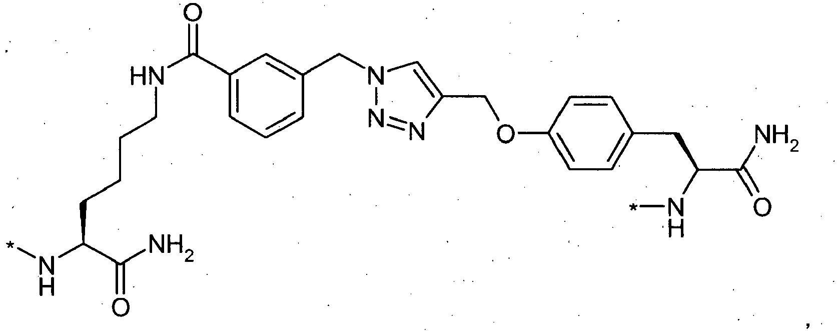 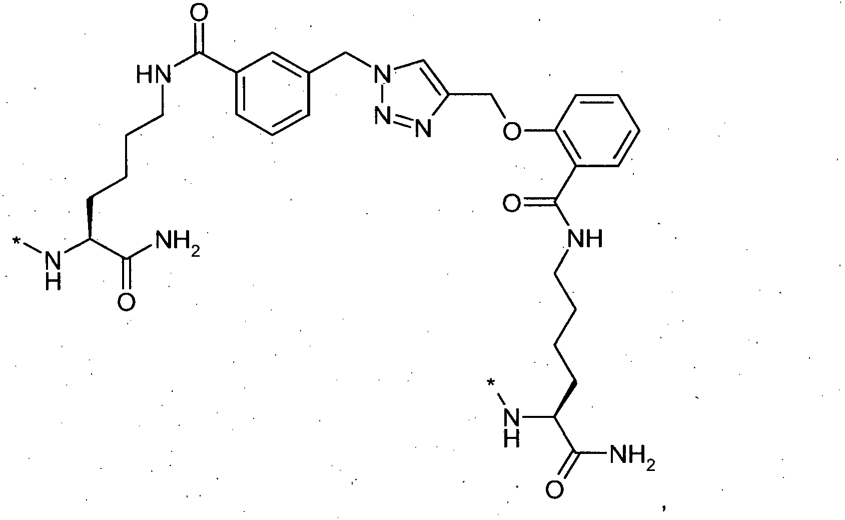 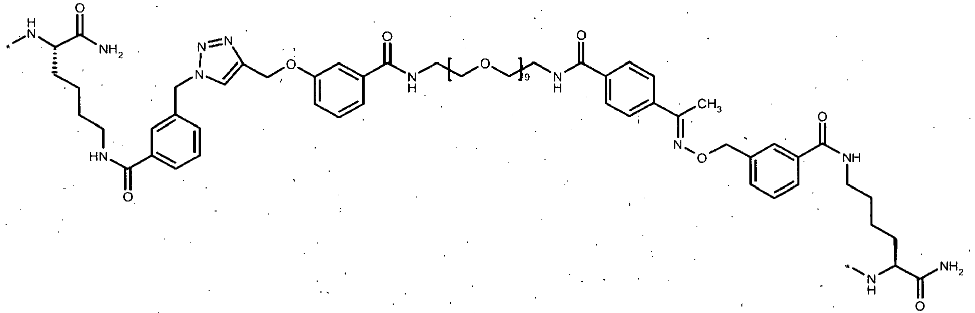 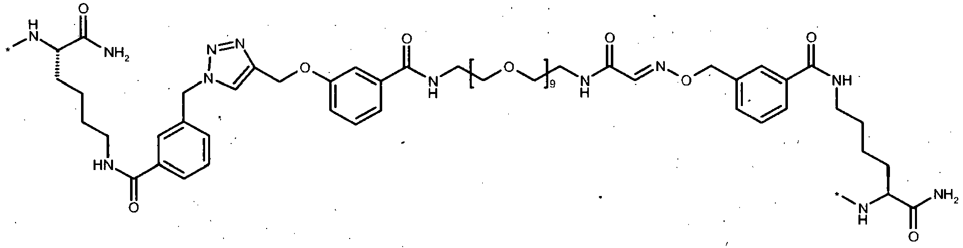 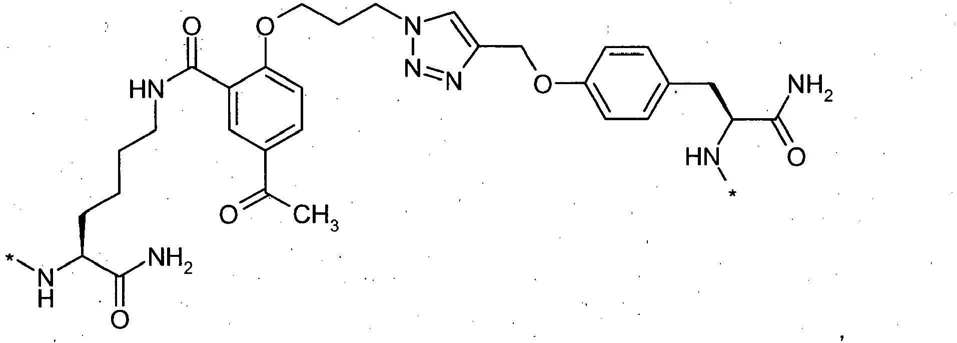 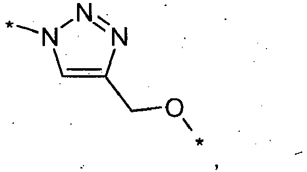 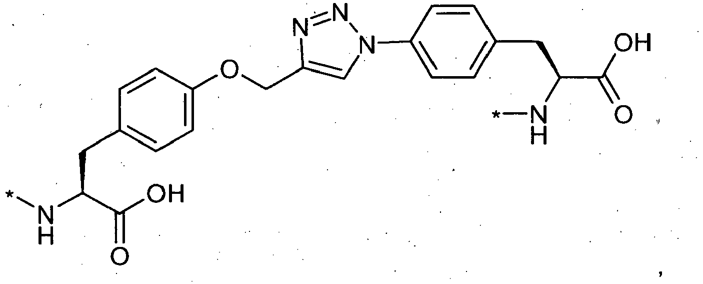 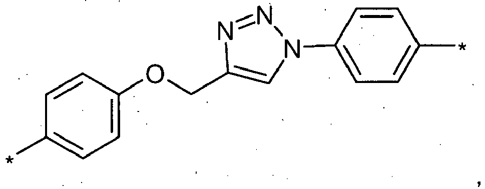 and

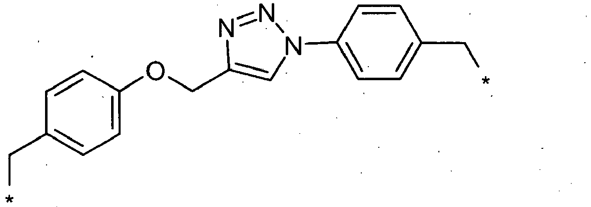

Herein described is a polypeptide compound comprising two polypeptides coupled via their C-termini, wherein the linking-moiety comprises a 1,2,3-triazole moiety, wherein said polypeptide compound is obtained or obtainable by use of a method according to the invention.

In one embodiment the present invention provides a method according to the invention for preparation of a polypeptide compound comprising two polypeptides coupled via their C-termini, wherein the linking-moiety comprises a 1,2,3-triazole moiety.

### Conjugation.

The coupled polypeptide compounds according to the present invention may also be conjugated, i.e. attachment (conjugation) to a chemical group, e.g. a non-polypeptide moiety.

Hence, the method may further comprise the simultaneous and/or subsequent step of conjugating at least one of the constituent polypeptides to a chemical group. Such conjugation may be performed via a reduced cysteine residue, or it may be performed via a glutamic acid residue.

It is to be understood that conjugation may be conducted on one of the constituent polypeptides before synthesis of the coupled polypeptide compound, or it may be conducted after the coupled polypeptide compound has been synthesized.

Conjugation may for instance be conducted as disclosed in WO 02/077218 and WO 01/58935 or as it is otherwise known in the art.

The conjugated chemical group may be a protractor group, i.e. a group which upon conjugation to a polypeptide increases the circulation half-life of said polypeptide, when compared to the un-modified polypeptide. The specific principle behind the protractive effect may be caused by increased size, shielding of peptide sequences that can be recognized by peptidases or antibodies, or masking of glycanes in such way that they are not recognized by glycan specific receptors present in e.g. the liver or on macrophages, preventing or decreasing clearance. The protractive effect of the protractor group can e.g. also be caused by binding to blood components such as albumin, or unspecific adhesion to vascular tissue. The conjugated polypeptide should substantially preserve its biological activity.

The protractor group may be selected from the group consisting of:
(a) a low molecular organic charged radical (15-1,000 Da), which may contain one or more carboxylic acids, amines sulfonic acids, phosphonic acids, or combination thereof,
(b) a low molecular (15-1,000 Da) neutral hydrophilic molecule, such as cyclodextrin, or a polyethylene chain which may optionally branched,
(c) a low molecular (15-1,000 Da) hydrophobic molecule such as a fatty acid or cholic acid or derivatives thereof,
(d) polyethyleneglycol with an average molecular weight of 2,000-60,000 Da.
(e) a well defined precision polymer such as a dendrimer with an exact molecular mass ranging from 700 to 20,000 Da, or more preferable between 700-10,000 Da,
(f) a substantially non-immunogenic polypeptide such as albumin, an antibody or a part thereof, e.g. an albumin fragment or an antibody fragment optionally containing an Fc-domain, and
(g) a high molecular weight organic polymer such as dextran.

The protractor group may be selected from the group consisting of dendrimers, polyalkylene oxide (PAO), including polyalkylene glycol (PAG), such as polyethylene glycol (PEG) and polypropylene glycol (PPG), branched PEGs, polyvinyl alcohol (PVA), polycarboxylate, poly-vinylpyrolidone, polyethylene-co-maleic acid anhydride, polystyrene-co-maleic acid anhydride, and dextran, including carboxymethyldextran. In one embodiment of the invention, the protractor group is a PEG group.

The term "branched polymer", or interchangeably "dendritic polymer", "dendrimer" or "dendritic structure" means an organic polymer assembled from a selection of monomer building blocks of which, some contains branches.

The protractor group may be selected from the group consisting of serum protein binding-ligands, such as compounds which bind to albumin, like fatty acids, C5-C24 fatty acid, aliphatic diacid (e.g. C5-C24). Other examples of protractor groups includes small organic molecules containing moieties that under physiological conditions alters charge properties, such as carboxylic acids or amines, or neutral substituents that prevent glycan specific recognition such as smaller alkyl substituents (e.g., C1-C5 alkyl). The protractor group may be albumin.

The chemical group may also be a non-polypeptide, such as a polyethyleneglycol (PEG), in particular one having an average molecular weight of in the range of 500-100,000, such as 1,000-75,000, or 2,000-60,000.

The term "polyethylene glycol" or "PEG" means a polyethylene glycol compound or a derivative thereof, with or without coupling agents, coupling or activating moieties (e.g., with thiol, triflate, tresylate, azirdine, oxirane, pyridyldithio, vinyl sulfone, or preferably with a maleimide moiety). Compounds such as maleimido monomethoxy PEG are exemplary of activated PEG compounds of the disclosure.

PEG is a suitable polymer molecule, since it has only few reactive groups capable of cross-linking compared to polysaccharides such as dextran. In particular, monofunctional PEG, e.g. methoxypolyethylene glycol (mPEG), is of interest since its coupling chemistry is relatively simple (only one reactive group is available for conjugating with attachment groups on the polypeptide). Consequently, the risk of cross-linking is eliminated, the resulting polypeptide conjugates are more homogeneous and the reaction of the polymer molecules with the polypeptide is easier to control.

To effect covalent attachment of the polymer molecule(s) to the polypeptide, the hydroxyl end groups of the polymer molecule are provided in activated form, i.e. with reactive functional groups. Suitable activated polymer molecules are commercially available, e.g. from Shearwater Corp., Huntsville, Ala., USA, or from PolyMASC Pharmaceuticals plc, UK. Alternatively, the polymer molecules can be activated by conventional methods known in the art, e.g. as disclosed in WO 90/13540. Specific examples of activated linear or branched polymer molecules for use in the present invention are described in the Shearwater Corp. 1997 and 2000 Catalogs (Functionalized Biocompatible Polymers for Research and pharmaceuticals, Polyethylene Glycol and Derivatives, incorporated herein by reference). Specific examples of activated PEG polymers include the following linear PEGs: NHS-PEG (e.g. SPA-PEG, SSPA-PEG, SBA-PEG, SS-PEG, SSA-PEG, SC-PEG, SG-PEG, and SCM-PEG), and NOR-PEG), BTC-PEG, EPOX-PEG, NCO-PEG, NPC-PEG, CDI-PEG, ALD-PEG, TRES-PEG, VS-PEG, IODO-PEG, and MAL-PEG, and branched PEGs such as PEG2-NHS and those disclosed in U.S. Pat. No. 5,932,462 and U.S. Pat. No. 5,643,575. Furthermore, the following publications, disclose useful polymer molecules and/or PEGylation chemistries: U.S. Pat. No. 5,824,778, U.S. Pat. No. 5,476,653, WO 97/32607, EP 229,108, EP 402,378, U.S. Pat. No. 4,902,502, U.S. Pat. No. 5,281,698, U.S. Pat. No. 5,122,614, U.S. Pat. No. 5,219,564, WO 92/16555, WO 94/04193, WO 94/14758, WO 94/17039, WO 94/18247, WO 94/28024, WO 95/00162, WO 95/11924, WO 95/13090, WO 95/33490, WO 96/00080, WO 97/18832, WO 98/41562, WO 98/48837, WO 99/32134, WO 99/32139, WO 99/32140, WO 96/40791, WO 98/32466, WO 95/06058, EP 439 508, WO 97/03106, WO 96/21469, WO 95/13312, EP 921 131, U.S. Pat. No. 5,736,625, WO 98/05363, EP 809 996, U.S. Pat. No. 5,629,384, WO 96/41813, WO 96/07670, U.S. Pat. No. 5,473,034, U.S. Pat. No. 5,516,673, EP 605 963, U.S. Pat. No. 5,382,657, EP 510 356, EP 400 472, EP 183 503 and EP 154 316.

The conjugation of the polypeptide and the activated polymer molecules may be conducted by use of any conventional method, e.g. as described in the following references (which also describe suitable methods for activation of polymer molecules): R. F. Taylor, (1991), "Protein immobilisation. Fundamental and applications", Marcel Dekker, N.Y.; S. S. Wong, (1992), "Chemistry of Protein Conjugation and Crosslinking", CRC Press, Boca Raton; G. T. Hermanson et al., (1993), "Immobilized Affinity Ligand Techniques", Academic Press, N.Y.). The skilled person will be aware that the activation method and/or conjugation chemistry to be used depends on the attachment group(s) of the polypeptide (examples of which are given further above), as well as the functional groups of the polymer (e.g. being amine, hydroxyl, carboxyl, aldehyde, sulfydryl, succinimidyl, maleimide, vinysulfone or haloacetate). The PEGylation may be directed towards conjugation to all available attachment groups on the polypeptide (i.e. such attachment groups that are exposed at the surface of the polypeptide) or may be directed towards one or more specific attachment groups, e.g. the N-terminal amino group (U.S. Pat. No. 5,985,265). Furthermore, the conjugation may be achieved in one step or in a stepwise manner (e.g. as described in WO 99/55377).

It will be understood that the PEGylation is designed so as to produce the optimal molecule with respect to the number of PEG molecules attached, the size and form of such molecules (e.g. whether they are linear or branched), and where in the polypeptide such molecules are attached. The molecular weight of the polymer to be used will be chosen taking into consideration the desired effect to be achieved. For instance, if the primary purpose of the conjugation is to achieve a conjugate having a high molecular weight and larger size (e.g. to reduce renal clearance), one may choose to conjugate either one or a few high molecular weight polymer molecules or a number of polymer molecules with a smaller molecular weight to obtain the desired effect. Preferably, however, several polymer molecules with a lower molecular weight will be used. This is also the case if a high degree of epitope shielding is desired. In such cases, 2-8 polymers with a molecular weight of e.g. about 5,000 Da, such as 3-6 such polymers, may for example be used. As the examples below illustrate, it may be advantageous to have a larger number of polymer molecules with a lower molecular weight (e.g. 4-6 with a M_{W} of 5,000) compared to a smaller number of polymer molecules with a higher molecular weight (e.g. 1-3 with a MW of 12,000-20,000) in terms of improving the functional in vivo half-life of the conjugate, even where the total molecular weight of the attached polymer molecules in the two cases is the same or similar. It is believed that the presence of a larger number of smaller polymer molecules provides the polypeptide with a larger diameter or apparent size than e.g. a single yet larger polymer molecule, at least when the polymer molecules are relatively uniformly distributed on the polypeptide surface.

It has further been found that advantageous results are obtained when the apparent size (also referred to as the "apparent molecular weight" or "apparent mass") of at least a major portion of the conjugate of the invention is at least about 50 kDa, such as at least about 55 kDa, such as at least about 60 kDa, e.g. at least about 66 kDa. This is believed to be due to the fact that renal clearance is substantially eliminated for conjugates having a sufficiently large apparent size. In the present context, the "apparent size" of a polypeptide is determined by the SDS-PAGE method.

Furthermore, it has been reported that excessive polymer conjugation can lead to a loss of activity of the polypeptide to which the chemical group (e.g. a non-polypeptide moiety) is conjugated (see further below). This problem can be eliminated, e.g., by removal of attachment groups located at the functional site or by reversible blocking the functional site prior to conjugation so that the functional site of the polypeptide is blocked during conjugation. Specifically, the conjugation between the polypeptide and the chemical group (e.g. non-polypeptide moiety) may be conducted under conditions where the functional site of the polypeptide is blocked by a helper molecule. Preferably, the helper molecule is one, which specifically recognizes a functional site of the polypeptide.

The polypeptide is preferably to interact with the helper molecule before effecting conjugation. (Often it is even advantageous to use the same helper molecule (e.g. an inhibitor) as the one used in the steps where mixed disulfides are reduced.) This ensures that the functional site of the polypeptide is shielded or protected and consequently unavailable for derivatization by the chemical group (e.g. non-polypeptide moiety) such, as a polymer.

Following its elution from the helper molecule, the conjugate of the chemical group and the protein can be recovered with at least a partially preserved functional site.

The following is a list of embodiments
1. A method for coupling two different polypeptides together at their respective C-terminus comprising
   (a) an enzyme catalyzed modification of the C-termini of the polypeptides to be coupled by which a chemical group comprising reactive group W is introduced to the C-terminus of the first polypeptide to be coupled, and a chemical group comprising reactive group Z is introduced to the C-terminus of the second polypeptide to be coupled
   (b) reacting
      the reactive group Z with a reactive group Y of a spacer-molecule to form a chemical group X and reacting the reactive group W with a reactive group V of the spacer-molecule to form a chemical group U, wherein spacer between the two polypeptides comprises at least one ethylene glycol diradical, wherein X and U are independently selected from the group consisting of diradicals of oxime and triazole wherein V, W, Y and Z are independently selected from the group consisting of alkoxylamino, oxo, azido and alkynyl,
2. A method according to embodiment 1, wherein the enzyme is carboxypeptidase Y or a variant or a fragment thereof, which variant or fragment retains the ability to catalyse a reaction, by which the C-terminal amino acid of a protein or peptide is replaced by a different chemical moiety.
3. A method according to embodiment 2, wherein the enzyme is carboxypeptidase Y.
4. A method according to any one of embodiments 1 to 3, wherein at least one of the polypeptides is human growth hormone.
5. A method according to any one of embodiments 1 to 4, wherein at least one of the polypeptides is a derivative of human growth hormone.
6. A method according to any one of embodiments 1 to 5, wherein at least one of the polypeptides is a variant of human growth hormone or a derivative of a variant of human growth hormone.
7. A method according to any one of embodiments 1 to 6, wherein at least one of the polypeptides is hGH-Leu-Ala.
8. A method according to any one of embodiments 1 to 7, wherein the chemical group Z is introduced via a linker A.
9. A method according to embodiment 8, wherein linker A is selected from the group consisting of bi-radicals of straight, branched and/or cyclic C 1-10 alkane, C2-10 alkene, C2 -ioalkyne, C1-10 heteroalkane, C2 -10 heteroalkene, C2-10 heteroalkyne, any of which may be substituted with oxo, wherein one or more homocyclic aromatic compound biradical(s) or heterocyclic aromatic compound biradical(s) may be inserted.
10. A method according to any one of embodiments 1 to 9, wherein the chemical group W is introduced via a linker B.
11. A method according to embodiment 10, wherein linker B is selected from the group consisting of bi-radicals of straight, branched and/or cyclic C1-10 alkane, C2 -10 alkene, C2 -10 alkyne, C1 -10 heteroalkane, C2-10 heteroalkene, C2-10 heteroalkyne, which is optionally substituted with oxo, wherein one or more homocyclic aromatic compound biradical(s) or heterocyclic aromatic compound biradical(s) may be inserted.
12. A method according to any one of embodiments 8 to 11, wherein linker A and linker B are independently selected from the group consisting of 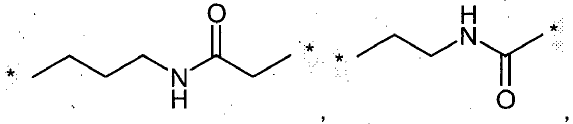 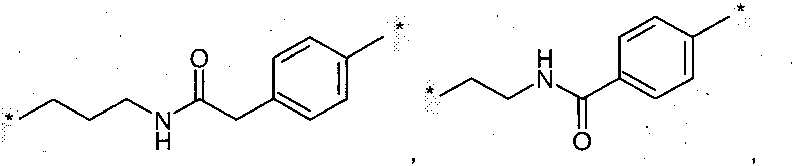 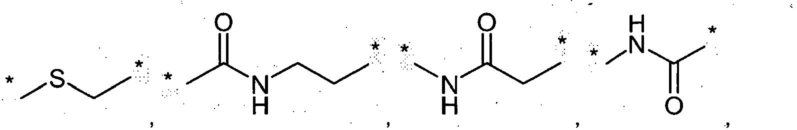 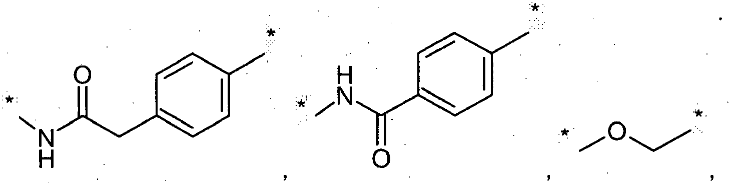 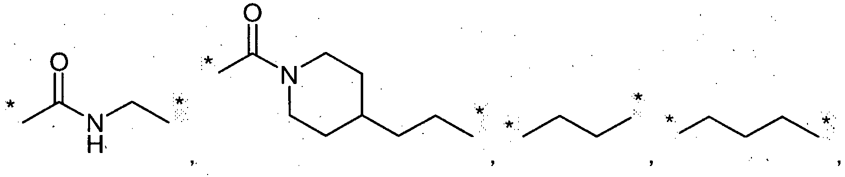 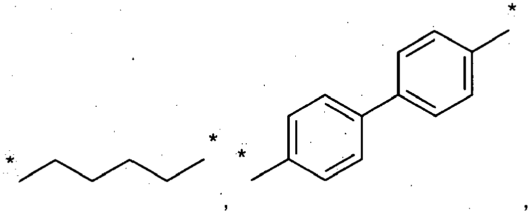 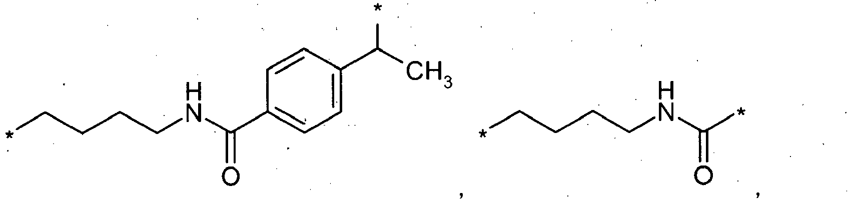 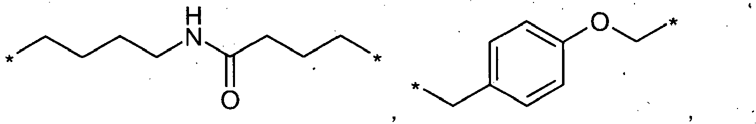 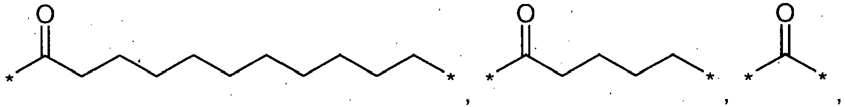 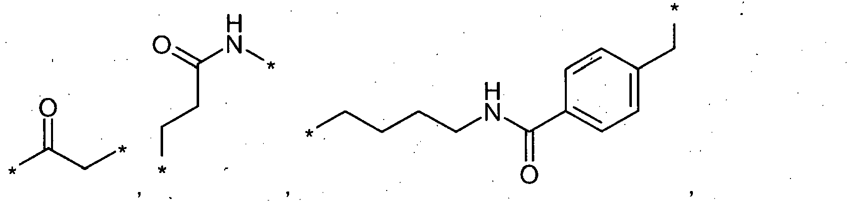 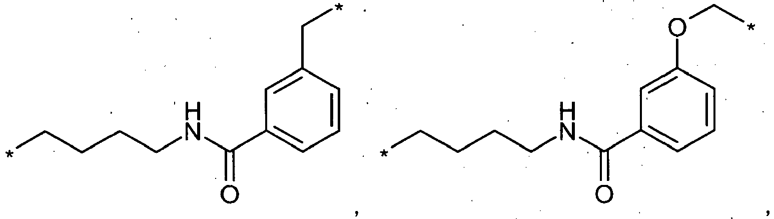 and

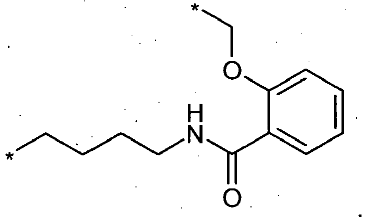

Embodiment 13 A method according to any of embodiments 1 to 12, wherein the moiety linking the two polypeptides in the end-product comprises a 1,2,3-triazole moiety.

The following linking moieties are disclosed: 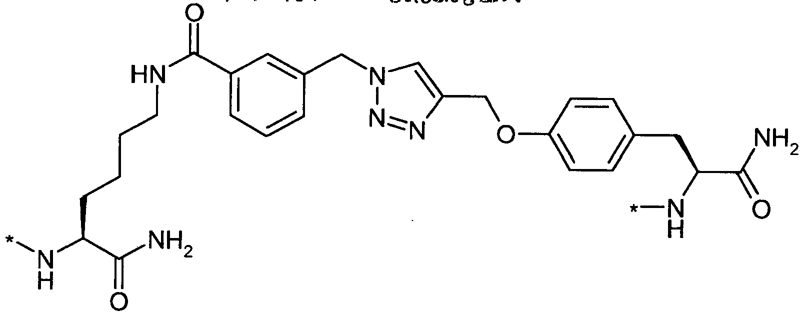 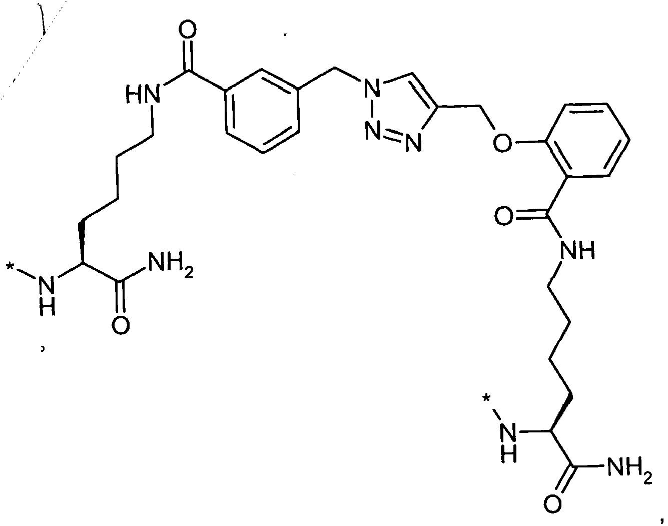 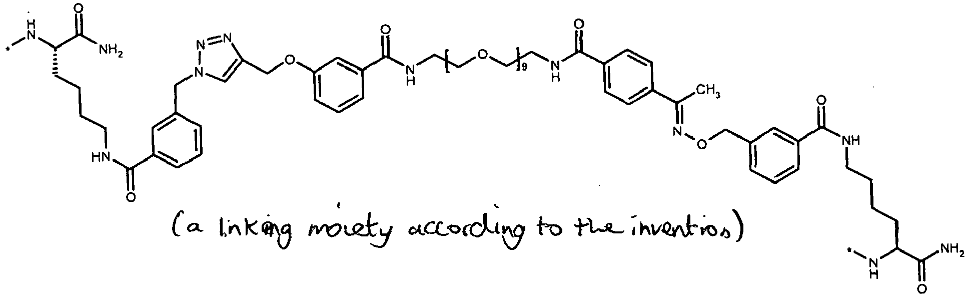 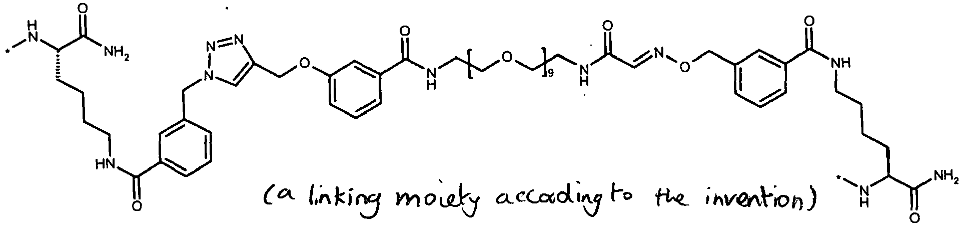 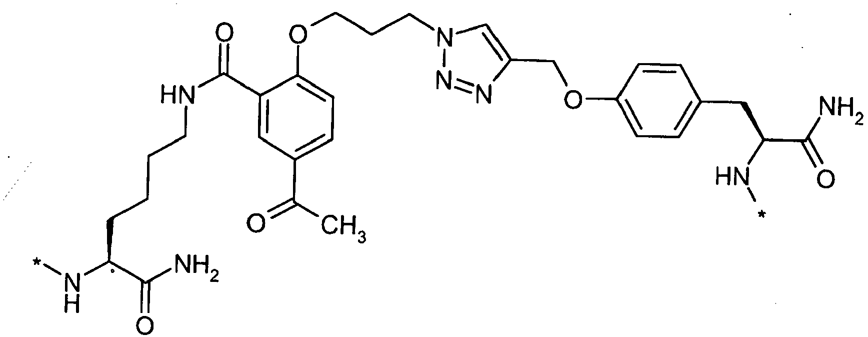 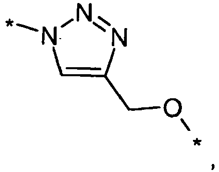 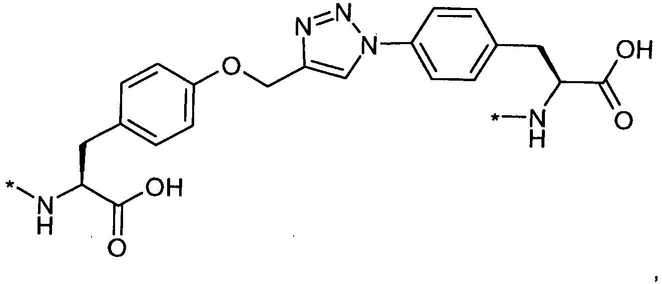 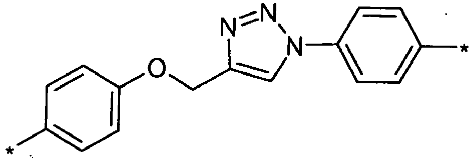 and

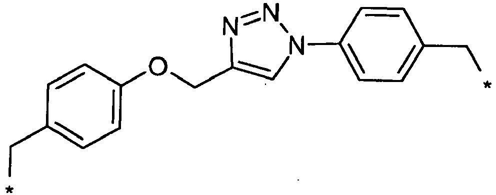

### Pharmaceutical compositions.

The coupled polypeptide compounds according to the present invention are applicable as pharmaceutical compositions for the treatment of disorders or diseases in patients.

Herein described are pharmaceutical compositions comprising a coupled polypeptide compound as an active ingredient, or a pharmaceutically acceptable salt thereof together with a pharmaceutically acceptable carrier or diluent.

The compounds disclosed herein may be formulated into pharmaceutical compositions comprising the compounds and a pharmaceutically acceptable carrier or diluent. Such carriers include water, physiological saline, ethanol, polyols, e.g., glycerol or propylene glycol, or vegetable oils. As used herein, "pharmaceutically acceptable carriers" also encompasses any and all solvents, dispersion media, coatings, antifungal agents, preservatives, isotonic agents and the like. Except insofar as any conventional medium is incompatible with the active ingredient and its intended use, its use in the compositions of the present invention is contemplated.

The compositions may be prepared by conventional techniques and appear in conventional forms, for example, capsules, tablets, solutions or suspensions. The pharmaceutical carrier employed may be a conventional solid or liquid carrier. Examples of solid carriers are lactose, terra alba, sucrose, talc, gelatine, agar, pectin, acacia, magnesium stearate and stearic acid. Examples of liquid carriers are syrup, peanut oil, olive oil and water. Similarly, the carrier or diluent may include any time delay material known to the art, such as glyceryl monostearate or glyceryl distearate, alone or mixed with a wax. The formulations may also include wetting agents, emulsifying and suspending agents, preserving agents, sweetening agents or flavouring agents. The formulations described herein may be formulated so as to provide quick, sustained, or delayed release of the active ingredient after administration to the patient by employing procedures well known in the art.

The pharmaceutical compositions can be sterilised and mixed, if desired, with auxiliary agents, emulsifiers, salt for influencing osmotic pressure, buffers and/or colouring substances and the like, which do not deleteriously react with the active compounds.

The route of administration may be any route, which effectively transports the active compound to the appropriate or desired site of action, such as oral or parenteral, e.g., rectal, transdermal, subcutaneous, intranasal, intramuscular, topical, intravenous, intraurethral, ophthalmic solution or an ointment, the oral route being preferred.

If a solid carrier for oral administration is used, the preparation can be tabletted, placed in a hard gelatine capsule in powder or pellet form or it can be in the form of a troche or lozenge. The amount of solid carrier may vary widely but will usually be from about 25 mg to about 1 g. If a liquid carrier is used, the preparation may be in the form of a syrup, emulsion, soft gelatine capsule or sterile injectable liquid such as an aqueous or nonaqueous liquid suspension or solution.

For nasal administration, the preparation may contain a compound of formula (I) dissolved or suspended in a liquid carrier, in particular an aqueous carrier, for aerosol application. The carrier may contain additives such as solubilizing agents, e.g. propylene glycol, surfactants, absorption enhancers such as lecithin (phosphatidylcholine) or cyclodextrin, or preservatives such as parabenes.

For parenteral application, particularly suitable are injectable solutions or suspensions, preferably aqueous solutions with the active compound dissolved in polyhydroxylated castor oil.

Tablets, dragees, or capsules having talc and/or a carbohydrate carrier or binder or the like are particularly suitable for oral application. Preferable carriers for tablets, dragees, or capsules include lactose, corn starch, and/or potato starch. A syrup or elixir can be used in cases where a sweetened vehicle can be employed.

A typical tablet, which may be prepared by conventional tabletting techniques, contains

### Core:

| | |
|---|---|
| Active compound (as free compound or salt thereof) | 10 mg |
| Colloidal silicon dioxide (Areosil^{®}) | 1.5 mg |
| Cellulose, microcryst. (Avicel^{®}) | 70 mg |
| Modified cellulose gum (Ac-Di-Sol^{®}) | 7.5 mg |
| Magnesium stearate | |
| Coating: | |
| HPMC | approx. 9 mg |
| *Mywacett^{®} 9-40 T | approx. 0.9 mg |

| | |
|---|---|
| *Acylated monoglyceride used as plasticizer for film coating. | |

The compounds described herein may be administered to a mammal, especially a human in need of such treatment, prevention, elimination, alleviation or amelioration of various diseases or disorders. Such mammals also include animals, both domestic animals, e.g. household pets, and non-domestic animals such as wildlife.

Usually, dosage forms suitable for oral, nasal, pulmonal or transdermal administration comprise from about 0.001 mg to about 100 mg, preferably from about 0.01 mg to about 50 mg of the compounds of formula I admixed with a pharmaceutically acceptable carrier or diluent.

The compounds may be administered concurrently, simultaneously, or together with a pharmaceutically acceptable carrier or diluent, whether by oral, rectal, or parenteral (including subcutaneous) route. The compounds are often, and preferably, in the form of an alkali metal or earth alkali metal salt thereof.

Suitable dosage ranges varies as indicated above depending upon the exact mode of administration, form in which administered, the indication towards which the administration is directed, the subject involved and the body weight of the subject involved, and the preference and experience of the physician or veterinarian in charge.

Compounds prepared by use of a method according to the present invention may be used in the treatment of diseases or disorders, which may be treated with growth hormone, for instance growth hormone deficiency (GHD), e.g. in adults; Turner syndrome; Prader-Willi syndrome (PWS); Noonan syndrome; Downs syndrome; chronic renal disease; juvenile rheumatoid arthritis; cystic fibrosis; HIV-infection in children receiving HAART treatment (HIV/HALS children); children born small for gestational age (SGA); short stature in children born with very low birth weight (VLBW); skeletal dysplasia; hypochondroplasia; achondroplasia; idiopathic short stature (ISS); fractures in or of long bones, such as tibia, fibula, femur, humerus, radius, ulna, clavicula, metacarpea, metatarsea, and digit; fractures in or of spongious bones, such as the skull, base of hand and base of foot; post-surgical treatment of patients who have undergone tendon or ligament surgery in, e.g., the hand, knee, or shoulder; treatment of patients undergoing distraction osteogenesis; post-surgical treatment of patients who have undergone hip or discus replacement, meniscus repair, spinal fusions or prosthesis fixation, such as in the knee, hip, shoulder, elbow, wrist or jaw; treatment of patients who have undergone fixation of osteosynthesis material, such as nails, screws or plates; non-union or mal-union of fractures; treatment of patients after osteatomia, e.g. from the tibia or 1st toe; treatment of patients after graft implantation; articular cartilage degeneration in the knee caused by trauma or arthritis; osteoporosis in patients with Turner syndrome; osteoporosis in men; treatment of adult patients in chronic dialysis (APCD); treatment of elderly patients in chronic dialysis; cardiovascular disease in APCD; cachexia in APCD; cancer in APCD; chronic obstructive pulmonary disease in APCD; HIV in APCD; chronic liver disease in APCD; fatigue syndrome in APCD; Crohn's disease; impaired liver function; treatment of males with HIV infections; short bowel syndrome; central obesity; HIV-associated lipodystrophy syndrome (HALS); male infertility; treatment of patients after major elective surgery; treatment of patients with negative nitrogen balance; alcohol/drug detoxification or neurological trauma; aging; frail elderly; osteoarthritis; treatment of patients with traumatically damaged cartilage; erectile dysfunction; fibromyalgia; memory disorders; depression; traumatic brain injury; subarachnoid haemorrhage; very low birth weight; metabolic syndrome; glucocorticoid myopathy; and short stature due to glucucorticoid treatment in children.

The use of the terms "a" and "an" and "the" and similar referents in the context of describing the invention are to be construed to cover both the singular and the plural, unless otherwise indicated herein or clearly contradicted by context.

Unless otherwise stated, all exact values provided herein are representative of corresponding approximate values (e.g., all exact exemplary values provided with respect to a particular factor or measurement can be considered to also provide a corresponding approximate measurement, modified by "about," where appropriate).

The description herein of any aspect or embodiment of the invention using terms such as "comprising", "having," "including," or "containing" with reference to an element or elements is intended to provide support for a similar aspect or embodiment of the invention that "consists of", "consists essentially of", or "substantially comprises" that particular element or elements, unless otherwise stated or clearly contradicted by context (e.g., a composition described herein as comprising a particular element should be understood as also describing a composition consisting of that element, unless otherwise stated or clearly contradicted by context).

All headings and sub-headings are used herein for convenience only and should not be construed as limiting the invention in any way.

The present invention is further illustrated by the following examples

### EXAMPLES

### HPLC Method 02-b1-2:

The RP-analyses was performed using an Alliance Waters 2695 system fitted with a Waters 2487 dualband detector. UV detections at 214nm and 254nm were collected using a Symmetry 300 C18, 5 um, 3.9 mm x 150 mm column, 42°C. The compounds are eluted with a linear gradient of 0-60% acetonitrile in water which is buffered with 0.05% trifluoroacetic acid over 15 minutes at a flow-rate of 1.0 min/min.

### HPLC Method 02-b4-4:

The RP-analyses was performed using an Alliance Waters 2695 system fitted with a Waters 2487 dualband detector. UV detections at 214nm and 254nm were collected using a Symmetry300 C18, 5 um, 3.9 mm x 150 mm column, 42°C. The compounds are eluted with a linear gradient of 5-95% acetonitrile in water which is buffered with 0.05% trifluoroacetic acid over 15 minutes at a flow-rate of 1.0 min/min.

### HPLC Method 03-b1-1:

The RP-analysis was performed using a Waters 2690 systems fitted with a Waters 996 diode array detector. UV detections were collected at 214, 254, 276, and 301 nm on a 218TP54 4.6 mm x 250 mm 5µ C-18 silica column (The Seperations Group, Hesperia), which was eluted at 0.5 ml/min at 42°C. The column was equilibrated with water, which was buffered with 0.1 % trifluoroacetic acid. After injection, the sample was eluted by a gradient of 0% to 60% acetonitrile, which was buffered with 0.1% trifluoroacetic acid, in a 0.1% aqueous solution of trifluoroacetic acid in water during 50 min.

### HPLC Method 03 b6 1:

HPLC (Method 03_B6_1): The RP-analysis was performed using a Waters 2690 systems fitted with a Waters 996 diode array detector. UV detections were collected at 214, 254, 276, and 301 nm on a 218TP54 4.6 mm x 250 mm 5µ C-18 silica column (The Seperations Group, Hesperia), which was eluted at 0.5 ml/min at 42°C. The column was equilibrated with 5% acetonitrile (+ 0.1% TFA) in an aqueous solution of TFA in water (0.1%). After injection, the sample was eluted by a gradient of 0% to 90% acetonitrile (+ 0.1% TFA) in an aqueous solution of TFA in water (0.1%) during 50 min.

Mass spectra for peptides were obtained on an Agilent 1100 Series in the range of 500-1800 Da or on Perkin Elmer PE API 100 in the range of 500-2000 Da. Typically the found signals for m/z correspond to a series of any of z = 1, 2, 3, 4, 5, or 6.

MALDI-TOF spectra were obtained on a Bruker Daltonix autoflex.

### Example 1

### (S)-2-(([Glu3,Leu10]GLP-2yl)leucylamino)-6-(3-(((1-(3-((2-(2-(2-(2-(2-(2-(2-(2-(2-(2-(2-(4-((4-((S)-2-(carbamoyl)-2-(([Glu180,Asp182,Glu189]hGH(180-191)yl)leucylamino)ethyl)-phenoxy)methyl)1,2,3-triazol-1-yl)ethoxy)ethoxy)ethoxy)ethoxy)ethoxy)ethoxy)ethoxy)-ethoxy)ethoxy)ethoxy)ethyl)carbamoyl)phenyl)ethylidene)aminoxy)methyl)benzoylamino)-hexanoic amide

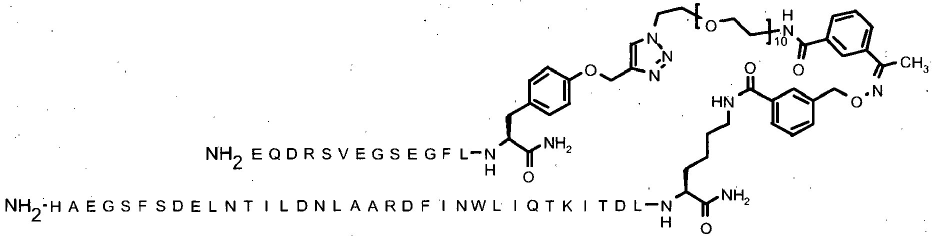

### Step 1

### [Glu³,Leu¹⁰]GLP-2ylleuccinylalanine

[Glu³,Leu¹⁰]GLP-2ylleuccinylalanine was prepared on an Applied Biosystems 433A Peptide Synthesizer by standard Fmoc-strategy, known to a person skilled in the art, starting with a commercially available Fmoc-Ala-Wang resin. Following amino acid derivatives were used:

| coupling no. | amino acid derivative |
|---|---|
| 1 | Fmoc-Leu-OH |
| 2 | Fmoc-Asp(OtBu)-OH |
| 3 | Fmoc-Thr(tBu)-OH |
| 4 | Fmoc-Ile-OH |
| 5 | Fmoc-Lys(Boc)-OH |
| 6 | Fmoc-Thr(tBu)-OH |
| 7 | Fmoc-Gln(Trt)-OH |
| 8 | Fmoc-Ile-OH |
| 9 | Fmoc-Leu-OH |
| 10 | Fmoc-Trp(Boc)-OH |
| 11 | Fmoc-Asn(Trt)-OH |
| 12 | Fmoc-Ile-OH |
| 13 | Fmoc-Phe-OH |
| 14 | Fmoc-Asp(OtBu)-OH |
| 15 | Fmoc-Arg(Pmc)-OH |
| 16 | Fmoc-Ala-OH |
| 17 | Fmoc-Ala-OH |
| 18 | Fmoc-Leu-OH |
| 19 | Fmoc-Asn(Trt)-OH |
| 20 | Fmoc-Asp(OtBu)-OH |
| 21 | Fmoc-Leu-OH |
| 22 | Fmoc-Ile-OH |
| 23 | Fmoc-Thr(tBu)-OH |
| 24 | Fmoc-Asn(Trt)-OH |
| 25 | Fmoc-Leu-OH |
| 26 | Fmoc-Glu(OtBu)-OH |
| 27 | Fmoc-Asp(OtBu)-OH |
| 28 | Fmoc-Ser(tBu)-OH |
| 29 | Fmoc-Phe-OH |
| 30 | Fmoc-Ser(tBu)-OH |
| 31 | Fmoc-Gly-OH |
| 32 | Fmoc-Glu(OtBu)-OH |
| 33 | Fmoc-Ala-OH |
| 34 | Fmoc-His(Trt)-OH |

A mixture of trifluoroacetic acid (10 ml), water (0.265 ml) and triisopropylsilane (0.265 ml) was added to the resin. It was shaken for 1.5 h. The liquid was collected. The resin was washed with trifluoroacetic acid (1 ml). The liquids were combined. The solution was concentrated under a stream of nitrogen. Ether (40 ml) was added. The precipitation was isolated by centrifugation. The crude product was purified on a reversed phase C₁₈-column on a HPLC, using a gradient of 37-65% acetonitrile in water in a buffer of 0.1% trifluoroacetic acid, as eluent.
HPLC: 8.81 min (method 02-B4-4).
MALDI-TOF: m/z = 3946
MS: m/z = 1317. 988, 790.

### Step 2:

### [1-Carbamoyl-2-(4-hydroxyphenyl)ethyl]-carbamic acid tert-butyl ester

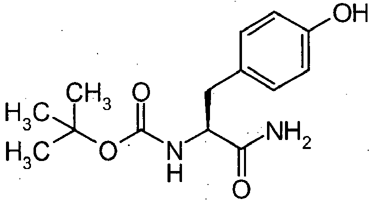

At 0°C, 1-(3-dimethylaminopropyl)-3-ethylcarbodiimide hydrochloride (17.0 g, 88,9 mmol) was added to a solution of (S)-2-(tert-butoxycarbonylamino)-3-(4-hydroxyphenyl)-propionic acid (25 g, 88.9 mmol) and 1-hydroxybenzotriazole (12.0 g, 88.9 mmol) in *N,N-*dimethylformamide (250 ml) and dichloromethane (250 ml). The reaction mixture was stirred at 0°C for 20 min. A 25% aqueous solution of ammonia in water (90 ml) was added. The reaction mixture was stirred for 3 days at room temperature. It was diluted with ethyl acetate (500 ml) and acidified with a 10% aqueous solution of sodium hydrogensulphate. The phases were separated. The aqueous phase was extracted with ethyl acetate (300 ml). The combined organic layers were washed with a mixture of water (250 ml) and a saturated aqueous solution of sodium hydrogencarbonate solution (250 ml). They were dried over magnesium sulphate. The solvent was removed in vacuo. The crude product was crystallized from ethyl acetate/heptane.
MS: m/z = 303 (M + Na)⁺.
¹H-NMR (DMSO-d₆): δ 1.31 (s 9 H); 2.80 (dd, 1 H); 2.83 (dd, 1 H); 4.00 (m, 1 H); 6.62 (d, 2 H); 6.70 (d, 1 H); 6.97 (br, 1 H); 7.03 (d, 2 H); 7.31 (br, 1 H); 9.14 (s, 1 H).

### Step 3:

### [(S)-1-Carbamoyl-2-(4-(prop-2-ynyloxy)phenyl)ethyl]carbamic acid tert-butyl ester

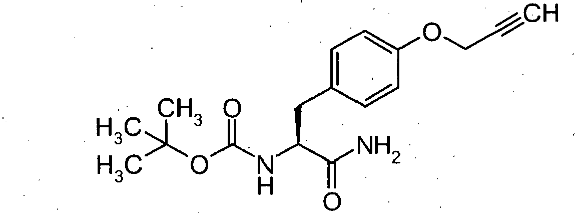

A mixture of of [(*S*)-1-carbamoyl-2-(4-hydroxyphenyl)ethyl]-carbamic acid tert-butyl ester (1.0 g, 3.57 mmol), tetrabutylammonium iodide (65 mg, 0.17 mmol), potassium carbonate (3.94 g, 29 mmol), propargyl bromide (0.38 ml, 4.28 mmol) and N,N-dimethylformamide (15 ml) was heated to 60°C for 16 h. It was cooled to room temperature, diluted with water (30 ml) and acidified with a 10% aqueous solution of sodium hydrogensulphate. The mixture was extracted with ethyl acetate (2 x 100 ml). The combined organic layers were washed with a saturated aqueous solution of sodium hydrogencarbonate (200 ml) and dried over magnesium sulphate. The solvent was removed in vacuo. The crude product was purified by flash chromatography on silica (100 g), using a mixture of dichloromethane/methanol (10:1) as eluent, to give 998 mg of [(*S*)-1-carbamoyl-2-(4-(prop-2-ynyloxy)phenyl)ethyl]carbamic acid tert-butyl ester.
MS: m/z = 341 (M + Na)⁺.
¹H-NMR (DMSO-d₆) δ 1.31 (s, 9 H); 2.50 (s, 1 H); 2.67 (dd, 1 H); 2.91 (dd, 1 H); 4.03 (m, 1 H); 4.74 (s, 2 H); 6.77 (d, 1 H); 6.86 (d, 2 H); 6.99 (s, 1 H), 7.17 (d, 2 H); 7.35 (s, 1 H).

### Step 4:

### (2S)-2-Amino-3-(4-(prop-2-ynyloxy)phenyl)propionamide

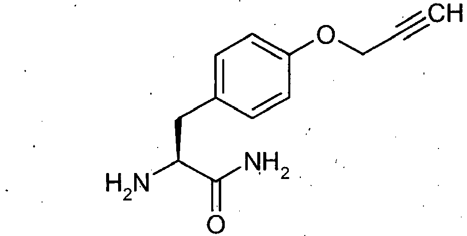

Trifluoroacetic acid (10 ml) was added to a solution of [(*S*)-1-crbamoyl-2-(4-(prop-2-ynyloxy)phenyl)ethyl]carbamic acid tert-butyl ester (998 mg, 3.13 mmol) in dichloromethane (10 ml). The reaction mixture was stirred for 1.5 h at room temperature. The solvent was removed. The residue was dissolved in dichloromethane (30 ml). The solvent was removed. The latter procedure was repeated twice to give 1.53 g of the trifluoroacetate salt of (2*S*)-2-amino-3-(4-(prop-2-ynyloxy)phenyl)propionamide.
HPLC (method 02-B4-4): R_{f} = 5.62 min.
MS: m/z = 219 (M + 1)⁺.
¹H-NMR (CDCl₃) δ 2.51 (s, 1 H); 3.02 (m, 2 H); 3.90 (m, 1 H); 4.78 (s, 2 H); 6.95 (d, 2 H); 7.20 (d, 2 H); 7.56 (s, 1 H); 7.87 (s, 1 H); 8.10 (br, 3 H).

### Step 5:

### Methyl 3-(((tert-butoxycarbonyl)aminoxy)methyl)benzoate

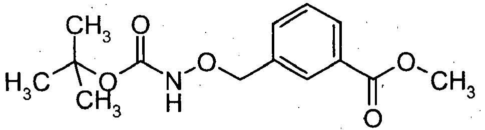

Methyl 3-bromomethylbenzoate (10.0 g, 43.7 mmol) was dissolved in acetnitrile (50 ml). tert-Butyl N-hydroxycarbamate (8.72 g, 65.5 mmol) and 1,8-diazabicycloundec-7-ene (DBU, 9.79 ml, 65.48 mmol) were added successively. The reaction mixture was heated to 50°C for 16 h. It was cooled to room temperature and diluted with water (200 ml) and concentrated hydrochloric acid (25 ml). It was extracted with ethyl acetate (3 x 200 ml). The combined organic layers were washed with brine (200 ml) and dried over sodium sulphate. The solvent was removed in vacuo to give 14.15 g of methyl 3-(((tert-butoxycarbonyl)aminoxy)methyl)benzoate, which was used without further purification. ¹H-NMR (CDCl₃): δ 1.48 (s, 9 H); 3.91 (s, 3 H); 4.90 (s, 2 H); 7.45 (m, 2 H); 7.60 (d, 1 H); 8.00 (d, 1 H); 8.05 (s, 1 H).
MS:m/z = 183.

### Step 6:

### 3-(((tert-Butoxycarbonyl)aminoxy)methyl)benzoic acid

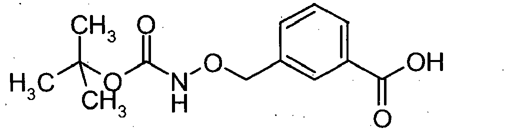

Crude methyl 3-(((tert-butoxycarbonyl)aminoxy)methyl)benzoate (12.28 g, 43.6 mmol) was dissolved in dioxan (50 ml). A solution of lithium hydroxide (1.26 g, 52.4 mmol) in water (50 ml) was added. Dioxan and water were added until a clear solution was obtained. The reaction mixture was stirred at room temperature for 16 h. An 1 N aqueous solution of sodium hydroxide (200 ml) was added. The aqueous solution was washed with tert-butyl methyl ether (2 x 200 ml). The aqueous phase was acidified by addition of a 10% aqueous solution of sodium hydrogensulphate. It was extracted with ethyl acetate (2 x 200 ml). The combined ethyl acetate layers were dried over sodium sulphate. The solvent was removed in vacuo to give 12.28 g of crude 3-(((tert-butoxycarbonyl)aminoxy)methyl)benzoic acid, which was used in the next step without further purification.
¹H-NMR (CDCl₃): δ 1.49 (s, 9 H); 4.92 (s, 2 H); 7.50 (t, 1 H); 7.65 (br, 1 H); 7.65 (d, 1 H); 8.07 (d, 1 H); 8.12 (s, 1 H).
MS: m/z = 169.

### Step 7:

### 2,5-Dioxopyrrolidine-1yl 3-(((tert-butoxycarbonyl)aminoxy)methyl)benzoate

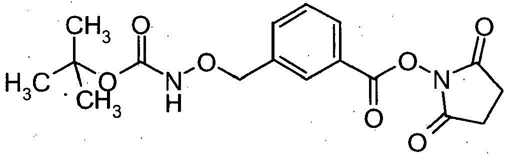

2-Succinimido-1,1,3,3-tetramethyluronium tetrafluoroborate (TSTU, 16.71 g, 55.13 mmol) was added to a solution of crude 3-(((tert-butoxycarbonyl)aminoxy)methyl)benzoic acid (12.28 g, 45.94 mmol) and triethlyamine (7.68 ml, 55.13 mmol) in *N,N-*dimethylformamide (75 ml). The reaction mixture was stirred at room temperature for 16 h. It was diluted with ethyl acetate (300 ml) and washed with water (3 x 150 ml). The organic layer was washed with a saturated aqueous solution of sodium hydrogencarbonate (200 ml) and dried over sodium sulphate. The solvent was removed in vacuo to give 11.04 g of crude 2,5-dioxopyrrolidine-1yl 3-(((tert-butoxycarbonyl)aminoxy)methyl)benzoate, which was used without further purification.
¹H-NMR (CDCl₃): δ 1.47 (s, 9 H); 2.91 (s, 4 H); 4.92 (s, 2 H); 7.28 (s, 1 H); 7.53 (t, 1 H); 7.75 (d, 1 H); 8.10 (d, 1 H); 8.15 (s, 1 H).
MS: m/z = 265

### Step 8:

### ((S)-5-(tert-Butoxycarbonylamino)-5-(carbamoyl)pentyl)carbamic acid benzyl ester

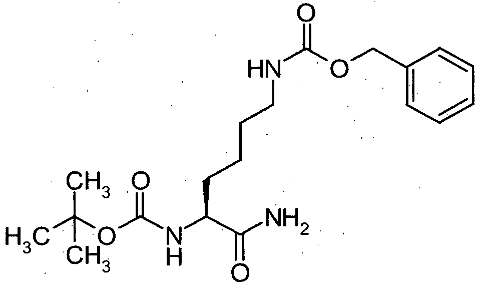

2,5-Dioxopyrrolidin-1-yl(S)-6-((benzyloxycarbonyl)amino)-2-((tert-butoxycarbonyl)amino)hexanoate (commercially available at e.g. Fluka or Bachem, 15. g, 31 mmol) was dissolved in dichloromethane (50 ml). A 25% solution of ammonia in water was added. The reaction mixture was stirred vigorously for 16 h at room temperature. The solvent was removd in vacuo to yield 21.27 g of crude ((*S*)-5-(tert-butoxycarbonylamino)-5-(carbamoyl)pentyl)carbamic acid benzyl ester, which was used in the next step without further purification.
¹H-NMR (DMSO-d₆): δ 1.2-1.6 (m, 6 H); 1.37 (s, 9 H); 2.95 (q, 2 H); 3.80 (td, 1 H); 5.00 (s, 2 H); 6.70 (d, 1 H); 6.90 (s, 1 H); 7.20-7.40 (m, 7 H).
MS: m/z = 280.

### Step 9:

### ((S)-5-Amino-1-(carbamoyl)pentyl)carbamic acid tert-butyl ester

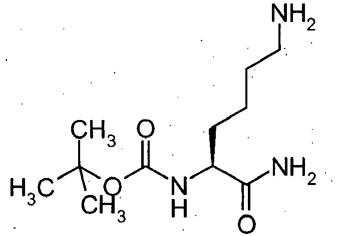

Crude ((*S*)-5-(tert-butoxycarbonylamino)-5-(carbamoyl)pentyl)carbamic acid benzyl ester (11.92 g, 31.41 mmol) was suspended in methanol (250 ml). Palladium on coal (50% wet) 1.67 g was added. The mixture was subjected to hydrogenation under pressure for 16 h. It was filtered through a plug of celite. The solvent was removed in vacuo to give 13.13 g of crude ((*S*)-5-amino-1-(carbamoyl)pentyl)carbamic acid tert-butyl ester, which was used in the next step without further purification.
¹H-NMR (DMSO-d₆): δ 1.30-1.60 (m, 6 H); 1.37 (s, 9 H); 2.65 (t, 2 H); 3.80 (dt, 1 H); 5.70 (br, 2 H); 6.80 (d, 1 H); 6.95 (s, 1 H); 7.30 (s, 1 H).

### Step 10:

### N-((S)-5-Amino-5-(carbamoyl)pentyl)-3-(aminooxymethyl)benzamide

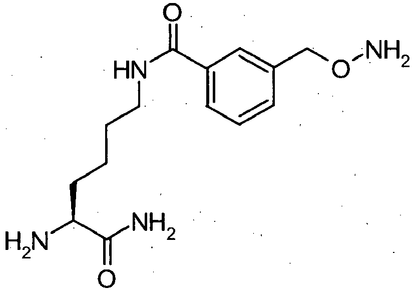

A solution of crude 2,5-dioxopyrrolidine-1 yl 3-(((tert-butoxycarbonyl)aminoxy)methyl)benzoate (11.45 g, 31.41 mmol) in *N,N*-dimethylformamide (100 ml) was added to a solution of ((*S*)-5-amino-1-(carbamoyl)pentyl)carbamic acid tert-butyl ester (7.71 g, 31.41 mmol) in *N,N*-dimethylformamide (50 ml). Ethyldiisopropylamine (16.13 ml, 94.23 mmol) was added. The reaction mixture was stirred at room temperature for 16 h. The solvent was removed in vacuo at 70°C. The residue was dissolved in dichloromethane (50 ml). Trifluoroacetic acid (50 ml) was added. The mixture was stirred for 1 h at room temperature. The solvent was removed in vacuo. The residue was dissolved in dichloromethane (100 ml). A 10% aqueous solution of sodium hydrogensulphate (50 ml) water (200 ml) were added successively. The aqueous phase was concentrated in vacuo to approximately 60 ml. This solution was divided into four parts. Each of them were subjected to a HPLC-chromatography on a C18 reversed phase column, using gradients of 0-20, 0-11, 0-9 or 0-2% respectively acetonitril in water in a buffer of 0.1 % trifluoroacetic acid to give together 4.38 g of N-((S)-5-amino-5-(carbamoyl)pentyl)-3-(aminooxymethyl)benzamide.
HPLC: Rₜ = 3.24 min (method 02-B1-2).
¹H-NMR (DMSO-d₆): δ 1.35 (m, 2 H); 1.55 (m, 2 H); 1.75 (m, 2 H); 3.25 (m, 2 H); 3.72 (m, 1 H); 5.05 (s, 2 H); 7.54 (m, 3 H); 7.88 (m, 3 H); 8.12 (br, 3 H); 8.55 (t, 1 H).
MS: m/z = 295

Step 11:

### 6-(3-(Aminoxymethyl)benzoylamino)-2-(([Glu3,Leu10]GLP-2yl)leucylamino)hexanoic amide

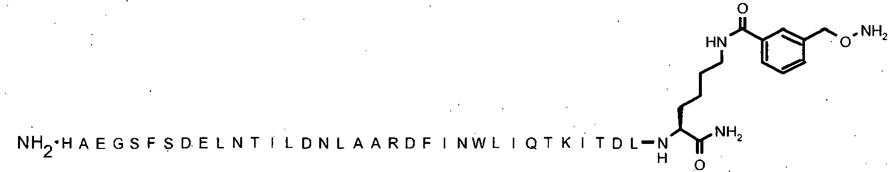

A solution of CPY (18 U in 0.090 ml water) was added to a solution of [Glu³,Leu¹⁰]GLP-2ylleuccinylalanine (7.1 mg, 1799 nmol), *N*-((S)-5-amino-5-(carbamoyl)pentyl)-3-(aminooxymethyl)benzamide (7.1 mg, 0.002 mmol) and beta-hyroxypropylcyclodextrin (71 mg) in a 250 mM HEPES/5 mM EDTA buffer (1.71 ml), in which the pH had been adjusted to 7.93 with an aqueous 1 N sodium hydroxide solution. The reaction mixture was left at room temperature for 2.5 h. It was diluted with water (18 ml) filtered and subjected to a HPLC-purification on a C₁₈-reversed phase column, using a gradient of 35-65% acetonitrile in water, which was buffered with 0.1 % trifluoroacetic acid as eluent to give 0.112 mg of 6-(3-(aminoxymethyl)benzoylamino)-2-(([Glu3,Leu10]GLP-2yl)leucylamino)hexanoic amide.
MS: m/z = 1385, 1039, 831, 693
HPLC: Rₜ = 8.97 min (method 02-B4-4).

### Step 12:

### [Glu¹⁸⁰,Asp¹⁸²,Glu¹⁸⁹]hGH(180-191)ylleucylalanine

[Glu¹⁸⁰,Asp¹⁸²,Glu¹⁸⁹]hGH(180-191)ylleucylalanine was prepared on an Applied Biosystems 433A Peptide Synthesizer by standard Fmoc-strategy, known to a person skilled in the art, starting with a commercially available Fmoc-Ala-Wang resin. Following amino acid derivatives were used:

| coupling no. | amino acid derivative |
|---|---|
| 1 | Fmoc-Leu-OH |
| 2 | Fmoc-Phe-OH |
| 3 | Fmoc-Gly-OH |
| 4 | Fmoc-Glu(OtBu)-OH |
| 5 | Fmoc-Ser(tBu)-OH |
| 6 | Fmoc-Gly-OH |
| 7 | Fmoc-Glu(OtBu)-OH |
| 8 | Fmoc-Val-OH |
| 9 | Fmoc-Ser(tBu)-OH |
| 10 | Fmoc-Arg(Pmc)-OH |
| 11 | Fmoc-Asp(OtBu)-OH |
| 12 | Fmoc-Gln(tBu)-OH |
| 13 | Fmoc-Glu(OtBu)-OH |

A mixture of trifluoroacetic acid (10 ml), water (0.265 ml) and triisopropylsilane (0.265 ml) was added to the resin. It was shaken for 1.5 h. The liquid was collected. The resin was washed with trifluoroacetic acid (1 ml). The liquids were combined. The solution was concentrated under a stream of nitrogen. Ether (40 ml) was added. The precipitation was isolated by centrifugation. The crude product was purified on a reversed phase C₁₈-column on a HPLC, using a gradient of 10-45% acetonitrile in water in a buffer of 0.1% trifluoroacetic acid, as eluent.
MS: m/z = 764, 1523
HPLC: Rₜ = 4.84 min (method 02-B4-4)

### Step 13:

### (S)-2-([Glu¹⁸⁰,Asp¹⁸²,Glu¹⁸⁹]hGH(180-191)ylleucylamino)-3-(4-(prop-2-ynyloxy)phenyl)propionic amide

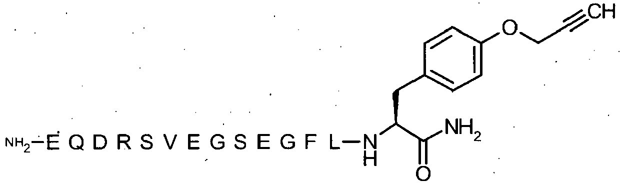

A solution of [Glu¹⁸⁰,Asp¹⁸²,Glu¹⁸⁹]hGH(180-191)ylleucylalanine (8 mg, 0.00525 mmol) and (2S)-2-Amino-3-(4-(prop-2-ynyloxy)phenyl)propionamide (261 mg, 0.788 mmol) was prepared in a buffer of 250 mM HEPES/5 mM EDTA and adjusted with 1 N sodium hydroxide to pH 7.93. The total volume of this solution was 5 ml. A solution of CPY (52 U) in water (0.26 ml) was added to it. The reaction mixture was kept at room temperature for 2.5 h. It was diluted with water (15 ml), filtered, and purified by HPLC-chormatography on a C₁₈-reversed phase column, using a gradient of 22-57% acetonitrile in water, which was buffered with 0.1 % trifluoroacetic acid, to give 2.75 mg of (*S*)-2-([Glu¹⁸⁰,Asp¹⁸²,Glu¹⁸⁹]hGH(180-191)ylleucylamino)-3-(4-(prop-2-ynyloxy)phenyl)propionic amide.
MS: m/z = 827 (M²⁺)
HPLC: Rₜ = 6.10 min (method 02-B4-4).

### Step 14:

### 3-Acetylbenzoic acid 2,5-dioxopyrrolidin-1-yl ester

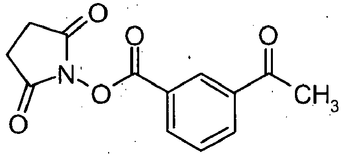

O-(N-Succinimidyl)-N,N-N',N'-tetrmethyluronium tetrafluoroborate (5.50 g, 18.3 mmol) was added to a solution of 3-acetylbenzoic acid (3.00 g, 18.3 mmol) and triethylamine (2.55 ml, 18.3 mmol) in *N,N*-dimethylformamide (25 ml). The reaction mixture was stirred for 16 h at room temperature. It was diluted with water (300 ml) and extracted with ethyl acetate (2 x 150 ml). The combined organic layers were dried over magnesium sulphate. The solvent was removed in vacuo. The crude product was crystallized from ethyl acetate/heptane to give 3.23 g of 3-acetylbenzoic acid 2,5-dioxopyrrolidin-1-yl ester. ¹H-NMR (CDCl₃) δ 2.67 (s, 3 H); 2.94 (s, 4 H); 7.66 (t, 1 H); 8.32 (m, 2 H); 8.69 (s, 1 H).

### Step 15:

### 3-Acetyl-N-[2-(2-{2-[2-(2-{2-[2-(2-{2-[2-(2-azidoethoxy)ethoxy]ethoxy}ethoxy)ethoxy]ethoxy}-ethoxy)ethoxy]ethoxy}ethoxy)ethyl]benzamide

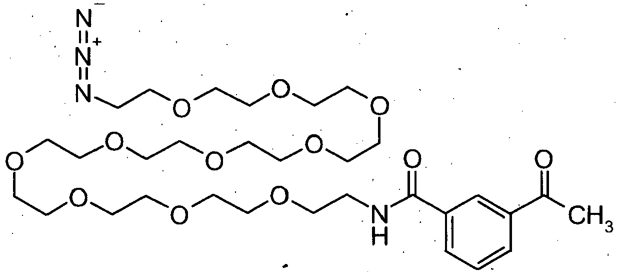

3-Acetylbenzoic acid 2,5-dioxopyrrolidin-1-yl ester (260 mg, 0.997 mmol) was added to a solution of commercially available *N*-[2-(2-{2-[2-(2-{2-[2-(2-{2-[2-(2-azidoethoxy)-ethoxy]ethoxy}ethoxy)ethoxy]ethoxy}ethoxy)ethoxy]ethoxy}ethoxy)ethyl]amine (Fluka, 500 mg, 0.949 mmol) and ethyldiisopropylamine (0.325 ml, 1.90 mmol) in dichloromethane (6 ml). The reaction mixture was stirred for 3 days at room temperature. It was diluted with dichloromethane (30 ml) and was washed successively with a 10% aqueous solution of sodium hydrogensulphate (30 ml) and a saturated aqueous solution of sodium hydrogencarbonate (30 ml). It was dried over sodium sulphate. The solvent was removed in vacuo to give 609 mg of 3-acetyl-N-[2-(2-{2-[2-(2-{2-[2-(2-{2-[2-(2-azidoethoxy)ethoxy]-ethoxy}ethoxy)ethoxy]ethoxy}ethoxy)ethoxy]ethoxy}ethoxy)ethyl]benzamide.
¹H-NMR (CDCl₃) δ 2.67 (s, 3 H); 3.39 (t, 2 H); 3.65 (m, 42 H); 7.30 (br, 1 H); 7.55 (t, 1 H); 8.08 (m, 2 H); 8.43 (s, 1 H).
MS: m/z = 673

Step 16:

### (S)-3-(4-((1-(2-(2-(2-(2-(2-(2-(2-(2-(2-(2-(2-(4-(Acetyl)benzoylamino)ethoxy)ethoxy)ethoxy)-ethoxy)ethoxy)ethoxy)ethoxy)ethoxy)ethoxy)ethoxy)ethyl)1,2,3-triazol-4-yl)methoxy)phenyl)-2-(([Glu¹⁸⁰,Asp¹⁸²,Glu¹⁸⁹]hGH(180-191)yl)leucylamino)propionic amide

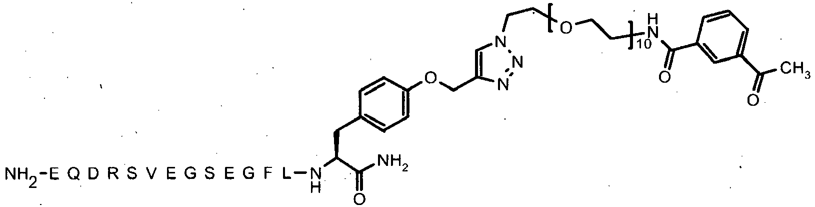

A solution of copper(II) sulphate pentahydrate (10 mg) in water (1 ml) was prepared. This solution was added to a solution of L-(+)-ascorbic acid (36.7 mg) in water (1 ml) and 2,6-lutidine (0.025 ml). This solution was left for 5 min. 0.831 ml of it was added to a solution of (*S*)-2-([Glu¹⁸⁰,Asp¹⁸²,Glu¹⁸⁹]hGH(180-191)ylleucylamino)-3-(4-(prop-2-ynyloxy)-phenyl)propionic amide (2.75 mg, 0.002 mmol) and 3-acetyl-N-[2-(2-{2-[2-(2-{2-[2-(2-{2-[2-(2-azidoethoxy)ethoxy]ethoxy}ethoxy)ethoxy]ethoxy}ethoxy)ethoxy]ethoxy}ethoxy)ethyl]-benzamide (11.2 mg, 0.017 mmol) in a buffer consisting of 2% 2,6-lutidine in water (2.55 ml). The reaction mixture was left for 16 h at room temperature. It was diluted with water (10 ml). (S)-3-(4-((1-(2-(2-(2-(2-(2-(2-(2-(2-(2-(2-(2-(4-(Acetyl)benzoylamino)ethoxy)ethoxy)ethoxy)-ethoxy)ethoxy)ethoxy)ethoxy)ethoxy)ethoxy)ethoxy)ethyl)1,2,3-triazol-4-yl)methoxy)phenyl)-2-(([Glu¹⁸⁰,Asp¹⁸²,Glu¹⁸⁹]hGH(180-191)yl)eucylamino)propionic amide (2.44 mg) was isolated by HPLC-chromatography on a C₁₈-reversed phase column, using a gradient of 22-56% acetonitrile in water, which was buffered with 0.1% trifluoroacetic acid, as eluent.
MS: m/z = 1163, 776, 582.
HPLC: Rₜ = 6.66 min (method 02-B4-4).

### Step 17:

A solution of (S)-3-(4-((1-(2-(2-(2-(2-(2-(2-(2-(2-(2-(2-(2-(4-(acetyl)benzoylamino)-ethoxy)ethoxy)ethoxy)ethoxy)ethoxy)ethoxy)ethoxy)ethoxy)ethoxy)ethoxy)ethyl)1,2,3-triazol-4-yl)methoxy)phenyl)-2-(([Glu¹⁸⁰,Asp¹⁸²,Glu¹⁸⁹]hGH(180-19)yl)leucylamino)propionic amide (0.63 mg, 270 nmol) in a MES-buffer (0.054 ml), which was adjusted to pH 5.7. This solution was added to 6-(3-(aminoxymethyl)benzoylamino)-2-(([Glu3,Leu10]GLP-2yl)leucylamino)-hexanoic amide (0.112 mg, 27 nmol). MES buffer (0.05 ml), which had been adjusted to pH 5.7, was added to the reaction mixture. The reaction mixture was left for 16 h at room temperature. It was kept at -18°C, until the (S)-2-(([Glu³,Leu¹⁰]GLP-2yl)leucylamino)-6-(3-(((1-(3-((2-(2-(2-(2-(2-(2-(2-(2-(2-(2-(2-(4-((4-((S)-2-(carbamoyl)-2-(([Glu¹⁸⁰,Asp¹⁸²,Glu¹⁸⁹]hGH(180-191)yl)leucylamino)ethyl)phenoxy)methyl)1,2,3-triazol-1-yl)ethoxy)ethoxy)ethoxy)ethoxy)ethoxy)ethoxy)ethoxy)ethoxy)ethoxy)ethoxy)ethyl)-carbamoyl)phenyl)ethylidene)aminoxy)methyl)benzoylamino)hexanoic amide was isolated by HPLC-chromatography on a C₁₈-reversed phase column, using a gradient of 35-67% acetonitrile in water, which was buffered with 0.1% trifluoroacetic acid.
MS: m/z = 719, 806, 923, 1078, 1293
HPLC: Rₜ = 8.72 min (method 02-B4-4).

### Example 2

### (S-)-2-(((((((Glutamyl)aspartyl)asparaginyl)glutamyl)phenylalanyl)leucyl)leucylamino)-6-(3-((4-((3-((2-(2-(2-(2-(2-(2-(2-(2-(2-(4-(1-((3-(((S)-5-carbamoyl-5-(((((((glutamyl)aspartyl)-arparaginyl)glutamyl)phenylalanyl)phenylalanyl)leucylamino)pentyl)carbamoyl)benzyloxy)-imino)ethyl)benzoylamino)ethoxy)ethoxy)ethoxy)ethoxy)ethoxy)ethoxy)ethoxy)ethoxy)-ethoxy)ethyl)carbamoyl)phenoxy)methyl)1,2,3-triazol-1-yl)methyl)benzoylamino)hexanoic amide

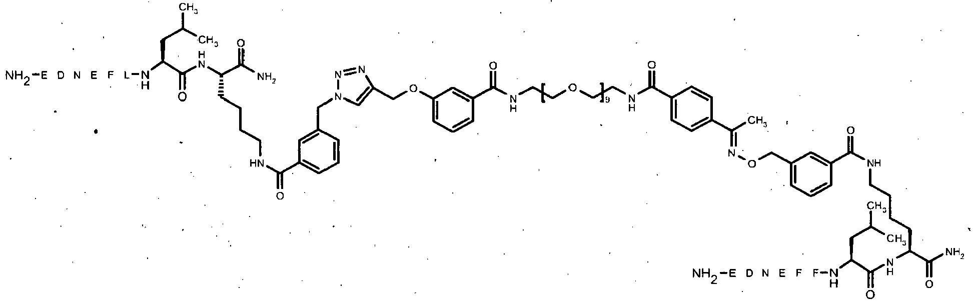

### Step 1:

### (((((((Glutamyl)aspartyl)asparaginyl)glutamyl)phenylalanyl)leucyl)leucyl)alanine

(((((((Glutamyl)aspartyl)asparaginyl)glutamyl)phenylalanyl)leucyl)leucyl)alanine was prepared on an Applied Biosystems 433A Peptide Synthesizer by standard Fmoc-strategy, known to a person skilled in the art, starting with a commercially available Fmoc-Ala-Wang resin. Following amino acid derivatives were used:

| coupling no. | amino acid derivative |
|---|---|
| 1 | Fmoc-Leu-OH |
| 2 | Fmoc-Leu-OH |
| 3 | Fmoc-Phe-OH |
| 4 | Fmoc-Glu(OtBu)-OH |
| 5 | F moc-Asn(Trt)-OH |
| 6 | Fmoc-Asp(OtBu)-OH |
| 7 | Fmoc-Glu(OtBu)-OH |

A mixture of trifluoroacetic acid (10.6 ml), water (0.265 ml) and triisopropylsilane (0.265 ml) was added to the resin. It was shaken for 1.5 h. The liquid was collected. The resin was washed with trifluoroacetic acid (1 ml). The liquids were combined. The solution was concentrated under a stream of nitrogen. Ether (40 ml) was added. The precipitation was isolated by centrifugation. The crude product was purified on a reversed phase C₁₈-column on a HPLC, using a gradient of 18-38% acetonitrile in water in a buffer of 0.1 % trifluoroacetic acid, as eluent.
MS: m/z = 950
HPLC: Rₜ = 18.79 min (method 02-B6-1).

Step 2:

### (((((((Glutamyl)aspartyl)asparaginyl)glutamyl)phenylalanyl)phenylalanyl)leucyl)alanine

(((((((Glutamyl)aspartyl)asparaginyl)glutamyl)phenylalanyl)phenylalanyl)leucyl)-alanine was prepared on an Applied Biosystems 433A Peptide Synthesizer by standard Fmoc-strategy, known to a person skilled in the art, starting with a commercially available Fmoc-Ala-Wang resin. Following amino acid derivatives were used:

| coupling no. | amino acid derivative |
|---|---|
| 1 | Fmoc-Leu-OH |
| 2 | Fmoc-Phe-OH |
| 3 | Fmoc-Phe-OH |
| 4 | Fmoc-Glu(OtBu)-OH |
| 5 | Fmoc-Asn(Trt)-OH |
| 6 | Fmoc-Asp(OtBu)-OH |
| 7 | Fmoc-Glu(OtBu)-OH |

A mixture of trifluoroacetic acid (10.6 ml), water (0.265 ml) and triisopropylsilane (0.265 ml) was added to the resin. It was shaken for 1.5 h. The liquid was collected. The resin was washed with trifluoroacetic acid (1 ml). The liquids were combined. The solution was concentrated under a stream of nitrogen. Ether (40 ml) was added. The precipitation was isolated bv centrifugation. The crude product was purified on a reversed phase C₁₈-column on a HPLC, using a gradient of 2-30% acetonitrile in water in a buffer of 0.1 % trifluoroacetic acid, as eluent.
MS: m/z = 984
HPLC: Rₜ = 19.40 min (method 03-B6-1).

### Step 3:

### Methyl 3-(azidomethyl)benzoate

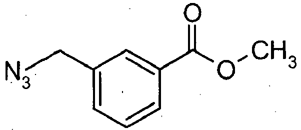

Sodium azide (5.68 g, 87 mmol) was added to a solution of methyl 3-(bromometyl)benzoate (5.00 g, 22 mmol) in *N,N*-dimethylformamide (50 ml). Tetrabutylammonium iodide (81 mg, 0.22 mmol) was added. The reaction mixture was heated to 60°C for 16 h. It was cooled to room temperature and given onto water (200 ml). This mixture was extracted with ethyl acetate (400 ml). The organic layer was washed with water (3 x 200 ml) and successively dried over sodium sulphate. The solvent was removed in vacuo to give 4.11 go of crude methyl 3-(azidomethyl)benzoate, which was used without further purification.
MS: m/z = 192.
¹H-NMR (CDCl₃): δ 3.92 (s, 3 H); 4.40 (s, 2 H); 7.50 (m, 2 H); 8.00 (m, 2 H).

### Step 4:

### 3-(Azidomethyl)benzoic acid

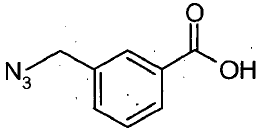

A solution of lithium hydroxide (3.81 g, 21.5 mmol) in water (25 ml) was added to a solution of crude methyl 3-(azidomethyl)benzoate (4.11 g, 21.5 mmol) in 1,4-dioxane (25 ml). Water and 1,4-dioxane was added until a clear solution was obtained. The reaction mixture was stirred for 16 h at room temperature. An 1 N aqueous solution of sodium hydroxide (100 ml) was added. The reaction mixture was washed with tert-butyl methyl ether (2 x 100 ml). The aqueous phase was acidified with a 10% aqueous solution of sodium hydrogensulphate. It was extracted with ethyl acetate (2 x 200 ml). The combined ethyl acetate phases were dried over magnesium sulphate. The solvent was removed in vacuo to give 3.68 g of crude 3-(azidomethyl)benzoic acid, which was used without further purification.
MS: m/z = 150
¹H-NMR (CDCl₃): δ 4.57 (s, 3 H); 7.55 (m, 2 H); 8.00 (m, 2 H); 13.10 (br, 1 H).

### Step 5:

### (S)-2-Amino-6-(3-(azidomethyl)benzoylamino)hexanoic amide

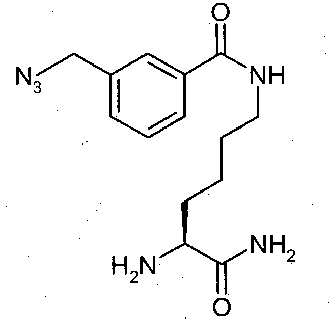

Rink Amide-resin (Novabiochem 01-64-0013, loading 0.70 mmol/g, 0.652 g, 2.7 mmol) was swelled in dichloromethane (50 ml). The solvent was removed. A 20% solution of piperidine in *N,N*-dimethylformamide (50 ml) was added. The mixture was shaken for 20 min at room temperature. The solvent was removed. The resin was washed with N-methylpyrrolidinone (3x 50 ml) and dichloromethane (5 x 50 ml). A solution of Boc-Lys(FMOC)-OH (5.00 g, 10.7 mmol) in N-methylpyrrolidinone (12.5 ml) and a solution of 1-hydroxybenzotriazole (1.63 g, 10.7 mmol) were added successively to the resin. A solution of diisopropylcarbodiimide (1.67 ml, 10.7 mmol) in dichloromethane (25 ml) was added. Ethyldiisopropylamine (1.83 ml, 10.7 mmol) was added. The reaction mixture was shaken for 2 days at room temperature. The liquid was removed. The resin was washed with *N-*methylpyrrolidinone (3x 50 ml) and dichloromethane (5 x 50 ml). A 20% solution of piperidine in *N,N*-dimethylformamide (50 ml) was added. The mixture was shaken for 20 min at room temperature. The solvent was removed. The resin was washed with *N*-methylpyrrolidinone (3x 50 ml) and dichloromethane (5 x 50 ml). A solution of the crude 3-(azidomethyl)benzoic acid (1.89 g, 10.7 mmol) in *N*-methylpyrrolidinone (12.5 ml) and dichloromethane (12.5 ml) was added, followed by a solution of 1-hydroxybenzotriazole (1.63 g, 10.7 mmol) in *N-*methylpyrrolidinone (12.5 ml). A solution of diisopropylcarbodiimide (1.67 ml, 10.7 mmol) in dichloromethane (12.5 ml) was added. Ethydiisopropylamine (1.83 ml, 10.7 mmol) was added. The reaction mixture was shaken for 16 h at room temperature. The liquid was removed. The resin was washed with *N*-methylpyrrolidinone (3x 50 ml) and dichloromethane (5 x 50 ml). A 50% solution of trifluoroacetic acid in dichloromethane (20 ml) was added to the resin. Triisopropylsilane (5 ml) was added. The reaction mixture was shaken for 1 h at room temperature. The liquid was collected. The resin was washed with dichloromethane (30 ml). These two latter liquids were combined. The solvent was removed in vacuo. The crude product was purified by HPLC-chromatography on a C₁₈-reversed-phase column, using a gradient of 13-33% acetonitrile in water, which was acidified by addition of 0.1% trifluoroacetic acid to give 300 mg of the trifluoroacetate salt of (*S*)-2-amino-6-(3-(azidomethyl)benzoylamino)hexanoic amide.
MS: m/z = 305
¹H-NMR (DMSO-d₆, trifluoroacetate salt): δ 1.37 (m, 2 H); 1.55 (m, 2 H); 1.77 (m, 2 H); 3.28 (m, 2 H); 3.71 (t, 1 H); 4.53 (s, 2 H); 7.51 (m, 3 H); 7.84 (m, 3 H); 8.10 (br, 3 H); 8.54 (t, 1 H).

Step 6:

### (S)-6-(3-(Azidomethyl)benzoylamino)-2-(((((((glutamyl)aspartyl)asparaginyl)glutamyl)-phenylalanyl)leucyl)leucylamino)hexanoic amide

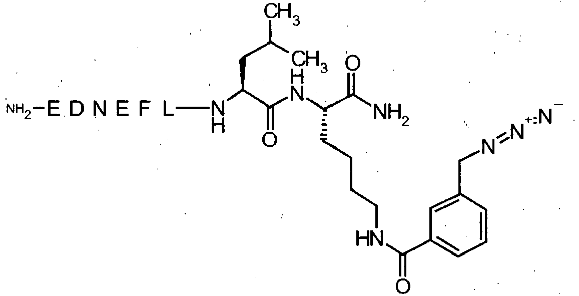

A solution of CPY (100 U) in water (0.5 ml) was added to a solution of (((((((glutamyl)aspartyl)asparaginyl)glutamyl)phenylalanyl)leucyl)leucyl)alanine (14 mg, 0.015 mmol) and (S)-2-amino-6-(3-(azidomethyl)benzoylamino)hexanoic amide (520 mg, 1.25 mmol) in a buffer of 250 mM HEPES/ 5 mM EDTA (4.50 ml), which had been adjusted to pH 7.99 with 1 N hydrochloric acid and 1 N aqueous sodium hydroxide. The reaction mixture was left at 30°C for 16 h. It was diluted with water (10 ml). (*S*)-6-(3-(Azidomethyl)-benzoylamino)-2-(((((((glutamyl)aspartyl)asparaginyl)glutamyl)phenylalanyl)leucyl)-leucylamino)hexanoic amide (4.6 mg) was isolated by reversed HPLC-chromatography on a C₁₈-column, using a gradient of 0-50% acetonitrile in water, which was buffered with 0.1 % trifluoroacetic acid.
MS: m/z = 1166
HPLC: Rₜ = 33.39 min (method 03-B1-1).

Step 7:

### (S)-6-(3-(Aminoxymethyl)benzoylamino)-2-(((((((glutamyl)aspartyl)asparaginyl)glutamyl)-phenylalanyl)phenylalanyl)leucylamino)hexanoic amide

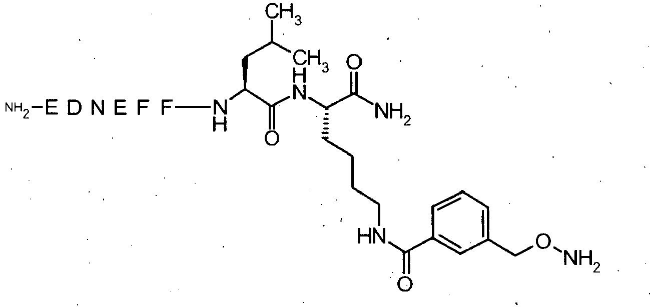

A solution of CPY (80 U) in water (0.400 ml) was added to a solution of (((((((gutamyl)aspartyl)asparaginyl)glutamyl)phenylalanyl)phenylalanyl)leucyl)alanine (25 mg, 0.025 mmol) and *N*-((S)-5-amino-5-(carbamoyl)pentyl)-3-(aminooxymethyl)benzamide (979 mg, 2.4 mmol) in a buffer or 250 mM HEPES/5 mM EDTA (7.10 ml), which was adjusted to pH 8.24 with 1 N hydrochloric acid and 1 N aqueous sodium hydroxide. The reaction mixture was left at 30°C for 2 h. It was diluted with water (10 ml). (*S*)-6-(3-(Aminoxymethyl)benzoylamino)-2-(((((((glutamyl)aspartyl)asparaginyl)glutamyl)-phenylalanyl)phenylalanyl)leucylamino)hexanoic amide (2.89 mg) was isolated by reversed HPLC-chromatography C₁₈-column, using a gradient of 0-40% acetonitrile in water, which was buffered with 0.1 % trifluoroacetic acid.
MS: m/z = 1189
HPLC: Rₜ = 20.20 min (method 03-B6-1).

### Step 8

### 3-(Prop-2-ynyloxy)benzoic acid ethyl ester

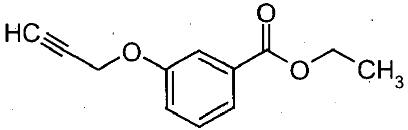

3-Bromoprop-1-yne (4.49 ml, 50.5 mmol) was added to a mixture of potassium carbonate (46.58 g, 336 mmol) and ethyl 3-hydroxybenzoate (7 g, 42 mmol) in *N,N-*dimethylformamide. The mixture was heated to 60°C for 15 min. Tetrabutylammonium iodide (0.78 g, 2.1 mmol) was added. The mixture was heated to 60°C for 16 h and cooled to room temperature. It was acidified with a 10% aqueous sodium hydrogensulphate solution (1 l) and extracted with ethyl actate (2 x 150 ml). The combined organic layers were wahed with a saturated aqueous solution of sodium hydrogencarbonate (300 ml) and dried over magnesium sulphate. The solvent was removed in vacuo. The crude product was purified by flash-chromatography on silica (120 g), using a mixture of ethyl acetate/heptane 1:4 as eluent, to give 7.3 g of 3-(prop-2-ynyloxy)benzoic acid ethyl ester.
¹H-NMR (CDCl₃) δ 1.40 (t, 3 H); 2.55 (s 1 H); 4.40 (q, 2 H); 4.75 (s, 2 H); 7.17 (d, 1 H); 7.37 (t, 1 H); 7.66 (s, 1 H); 7.70 (d, 1 H).

### Step 9

### 3-(Prop-2-ynyloxy)benzoic acid

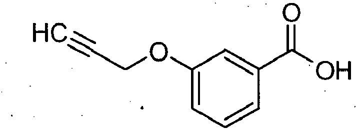

A solution of lithium hydroxide (1.65 g, 39.4 mmol) in water (25 ml) was added to a solution of 3-(prop-2-ynyloxy)benzoic acid ethyl ester (7.30 g, 35.8 mmol) in dioxane (25 ml). Water and dioxane were added, until a clear solution was obtained. The reaction mixtue was stirred at room temperature for 16 h. A 1 N aqueous solution of sodium hydroxide (50 ml) was added. This mixture was washed with tert-butyl methyl ether (2 x 120 ml). The aqueous phase was acidified with a 10% aqueous solution of sodium hydrogen sulphate to pH 2. It was extracted with ethyl acetate (2 x 200 ml). The combined ethyl acetate layers were dried over magnesium sulphate. The solvent was removed in vacuo to give 5.9 g of 3-(prop-2-ynyloxy)benzoic acid.
¹H-NMR (CDCl₃) δ 2.55 (s, 1 H); 4.77 (s, 2 H); 7.25 (d, 1 H); 7.42 (t, 1 H); 7.71 (s, 1 H); 7.77 (d, 1 H).

### Step 10

### 3-Prop-2-ynyloxy-benzoic acid 2,5-dioxopyrrolidin-1-yl ester

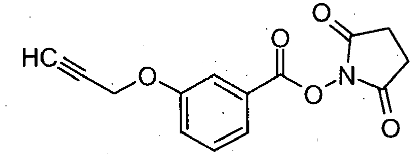

2-Succinimido-1,1,3,3-tetramethyluronium tetrafluoroborate (TSTU, 4.75 g, 15.67 mmol) was added to a solution of 3-(prop-2-ynyloxy)benzoic acid (2.30 g, 13.06 mmol) and triethylamine (2.18 ml, 15.67 mmol) in *N,N*-dimethylformamide. The reaction mixture was stirred at room temperature for 16 h. It was diluted with ethyl acetate (500 ml) and washed with water (3 x 200 ml) and a saturated aqueous solution of sodium hydrogencarbonate (200 ml). It was dried over sodium sulphate. The solvent was removed in vacuo to give 3.22 g of 3-prop-2-ynyloxy-benzoic acid 2,5-dioxopyrrolidin-1-yl ester.
¹H-NMR (CDCl₃) δ 2.55 s, 1 H; 2.91 (s, 4 H); 4.75 (s, 2 H); 7.30 (d, 1 H); 7.45 (t, 1 H); 7.70 (s, 1 H); 7.80 (d, 1 H).
MS; m/z = 297 (M + Na)⁺

### Step11

### N-(2-(2-(2-(2-(2-(2-(2-(2-(2-(2-(tert-Butoxycarbonylamino)ethoxy)ethoxy)ethoxy)ethoxy)-ethoxy)ethoxy)ethoxy)ethoxy)ethoxy)ethyl)-3-(prop-2-ynyloxy)benzoic amide

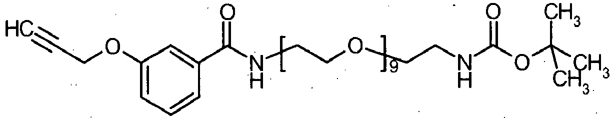

2-{2-[2-(2-{2-[2-(2-{2-[2-(2-Aminoethoxy)ethoxy]ethoxy}ethoxy)ethoxy]ethoxy}-ethoxy)ethoxy]ethoxy}ethyl)carbamic acid tert-butyl ester (commercially available at Fluka, 1.0 g, 1.55 mmol) and ethyldiisopropylamine (0.88 ml, 5.2 mmol) were added successively to a solution of 3-prop-2-ynyloxy-benzoic acid 2,5-dioxopyrrolidin-1-yl ester (470 mg, 1.72 mmol) in dichloromethane (30 ml). The reaction mixture was stirred for 16 h at room temperature. The solvent was removed in vacuo. The residue was dissolved in dichloromethane (50 ml) and washed with a saturated aqueous solution of sodium hydrogencarbonate (20 ml). The organic phase was dried over sodium sulphate. The solvent was removed in vacuo.

Amberlyst 15 (0.5 g) was dissolved in dichloromethane (10 ml). Ethanol (1 ml) was added. The mixture was gently stirred for 10 min. The solvent was removed by filtration. The resin was washed with dichloromethane (10 ml).

The crude product from the reaction mixture was dissolved in dichloromethane (10 ml) and ethanol (1 ml). The amberlyst 15 resin was added. The mixture was gently stirred for 30 min. The resin was removed by filtration. The solvent was removed in vacuo from the filtrate to give 1.21 g of *N*-(2-(2-(2-(2-(2-(2-(2-(2-(2-(2-(tert-butoxycarbonylamino)ethoxy)-ethoxy)ethoxy)ethoxy)ethoxy)ethoxy)ethoxy)ethoxy)ethoxy)ethyl)-3-(prop-2-ynyloxy)benzoic amide.

### Step 12 .

### N-(2-(2-(2-(2-(2-(2-(2-(2-(2-(2-aminoethoxy)ethoxy)ethoxy)ethoxy)ethoxy)ethoxy)ethoxy)-ethoxy)ethoxy)ethyl)-3-(prop-2-ynyloxy)benzoic amide

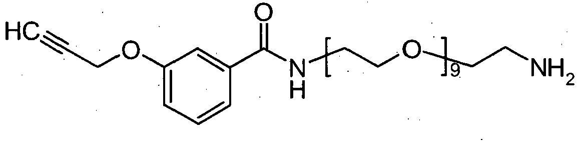

Trfiluoroacetic acid (10 ml) was added to a solution of *N*-(2-(2-(2-(2-(2-(2-(2-(2-(2-(2-(tert-butoxycarbonylamino)ethoxy)ethoxy)ethoxy)ethoxy)ethoxy)ethoxy)ethoxy)ethoxy)-ethoxy)ethyl)-3-(prop-2-ynyloxy)benzoic amide (1.01 g, 1.41 mmol) in dichloromethane (10 ml). The reaction mixture was stirred for 0.5 h at room temperature. The solvent was removed in vacuo to give 1.59 g of *N*-(2-(2-(2-(2-(2-(2-(2-(2-(2-(2-aminoethoxy)ethoxy)-ethoxy)ethoxy)ethoxy)ethoxy)ethoxy)ethoxy)ethoxy)ethyl)-3-(prop-2-ynyloxy)benzoic amide.

### Step 13

### 4-Acetylbenzoic acid 2,5-dioxopyrrolidin-1-yl ester

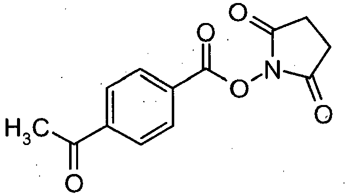

2-Succinimido-1,1,3,3-tetramethyluronium tetrafluoroborate (TSTU, 18.5 g, 60.9 mmol) was added to a solution of 4-acetylbenzoic acid (10.0 g, 60.9 mmol) and triethylamine (8.49 ml, 60.9 mmol) in *N,N*-dimethylformamide (50 ml). The reaction mixture was stirred for 2 h at room temperature. It was diluted with ethyl acetate (400 ml) and washed with water (3 x 200 ml). The organic layer was dried over sodium sulphate. The solvent was removed in vacuo to give 13.38 g of 4-acetylbenzoic acid 2,5-dioxopyrrolidin-1-yl ester.
¹H-NMR (CDCl₃): δ 2.67 (s, 3 H); 2.93 (s, 4 H); 8.05 (d, 2 H); 8.20 (d, 2 H).
MS: m/z = 284 (M+Na)⁺

### Step 14

### N-(2-(2-(2-(2-(2-(2-(2-(2-(2-(2-(4-acetylbenzoylamino)ethoxy)ethoxy)ethoxy)ethoxy)ethoxy)-ethoxy)ethoxy)ethoxy)ethoxy)ethyl)-3-(prop-2-ynyloxy)benzoic amide

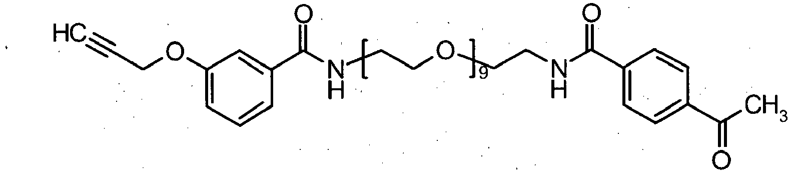

4-Acetylbenzoic acid 2,5-dioxopyrrolidin-1-yl ester (118 mg, 0.453 mmol) and ethyldiisopropylamine (1.06 ml, 6.17 mmol) were given successively to a solution of the crude *N*-(2-(2-(2-(2-(2-(2-(2-(2-(2-(2-aminoethoxy)ethoxy)ethoxy)ethoxy)ethoxy)ethoxy)-ethoxy)ethoxy)ethoxy)ethyl)-3-(prop-2-ynyloxy)benzoic amide in dichloromethane (10 ml). The reaction mixture was stirred for 16 h at room temperature. It was diluted with dichloromethane (20 ml), washed successively with a 10% aqueous solution of sodium hydrogensulphate (50 ml) and a saturated aqueous solution of sodium hydrogencarbonate (50 ml). The solvent was removed in vacuo to give 290 mg of crude *N*-(2-(2-(2-(2-(2-(2-(2-(2-(2-(2-(4-acetylbenzoylamino)ethoxy)ethoxy)ethoxy)ethoxy)ethoxy)ethoxy)ethoxy)ethoxy)-ethoxy)ethyl)-3-(prop-2-ynyloxy)benzoic amide.
MS: m/z = 761, 381 (M²⁺).

### Step 15

### (S)-6-(3-((4-((3-((2-(2-(2-(2-(2-(2-(2-(2-(2-(2-(4-(Acetyl)benzoylamino)ethoxy)ethoxy)ethoxy)-ethoxy)ethoxy)ethoxy)ethoxy)ethoxy)ethoxy)ethyl)carbamoyl)phenoxy)methyl)1,2,3-triazol-1-yl)methyl)benzoylamino)-2-(((((((glutamyl)aspartyl)asparaginyl)glutamyl)phenylalanyl)leucyl)-leucylamino)hexanoic amide

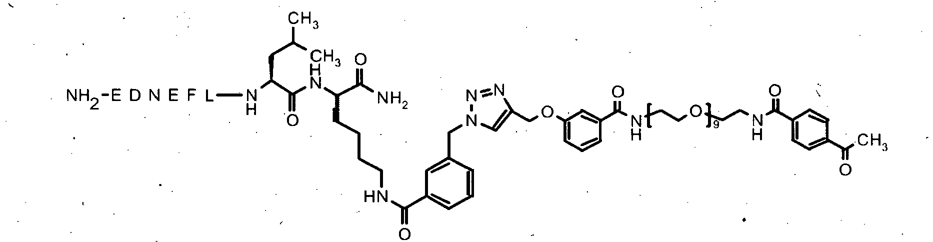

A solution of copper(II) sulphate pentahydrate (49.3 mg) in water (10.97 ml) was added to a solution of ascorbic acid (173.81 mg) in a mixture of water (10.75 ml) and 2,6-lutidine (0.219 ml). The mixture was kept at room temperature for 5 min. From this solution, 2.20 ml were added to a solution of (*S*)-6-(3-(azidomethyl)benzoylamino)-2-(((((((glutamyl)-aspartyl)asparaginyl)glutamyl)phenylalanyl)leucyl)leucylamino)hexanoic amide (2.3 mg, 0.002 mmol) and crude *N*-(2-(2-(2-(2-(2-(2-(2-(2-(2-(2-(4-acetylbenzoylamino)ethoxy)-ethoxy)ethoxy)ethoxy)ethoxy)ethoxy)ethoxy)ethoxy)ethoxy)ethyl)-3-(prop-2-ynyloxy)benzoic amide (15 mg, 0.02 mmol) in a mixture of water (1.73 ml) and 2,6-lutidine (0.02 ml). The reaction mixture was gently shaken for 16 h at room temperature. It was diluted with water (12 ml). (*S*)-6-(3-((4-((3-((2-(2-(2-(2-(2-(2-(2-(2-(2-(2-(4-(Acetyl)benzoylamino)ethoxy)-ethoxy)ethoxy)ethoxy)ethoxy)ethoxy)ethoxy)ethoxy)ethoxy)ethyl)carbamoyl)phenoxy)-methyl)1,2,3-triazol-1-yl)methyl)benzoylamino)-2-(((((((glutamyl)aspartyl)asparaginyl)-glutamyl)phenylalanyl)leucyl)leucylamino)hexanoic amide (0.78 mg) was isolated by reversed phase HPLC on a C₁₈-column, using a gradient of 10-50% acetonitrile in water, which was buffered with 0.1 % trifluoroacetic acid, as eluent.
MS: m/z = 963 (M²⁺)
HPLC: Rₜ = 26.47 (method 03-B6-1).

### Step 16

(*S*)-6-(3-((4-((3-((2-(2-(2-(2-(2-(2-(2-(2-(2-(2-(4-(Acetyl)benzoylamino)ethoxy)-ethoxy)ethoxy)ethoxy)ethoxy)ethoxy)ethoxy)ethoxy)ethoxy)ethyl)carbamoyl)phenoxy)-methyl)1,2,3-triazol-1-yl)methyl)benzoylamino)-2-(((((((glutamyl)aspartyl)asparaginyl)-glutamyl)phenylalanyl)leucyl)leucylamino)hexanoic amide (0.78 mg, 405 nmol) was dissolved in water (0.40 ml) and ethyldiisopropylamine (0.001 ml). This solution was added to a solution of (*S*)-6-(3-(Aminoxymethyl)benzoylamino)-2-(((((((glutamyl)aspartyl)-asparaginyl)glutamyl)phenylalanyl)phenylalanyl)leucylamino)hexanoic amide (1.2 mg, 1012 nmol) in water (0.40 ml) and ethyldiisopropylamine (0.002 ml). Acetic acid was added until pH 4.3 was obtained. Ethyldiisopropylamine (0.005 ml) and a 4 M aqueous solution of sodium hydroxide (0.015 ml) were added to obtain pH 10.74. The reaction mixture was shaken gently for 1.5 h at 30°C. Water (2.5 ml) was added. After 0.5 h more, (S)-2-(((((((glutamyl)aspartyl)asparaginyl)glutamyl)phenylalanyl)leucyl)leucylamino)-6-(3-((4-((3-((2-(2-(2-(2-(2-(2-(2-(2-(2-(2-(4-(1-((3-(((S)-5-carbamoyl-5-(((((((glutamyl)aspartyl)-arparaginyl)glutamyl)phenylalanyl)phenylalanyl)leucylamino)pentyl)carbamoyl)benzyloxy)-imino)ethyl)benzoylamino)ethoxy)ethoxy)ethoxy)ethoxy)ethoxy)ethoxy)ethoxy)ethoxy)-ethoxy)ethyl)carbamoyl)phenoxy)methyl)1,2,3-triazol-1-yl)methyl)benzoylamino)hexanoic amide (0.3 mg) was isolated by reversed phase HPLC on a C₁₈-column, using a gradient of 25-65% acetonitrile in water, which was buffered with 0.1 % TFA, as eluent.
MS: m/z = 1033 (M³⁺), 775 (M⁴⁺)
HPLC: Rₜ = 28.12 (method 03-B6-1).

### Example 3

### Alternative production of (S)-2-Amino-6-(3-(azidomethyl)benzoylamino)hexanoic amide

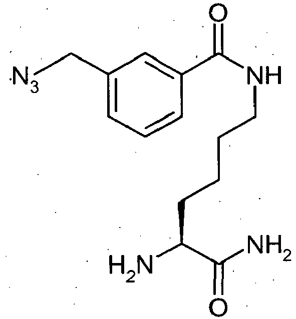

### Step 1:

### Pyrrolidin-2,5-dione-1-yl 3-(azidomethyl)benozoic ester

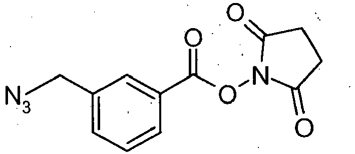

2-Succinimido-1,1,3,3-tetramethyluronium tetrafluoroborate (TSTU, 32.52 g, 107 mmol) was added to a solution of 3-(azidomethyl)benzoic acid (19.01 g, 107 mmol) and triethylamine (14.96 ml, 107 mmol) in *N,N*-dimethylformamide (50 ml). The reaction mixture was stirred for 16 h at room temperature. It was diluted with ethyl acetate (250 ml) and washed with water (3 x 120 ml). The organic layer was washed with a saturated aqueous solution of sodium hydrogencarbonate (150 ml) and dried over sodium sulphate. The solvent was removed in vacuo to give 25.22 g of pyrrolidin-2,5-dione-1-yl 3-(azidomethyl)benozoic ester.
1 H-NMR (CDCl₃) δ 2.92 (m, 4 H); 4,45 (s, 2 H); 7.55 (t, 1 H), 7.65 (d, 2 H); 8.10 (m, 2 H).

### Steep 2:

### (S)-6-(3-(Aminomethyl)benzoylamino)2-(tert-butoxycarbonylamino)hexanoic amide

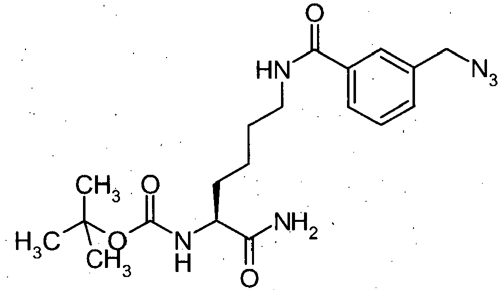

Crude (S)-5-amino-1-(carbamoyl)pentyl)carbamic acid tert-butyl ester (10.26 g, 41.82 mmol) was dissolved in *N,N*-dimethylformamide (150 ml). Pyrrolidin-2,5-dione-1-yl 3-(azidomethyl)benozoic ester (11.47 g, 41.822 mmol) and ethyldiisopropylamine (21.48 ml, 125.5 mmol) were added successively. The reaction mixture was stirred for 16 h at room temperature. It was diluted with ethyl acetate (500 ml) and washed first with a 10% aqueous solution of sodium hydrogensulphate (200 ml), water (3 x 250 ml) and a saturated aqueous solution of sodium hydrogencarbonate (200 ml). It was dried over sodium sulphate. The solvent was removed in vacuo to give 6.05 g of (S)-6-(3-(aminomethyl)benzoylamino)2-(tert-butoxycarbonylamino)hexanoic amide.
1H-NMR (CDCl₃) δ 1.40 (s, 9 H); 1.63 (m, 4 H); 1.83 (m, 2 H); 3.43 (q, 2 H); 4.15 (m, 1 H); 4.37 (s, 2 H); 5.56 (d, 1 H); 6.08 (s, 1 H); 6.75 (s, 1 H); 7.00 (s, 1 H); 7.43 (m, 2 H); 7.77 (m, 2 H).
MS: m/z = 427 (M+Na)⁺, 305 (M-Boc)⁺.

### Step 3:

Gaseous hydrogen chloride was bubbled two times for 15 min each through a suspension of (S)-6-(3-(aminomethyl)benzoylamino)2-(tert-butoxycarbonylamino)hexanoic amide (6.05 g, 14.96 mmol) in ethyl acetate (75 ml). The solvent was removed in vacuo. The crude product was purified by 9 runs of a HPLC-chromatography on a C18-reversed phase column, using a gradient of 8-28% acetonitrile in water, which was buffered with 0.1% trifluoroacetic acid, to give together 5.03 g of the trifluoroacetic acid salt of (*S*)-2-amino-6-(3-(azidomethyl)benzoylamino)hexanoic amide
HPLC: 6.53 min (method 02-b1-2).
1 H-NMR (DMSO-d₆) δ 1.36 (m, 2 H); 1.55 (m, 2 H); 1.75 (m, 2 H); 3.26 (q, 2 H); 3.70 (m, 1 H); 4.53 (s, 2 H); 7.52 (m, 3 H); 7.84 (m, 3 H); 8.06 (br, 3 H); 8.54 (t, 1 H).
MS: m/z = 305 (M+1)⁺

### Example 4 (Reference example)

### (S)-2-(((((((Glutamyl)aspartyl)asparagyl)glutamyl)phenylalanyl)phenylalanyl)leucylamino)-6-(3-((4-((3-((2-(2-(2-(2-(2-(2-(2-(2-(2-(2-(((3-(((S)-5-(carbamoyl)-5-(((((((glutamyl)aspartyl)-asparagyl)glutamyl)phenylalanyl)phenylalanyl)leucylamino)pentyl)carbamoyl)benzyloxy)-imino)acetylamino)ethoxy)ethoxy)ethoxy)ethoxy)ethoxy)ethoxy)ethoxy)ethoxy)ethoxy)ethyl)-carbamoyl)phenoxy)methyl)-1,2,3-triazol-1-yl)methyl)benzoylamino)hexanoic amide

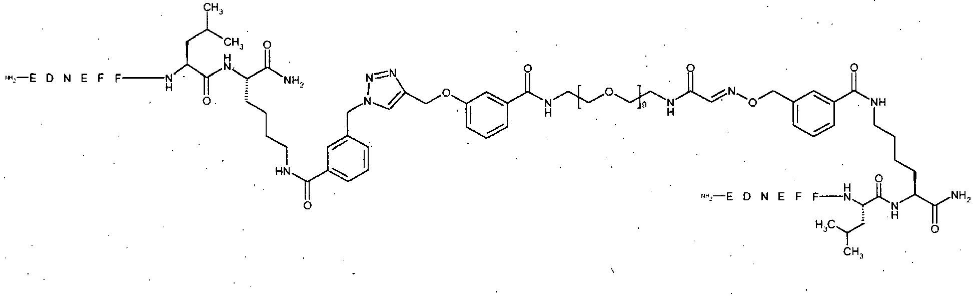

### Step 1

### (S)-6-(3-(Azidomethyl)benzoylamino)-2-(((((((glutamyl)aspartyl)asparagyl)glutamyl)-phenylalanyl)phenylalanyl)leucylamino)hexanoic amide

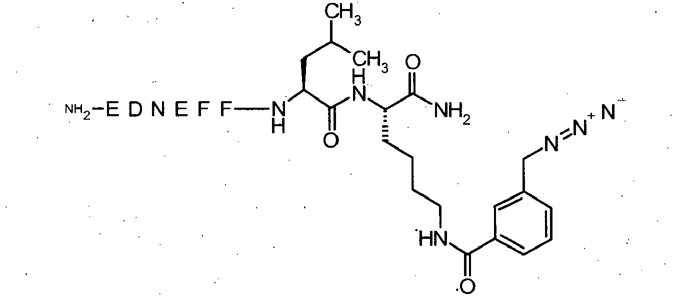

A solution of CPY (100 U) in water (0.50 ml) was added to a solution of (((((((glutamyl)aspartyl)asparaginyl)glutamyl)phenylalanyl)phenylalanyl)leucyl)alanine (25 mg, 0.025 mmol) and (S)-2-amino-6-(3-(azidomethyl)benzoylamino)hexanoic amide (523 mg, 1.25 mmol) in a solution (7.20 ml) of 250 mM HEPES/5 mM EDTA, which was adjusted with 1 N hydrochloric acid and an aqueous 4 M solution of sodium hydroxide to pH 7.94. The reaction mixture was kept at 30°C for 1 day. It was diluted with water (10 ml). (S)-6-(3-(Azidomethyl)benzoylamino)-2-(((((((glutamyl)aspartyl)asparagyl)glutamyl)phenylalanyl)-phenylalanyl)leucylamino)hexanoic amide (5.57 mg) was isolated by reversed phase HPLC-chromatography on a C₁₈-column, using a gradient of 5-50% acetonitrile in water, which was buffered with 0.1% trifluoroacetic acid, as eluent.
MS: m/z = 1200
HPLC: Rₜ = 25.40 min (method 03-B6-1).

### Step 2:

### (S)-3-tert-Butoxy-2-tert-butoxycarbonylaminopropionic acid 2,5-dioxo-pyrrolidin-1-yl ester

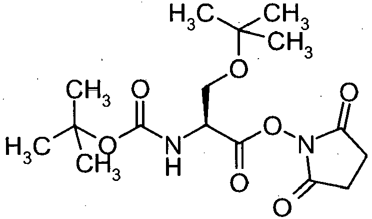

2-Succinimido-1,1,3,3-tetramethyluronium tetrafluoroborate (TSTU, 5.80 g, 19.13 mmol) was added to a solution of (S)-3-tert-butoxy-2-tert-butoxycarbonylaminopropionic acid (5.00 g, 19.13 mmol) and triethylamine (2.67 ml, 19.13 mmol) in *N,N*-dimethylformamide. The reaction mixture was stirred for 16 h at room temperature. It was diluted with ethyl acetate (100 ml) and washed with water (3 x 50 ml) and a saturated aqueous solution of sodium hydrogencarbonate (50 ml). It was dried over sodium sulphate. The solvent was removed in vacuo to give 5.93 g of (S)-3-tert-butoxy-2-tert-butoxycarbonylaminopropionic acid 2,5-dioxo-pyrrolidin-1-yl ester.
¹H-NMR (CDCl₃) δ 1.20 (s, 9 H); 1.46 (s, 9 H); 2.83 (s, 4 H); 3.66 (broad d, 1 H), 3.93 (broad d, 1 H); 4.79 (broad d, 1 H); 5.42 (broad d, 1 H).
MS: m/z = 381 (M+Na)⁺, 204 (M-Boc)⁺

### Step 3:

### N-(2-(2-(2-(2-(2-(2-(2-(2-(2-(2-((S)-3-tert-Butoxy-2-(tert-butoxycarbonylamino)-propionylamino)ethoxy)ethoxy)ethoxy)ethoxy)ethoxy)ethoxy)ethoxy)ethoxy)ethoxy)ethyl)-3-(prop-2-ynyloxy)benzoic amide

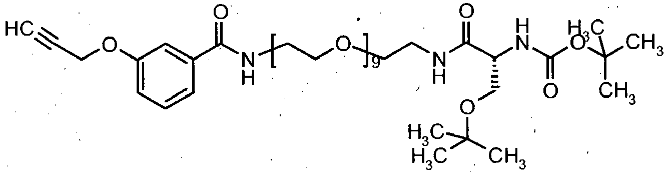

(S)-3-tert-Butoxy-2-tert-butoxycarbonylaminopropionic acid 2,5-dioxo-pyrrolidin-1-yl ester (138 mg, 0.384 mmol) and ethyldiisopropylamine (0.99 ml, 5.76 mmol) were added successively go a solution of the trifluoroacetate salt of *N*-(2-(2-(2-(2-(2-(2-(2-(2-(2-(2-aminoethoxy)ethoxy)ethoxy)ethoxy)ethoxy)ethoxy)ethoxy)ethoxy)ethoxy)ethyl)-3-(prop-2-ynyloxy)benzoic amide (280 mg, 0.384 mmol). The reaction mixture was stirred for 3 days at room temperature. It was diluted with dichloromethane (20 ml) and washed successively with a 10% aqueous solution of sodium hydrogen sulphate and a saturated aqueous solution of sodium hydrogencarbonate. The solvent was removed in vacuo to giv 300 mg of *N*-(2-(2-(2-(2-(2-(2-(2-(2-(2-(2-((S)-3-tert-butoxy-2-(tert-butoxycarbonylamino)propionylamino)-ethoxy)ethoxy)ethoxy)ethoxy)ethoxy)ethoxy)ethoxy)ethoxy)ethoxy)ethyl)-3-(prop-2-ynyloxy)-benzoic amide.
MS: m/z = 858.

Step 4:

### N-(2-(2-(2-(2-(2-(2-(2-(2-(2-(2-((S)-2-Amino-3-hydroxypropionylamino)ethoxy)ethoxy)-ethoxy)ethoxy)ethoxy)ethoxy)ethoxy)ethoxy)ethoxy)ethyl)-3-(prop-2-ynyloxy)benzoic amide

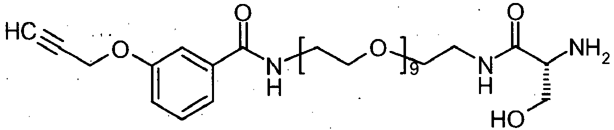

Trifluoroacetic acid (10 ml) was added to a solution of of *N*-(2-(2-(2-(2-(2-(2-(2-(2-(2-(2-((S)-3-tert-butoxy-2-(tert-butoxycarbonylamino)propionylamino)ethoxy)ethoxy)ethoxy)-ethoxy)ethoxy)ethoxy)ethoxy)ethoxy)ethoxy)ethyl)-3-(prop-2-ynyloxy)benzoic amide (300 mg, 0.35 mmol) in dichloromethane (10 ml). The reaction mixture was stirred for 30 min. The solvent was removed in vacuo. The residue was dried in vacuo to give 390 mg of the trifluoroacetate salt of *N*-(2-(2-(2-(2-(2-(2-(2-(2-(2-(2-((S)-2-amino-3-hydroxypropionylamino)-ethoxy)ethoxy)ethoxy)ethoxy)ethoxy)ethoxy)ethoxy)ethoxy)ethoxy)ethyl)-3-(prop-2-ynyloxy)-benzoic amide.

### Step 5:

### (S)-6-(3-((4-((3-((2-(2-(2-(2-(2-(2-(2-(2-(2-(2-((S)-2-Amino-3-hydroxypropionylamino)ethoxy)-ethoxy)ethoxy)ethoxy)ethoxy)ethoxy)ethoxy)ethoxy)ethoxy)ethyl)carbamoyl)phenoxy)-methyl)1,2,3-triazol-1-yl)methyl)benzoylamino)hexanoic amide

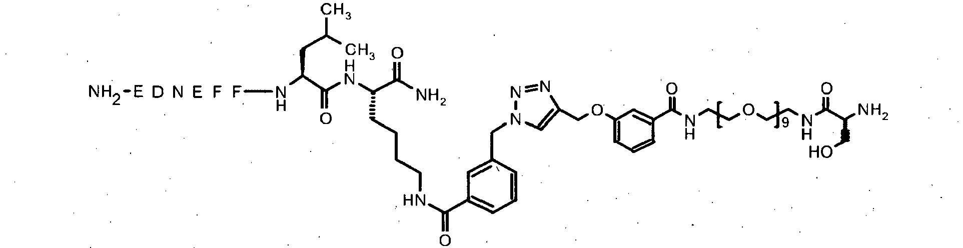

A solution of copper(II) sulphate pentahydrate (24.7 mg) in water (5.49 ml) was added to a solution of ascorbic acid (87 mg) in a mixture of water (5.38 ml) and 2,6-lutidine (0.11 ml). The solution was left at room temperature for 5 min. 5.2 ml of this solution was given to a solution of the trifluoroacetate salt of *N*-(2-(2-(2-(2-(2-(2-(2-(2-(2-(2-((S)-2-amino-3-hydroxypropionylamino)ethoxy)ethoxy)ethoxy)ethoxy)ethoxy)ethoxy)ethoxy)ethoxy)-ethoxy)ethyl)-3-(prop-2-ynyloxy)benzoic amide (76 mg, 0.093 mmol) and (S)-6-(3-(azidomethyl)benzoylamino)-2-(((((((glutamyl)aspartyl)asparagyl)glutamyl)-phenylalanyl)phenylalanyl)leucylamino)hexanoic amide (5.57 mg, 0.005 mmol) in a mixture of water (3.46 ml) and 2,6-lutidine (0.040 ml). The reaction mixture was left at room temperature for 16 h. It was diluted with water (10 ml). (S)-6-(3-((4-((3-((2-(2-(2-(2-(2-(2-(2-(2-(2-(2-((S)-2-Amino-3-hydroxypropionylamino)ethoxy)ethoxy)ethoxy)ethoxy)ethoxy)-ethoxy)ethoxy)ethoxy)ethoxy)ethyl)carbamoyl)phenoxy)methyl)1,2,3-triazol-1-yl)methyl)-benzoylamino)hexanoic amide (2.89 mg) was isolated by reversed phase HPLC-chromatography on a C₁₈-column, using a gradient of 20-40% acetonitrile in water, which was buffered with 0.1 % trifluoroacetic acid, as eluent.
MS: m/z = 951 (M²⁺).
HPLC: Rₜ = 23.49 min (method 03-B6-1).

### Step 6:

(S)-6-(3-((4-((3-((2-(2-(2-(2-(2-(2-(2-(2-(2-(2-((S)-2-Amino-3-hydroxypropionylamino)ethoxy)ethoxy)ethoxy)ethoxy)ethoxy)ethoxy)ethoxy)ethoxy)-ethoxy)ethyl)carbamoyl)phenoxy)methyl)1,2,3-triazol-1-yl)methyl)benzoylamino)hexanoic amide (2.89 mg, 0.002 mmol) was dissolved in a mixture of water (0.200 ml) and triethanolamine (0.0004 ml). A solution of 2-hydroxyethyl methyl sulfide (0.0024 ml) in water (0.011 ml) was added. A solution of sodium periodate (2.4 mg) in water (0.020 ml) was added. The reaction mixture was left at room temperature for 30 min. This solution was added to a solution of (*S*)-6-(3-(aminoxymethyl)benzoylamino)-2-(((((((glutamyl)aspartyl)-asparaginyl)glutamyl)phenylalanyl)phenylalanyl)leucylamino)hexanoic amide (2.86 mg, 0.002 mmol) in a mixture of water (0.600 ml), triethanolamine (0.0012 ml), and ethyldiisopropylamine (0.002 ml). Acetic acid (0.004 ml) was added. A precipitation occurred. 4 M aqueous sodium hydroxide (0.020 ml) was added to obtain pH 7.07. This solution was left for 16 h at room temperature. It was diluted with water (5 ml). (S)-2-(((((((Glutamyl)aspartyl)asparagyl)glutamyl)phenylalanyl)phenylalanyl)leucylamino)-6-(3-((4-((3-((2-(2-(2-(2-(2-(2-(2-(2-(2-(2-(((3-(((S)-5-(carbamoyl)-5-(((((((glutamyl)aspartyl)-asparagyl)glutamyl)phenylalanyl)phenylalanyl)leucylamino)pentyl)-carbamoyl)benzyloxy)imino)acetylamino)ethoxy)ethoxy)ethoxy)ethoxy)ethoxy)ethoxy)-ethoxy)ethoxy)ethoxy)ethyl)carbamoyl)phenoxy)methyl)-1,2,3-triazol-1-yl)methyl)-benzoylamino)hexanoic amide (0.83 mg) was isolated by reversed phase HPLC on a C18-column, using a gradient of 20-45% acetonitrile in water, which was buffered with 0.1% trifluoroacetic acid, as eluent.
MS: m/z = 1015 (M³⁺).
HPLC: Rₜ = 26.73 min (method 03-B6-1).

### Example 5 (Reference example)

### (S)-2-(((((((Glutamyl)aspartyl)asparagyl)glutamyl)phenylalanyl)leucyl)amino)6-(3-((4-((4-(((S)-5-carbamoyl-5-(((((((glutamyl)aspartyl)asparagyl)glutamyl)phenylalanyl)leucyl)amino)-penty))carbamoyl)phenoxy)methyl)1,2,3-triazol-1-yl)methyl)benzoylamino)hexanoic amide

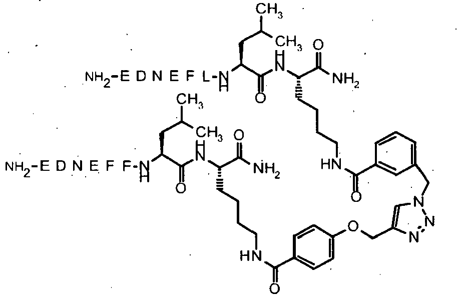

### Step 1:

### 4-(Prop-2-ynyloxy)benzoic acid.methyl ester

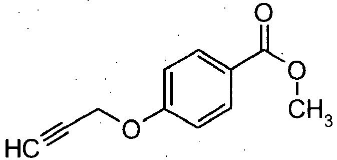

Propargyl bromide (15.0 ml, 164 mmol) was added to a mixture of commercially available methyl 4-hydroxybenzoate (20 g, 131 mmol) and potassium carbonate (36.3 g, 263 mmol) in acetone (500 ml). The mixture was stirred at room temperature for 2 days. The solids were removed by filtration. The solvent was removed from the filtrate to give 25.1 g of crude 4-(prop-2-ynyloxy)benzoic acid methyl ester, which was used for the next step without further purification.
¹H-NMR (CDCl₃): δ 2.55 (t, 1 H); 3.89 (s, 3 H); 4.75 (d, 2 H); 7.00 (d, 2 H); 8.01 (d, 2 H). MS: m/z = 191 (M+1)⁺.

### Step 2:

### 4-(Prop-2-ynyloxy)benzoic acid

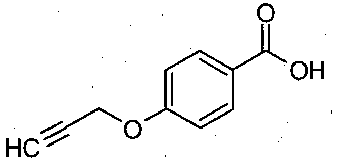

4-(Prop-2-ynyloxy)benzoic acid methyl ester (10 g, 52.6 mmol) was dissolved in ethanol (150 ml). An 1 N solution of sodium hydroxide in water (60 ml, 60 mmol) was added. The reaction mixture was stirred for 16 h at room temperature. The solvent was removed in vacuo. The residue was dissolved in a mixture of ethyl acetate (100 ml) and a 5% aqueous solution of citric acid (100 ml). The phases were separated. The organic layer was dried over magnesium sulphate. The solvent was removed in vacuo to give 9.4 g of 4-(prop-2-ynyloxy)-benzoic acid, which was used in the next step without further purification.
¹H-NMR (DMSO-d₅): δ 2.51 (t, 1 H); 4.89 (d, 2 H); 7.08 (d, 2 H); 7.81 (d, 2 H).
MS: m/z = 177 (M+1)⁺

### Step 3:

### N-((S)-5-Amino-5-carbamoylpentyl)-4-(prop-2-ynyloxy)benzamide

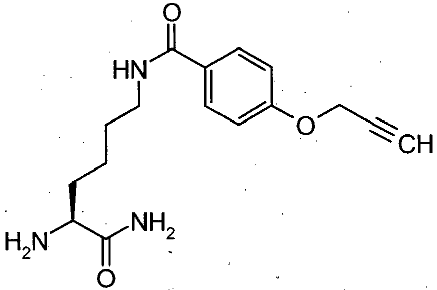

Fmoc-protected Rink-resin (commercially available at Novabiochem, loading 0.70 mmol/g, 3.81 g, 2.66 mmol) was washed with dichloromethane (100 ml). A 20% solution of piperidine in *N,N*-dimethylformamide (100 ml) was added. The reaction vessel was shaken for 20 min at room temperature. The liquid was removed. The resin was washed with *N-*methylpyrrolidinone (3 x 100 ml) and dichloromethane (5 x 100 ml). A solution of Boc-Lys(Fmoc)-OH (5.0 g, 10.67 mmol) in *N*-methylpyrrolidinone (40 ml) was added. A solution of 1-hydroxybenzotriazole (1.63 g, 10.67 mmol) in *N*-methylpyrrolidinone (10 ml) was added. A solution of diisopropylcarbodiimide (1.67 ml, 10.67 mmol) in dichloromethane (25 ml) and a solution of ethyldiisopropylamine (1.83 ml. 10.67 mmol) were added successively. The reaction vessel was shaken for 16 h at room temperature. The liquid was removed. The resin was washed with *N*-methylpyrrolidinone (3 x 100 ml) and dichloromethane (5 x 100 ml). A 20% solution of piperidine in *N,N*-dimethylformamide (100 ml) was added. The reaction vessel was shaken for 20 min at room temperature. The liquid was removed. The resin was washed with *N*-methylpyrrolidinone (3 x 100 ml) and dichloromethane (5 x 100 ml). A mixture of 4-(prop-2-ynyloxy)benzoic acid (1.88 g, 10.67 mmol) and 1-hydroxybenzo-triazole (1.63 g, 10.67 mmol) in *N*-methylpyrrolidinone (50 ml) and a solution of diisopropylcarbodiimide (1.67 ml, 10.67 mmol) and ethyldiisopropylamine (1.83 ml, 10.67 mmol) in dichloromethane (50 ml) were added successively. The reaction vessel was shaken for 16 h at room temperature. The liquid was removed. The resin was washed with N-methylpyrrolidinone (3 x 100 ml) and dichloromethane (5 x 100 ml). A 50% solution of trifluoroacetic acid to which had been added triisopropylsilane (10 ml) was added to the resin. Another 15 ml of triisopropylsilane were added. The reaction vessel was shaken at room temperature for 1 h. The liquid was collected. The solvents were removed in vacuo. The residue was dissolved in toluene (100 ml). The solvent was again removed in vacuo. The crude product was purified by HPLC-chromatography on a C₁₈-reversed phase column, using a gradient of 13-33% acetonitrile in water, which was buffered with 0.1 % trifluoroacetic acid, as eluent, to give 310 mg of the trifluoroacetic acid salt of *N*-((*S*)-5-amino-5-carbamoylpentyl)-4-(prop-2-ynyloxy)benzamide.
¹H-NMR (DMSO-d₆): δ 1.34 (m, 2 H); 1.53 (m, 2 H); 1.75 (m, 2 H); 3.24 (q, 2 H); 3.61 (s, 1 H); 3.70 (br, 1 H); 4.87 (s, 2 H); 7.04 (d, 2 H); 7.57 (br, 1 H), 7.81 (d, 2 H); 7.84 (br, 1 H); 8.07 (br, 3 H); 8.34 (t, 1 H).
MS: m/z = 304 (M+1)⁺.
HPLC: Rₜ = 6.48 min (method 02-b1-2).

Step 4:

### (S)-2-(((((((Glutamyl)aspartyl)asparagyl)glutamyl)phenylalanyl)leucyl)amino)-6-(4-(prop-2-ynyloxy)benzoylamino)hexanoic amide

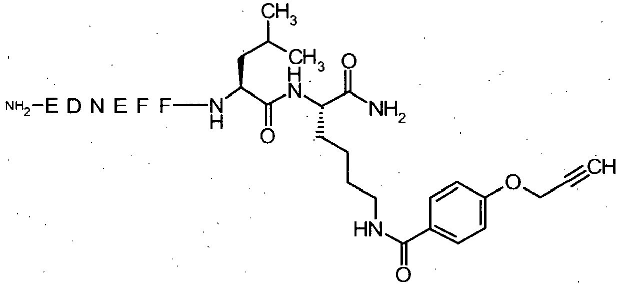

(((((((Glutamyl)aspartyl)asparaginyl)glutamyl)phenylalanyl)phenylalanyl)leucyl)-alanine (14.4 mg, 0.015 mmol) was dissolved in water (0.500 ml) and a 4 N aqueous solution of sodium hydroxide (0.002 ml). This solution was added to a solution of *N*-((S)-5-Amino-5-carbamoylpentyl)-4-(prop-2-ynyloxy)benzamide (300 mg, 0.72 mmol) in a buffer of 0.25 M HEPES and 5 mM EDTA (1.00 ml), which had been adjusted to pH 8 with 1 N hydrochloric acid. The solution was adjusted to pH 7.75 by addition of a 4 N aqueous solution of sodium hydroxide and 1 N hydrochloric acid. This mixture was warmed to 30°C. A buffer (0.677 ml), consisting of 0.25 M HEPES and 5 mM EDTA (1.00ml), which had been adjusted to pH 8 with 1 N hydrochloric acid, was added, giving rise to a total volume of 2.67 ml. A solution of CPY (50 U) in water (0.250 ml) was added. The reaction vessel was shaken gently at 30°C for 2.5 h. The reaction mixture was diluted with water (5 ml) and filtered. It was subjected to a HPLC-chromatography on a C₁₈-reversed phase column, using a gradient of 10-50% acetonitrile in water, which was buffered with 0.1 % trifluoracetic acid to give 0.5 mg of (*S*)-2-(((((((Glutamyl)aspartyl)asparagyl)glutamyl)phenylalanyl)leucyl)amino)-6-(4-(prop-2-ynyloxy)benzoylamino)hexanoic amide.
MS: m/z = 1199 (M+1)⁺
HPLC: Rₜ = 24.88 min (method 03-b6-1)

### Step 5:

A solution of copper sulphate pentahydrate (10.5 mg) in water (2.34 ml) was added to a solution of ascorbic acid (37 mg) in water (2.29 ml) and 2,6-lutidine (0.047 ml). This mixture was stirrred at room temperature for 5 min. 2.21 ml of this solution was added to a solution of (*S*)-6-(3-(azidomethyl)benzoylamino)-2-(((((((glutamyl)aspartyl)asparaginyl)-glutamyl)phenylalanyl)leucyl)leucylamino)hexanoic amide (2,3 mg, 2000 nmol) and (S)-2-(((((((glutamyl)aspartyl)asparagyl)glutamyl)phenylalanyl)leucyl)amino)-6-(4-(prop-2-ynyloxy)-benzoylamino)hexanoic amide (0.50 mg, 417 nmol) in a 2% solution of 2,6-lutidine in water (1.96 ml). The reaction mixture was gently shaken at room temperature for 2 h. It was subjected to a HPLC-chromatography on a C₁₈-revresed phase column using a gradient of 10-60% acetonitrile in water, which was buffered with 0.1 % trifluoroacetic acid to give 0.1 mg of (*S*)-4-(((((((glutamyl)aspartyl)asparagyl)glutamyl)phenylalanyl)leucyl)amino)-6-(3-((4-((4-(((S)-5-carbamoyl-5-(((((((glutamyl)aspartyl)asparagyl)glutamyl)phenylalanyl)leucyl)amino)-pentyl)carbamoyl)phenoxy)methyl)1,2,3-triazol-1-yl)methyl)benzoylamino)hexanoic amide.
MS: m/z = 1183 (M²⁺+1), 789 (M³⁺+1).
HPLC: Rₜ = 24.78 min (method 03-b6-1).

### Example 6 (Reference example)

### (S)-2-((((((((Arginyl)lysyl)aspargyl)lysyl)phenylalanyl)phenylalanyl)leucyl)amino)-6-(3-(((1-((((S)-5-carbamoyl-5-((((((((arginyl)lysyl)aspargyl)lysyl)phenylalanyl)phenylalanyl)leucyl)-amino)pentyl)carbamoyl)phenyl)ethylidene)aminoxy)methyl)benzoylamino)hexanoic amide

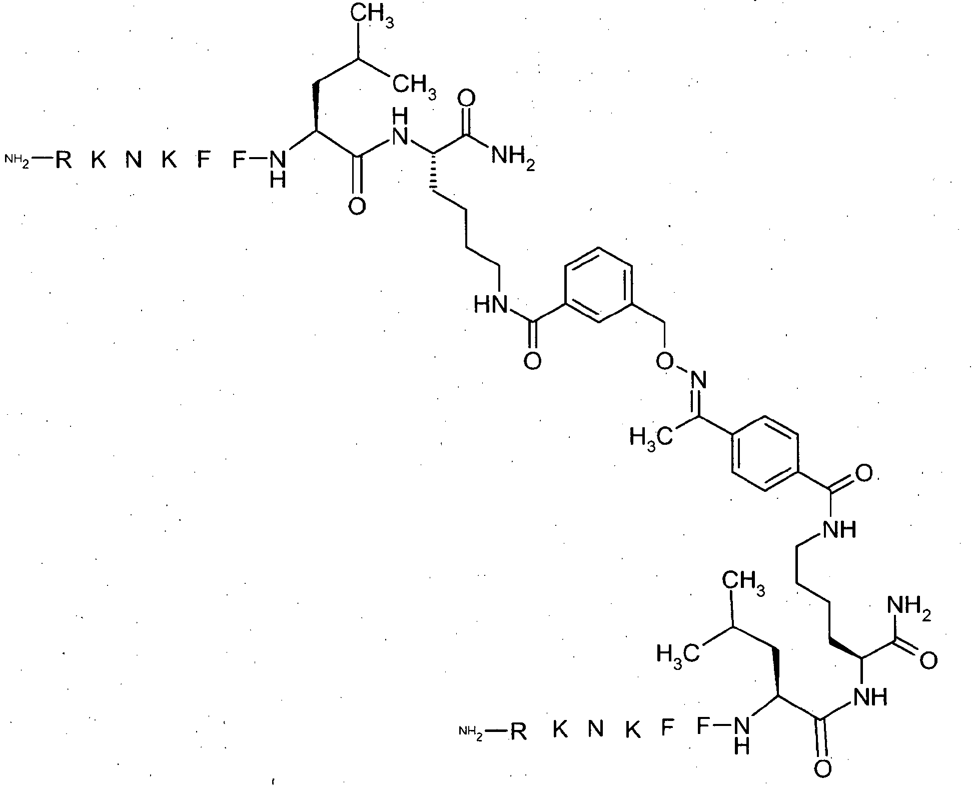

### Step 1:

### (((((((Arginyl)lysyl)aspargyl)lysyl)phenylalanyl)phenylalanyl)leucyl)alanine

(((((((Arginyl)lysyl)aspargyl)lysyl)phenylalanyl)phenylalanyl)leucyl)alanine was prepared on an Applied Biosystems 433A Peptide Synthesizer by standard Fmoc-strategy, known to a person skilled in the art, starting with a commercially available Fmoc-Ala-Wang resin. Following amino acid derivatives were used:

| coupling no. | amino acid derivative |
|---|---|
| 1 | Fmoc-Leu-OH |
| 2 | Fmoc-Phe-OH |
| 3 | Fmoc-Phe-OH |
| 4 | Fmoc-Lys(Boc)-OH |
| 5 | Fmoc-Asn(Trt)-OH |
| 6 | Fnioc-Lys(Boc)-OH |
| 7 | Fmoc-Arg(Pmc)-OH |

A mixture of trifluoroacetic acid (10.6 ml), water (0.265 ml) and triisopropylsilane (0.265 ml) was added to the resin. It was shaken for 1.5 h. The liquid was collected. The resin was washed with trifluoroacetic acid (1 ml). The liquids were combined. The solution was concentrated under a stream of nitrogen. Ether (40 ml) was added. The precipitation was isolated by centrifugation. It was dried at the air. The crude product was purified on a reversed phase C₁₈-column on a HPLC, using a gradient of 15-45% acetonitrile in water in a buffer of 0.1 % trifluoroacetic acid, as eluent.
MS: m/z = 1023 (M⁺)
HPLC: Rₜ = 16.18 min (method 02-b6-1).

Step 2:

### (S)-2-((((((((Arginyl)lysyl)aspargyl)lysyl)phenylalaninyl)phenylalaninly)leucyl)amino)-6-(3-(aminoxymethyl)benzoylamino)hexanoic amide

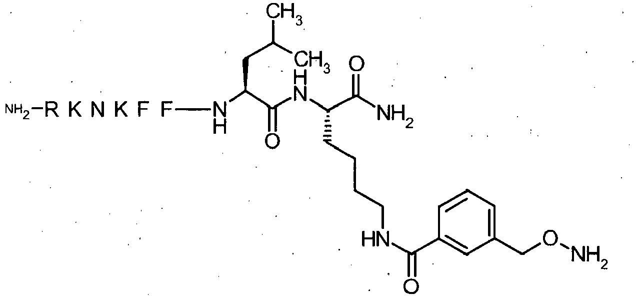

(((((((Arginyl)lysyl)aspargyl)lysyl)phenylalanyl)phenylalanyl)leucyl)alanine (27.3 mg, 0.027 mmol) was dissolved in water (500 ml). This solution was added to a solution of *N-*((S)-5-amino-5-(carbamoyl)pentyl)-3-(aminooxymethyl)benzamide (544 mg, 1.33 mmol) in a buffer (2.10 ml), consisting of 0.25 M HEPES and 5 mM EDTA, which had been adjusted to pH 8.0 with 1 N hydrochloric acid. The pH was adjusted to pH 8.07 by addition of a 4 N aqueous solution of sodium hydroxide (0.175 ml). Buffer (2.18 ml), consisting of 0.25 M HEPES and 5 mM EDTA, which had been adjusted to pH 8.0 with 1 N hydrochloric acid, was added. A solution of CPY (75 U) in water (0.375 ml) was added. The reaction mixture was gently shaken at 30°C for 3.5 h. It was diluted with water (10 ml) and filtrated. The filtrate was purified by HPLC-chromatography on a C₁₈-reversed phase column, using a gradient of 5-55% acetonitrile in water, which was buffered with 0.1% trifluoroacetic acid, to give after lyophilization 1.6 mg of (*S*)-2-((((((((arginyl)lysyl)aspargyl)lysyl)phenylalaninyl)-phenylalaninly)leucyl)amino)-6-(3-(aminoxymethyl)benzoylamino)hexanoic amide.
MS: m/z = 1229 (M⁺)
HPLC:Rₜ = 18.41 min (method 03-b6-1).

### Step 3:

### 4-Acetylbenzoic acid 2,5-dioxopyrrolidin-1-yl ester

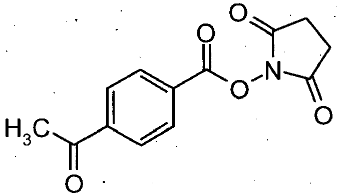

2-Succinimido-1,1,3,3-tetramethyluronium tetrafluoroborate (TSTU, 18.5 g, 60.9 mmol) was added to a solution of 4-acetylbenzoic acid (10.0 g, 60.9 mmol) and triethylamine (8.49 ml, 60.9 mmol) in *N,N*-dimethylformamide (50 ml). The reaction mixture was stirred for 2 h at room temperature. It was diluted with ethyl acetate (400 ml) and washed with water (3 x 200 ml). The organic layer was dried over sodium sulphate. The solvent was removed in vacuo to give 13.38 g of 4-acetylbenzoic acid 2,5-dioxopyrrolidin-1-yl ester.
¹H-NMR (CDCl₃): δ 2.67 (s, 3 H); 2.93 (s, 4 H); 8.05 (d, 2 H); 8.20 (d, 2 H).
MS: m/z = 284 (M+Na)⁺

### Step 4:

### 4-Acetyl-N-((5S)-5-amino-5-carbamoylpentyl)benzamide

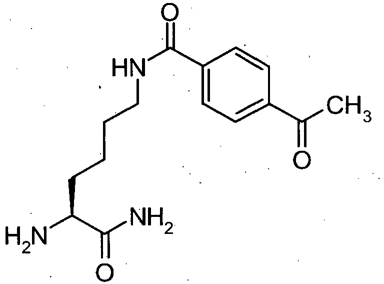

4-Acetylbenzoic acid 2,5-dioxopyrrolidin-1-yl ester (8.21 g, 31.4 mmol) was added to a suspension of crude ((S)-5-amino-1-(carbamoyl)pentyl)carbamic acid tert-butyl ester (7.71 g, 31.4 mmol) in *N,N*-dimethylformamide (200 ml). Ethyldiisopropylamine (16.14 ml, 94.3 mmol) was added. The reaction mixture was stirred for 3 days at room temperature. The solvent was removed in vacuo, at 70°C. The residue, was dissolved in dichloromethane (50 ml). Trifluoroacetic acid (50 ml) was added. The reaction mixture was stirred for 1 h at room temperature. The solvent was removed in vacuo. The residue was taken up dichloromethane (200 ml). A 10% aqueous solution of sodium hydrogen sulphate (50 ml) was added. The mixture was extracted with water (200 ml). The aqueous phase was concentrated in vacuo to approximately 60 ml. It was divided into three parts. Each part was purified by HPLC-chormatography on a C18 reversed phase column, using a gradient of 0-20% of acetonitrile in water, which was buffered with 0.1% trifluoroacetic acid, to give together 8.00 g of the trifluoroacetic salt of 4-acetyl-N-((5*S*)-5-amino-5-carbamoylpentyl)-benzamide.

### Step 5:

### (S)-2-((((((((Arginyl)lysyl)asparagyl)lysyl)phenylalanyl)phenylalanyl)leucyl)amino)-6-(4-acetylbenzoylamino)hexanoic amide

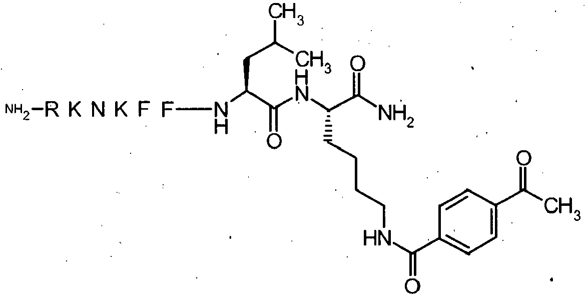

(((((((Arginyl)lysyl)aspargyl)lysyl)phenylalanyl)phenylalanyl)leucyl)alanine (27.3 mg, 0.027 mmol) was dissolved in water (0.500 ml). It was added to a solution of 4-acetyl-N-((5*S*)-5-amino-5-carbamoylpentyl)benzamide (110 mg, 0.27 mmol) in a buffer (1.10 ml), consisting of 0.25 M HEPES and 5 mM EDTA, which had been adjusted to pH 8.0 with 1 N hydrochloric acid. The pH was adjusted to pH 7.94 with a 4 N aqueous solution of sodium hydroxide (0.040 ml). Buffer (0.778 ml), consisting of 0.25 M HEPES and 5 mM EDTA, which had been adjusted to pH 8.0 with 1 N hydrochloric acid, was added. A solution of CPY (50 U) in water (0.250 ml) was added. The reaction vessel was shaken gently at 30°C for 1.5 h. The reaction mixture was diluted with water (5 ml) and filtered. The filtrate was subjected to a HPLC-chromatography on a C₁₈-reversed phase column, using a gradient of 5-65% acetonitrile in water, which was buffered with 0.1 % trifluoroacetic acid to give 3.09 mg of (*S*)-2-((((((((arginyl)lysyl)asparagyl)lysyl)phenylalanyl)phenylalanyl)leucyl)amino)-6-(4-acetylbenzoylamino)hexanoic amide.
MS: m/z = 1226 (M⁺).
HPLC: Rₜ = 21.25 min (method 03-b6-1).

### Step 6:

A solution of (*S*)-2-((((((((arginyl)lysyl)asparagyl)lysyl)phenylalanyl)phenylalanyl)-leucyl)amino)-6-(4-acetylbenzoylamino)hexanoic amide (3.09 mg, 0.003 mmol) in water (0.40 ml) was added to a solution of (*S*)-2-((((((((arginyl)lysyl)aspargyl)lysyl)phenylalaninyl)-phenylalaninly)leucyl)amino)-6-(3-(aminoxymethyl)benzoylamino)hexanoic amide (1.61 mg, 0.001 mmol) in water (0.40 ml). The pH was found to be PH 2.66. The reaction vessel was shaken gently at room temperature. After 2 h, the reaction mixture was diluted with water (5 ml). It was filtrated. The filtrate was subjected to a HPLC-purification on a C₁₈-reversed phase column, using a gradient of 10-60% acetonitrile in water, which was buffered with 0.1% trifluoroacetic acid to give 0.1 mg of (*S*)-2-((((((((arginyl)lysyl)aspargyl)lysyl)-phenylalanyl)phenylalanyl)leucyl)amino)-6-(3-(((1-((((S)-5-carbamoyl-5-((((((((arginyl)lysyl)aspargyl)lysyl)phenylalanyl)phenylalanyl)leucyl)amino)pentyl)-carbamoyl)phenyl)ethylidene)aminoxy)methyl)benzoylamino)hexanoic amide
MS: m/z = 813 (M³⁺), 1219 (M²⁺).
HPLC: Rₜ = 23.77 min (method 03-b6-1).

### Example 7 (Reference example)

### N,N'-bis(2-(2-(2-(4-((3-(N-((S)-5-(carbamoyl)-5-(hGHylleucinylamino)pentyl)carbamoyl)-benzyloxy)imino)butoxy)ethoxy)ethoxy)ethoxy)ethoxy)omega-carbamoyl3.4 kDa PEG-carboxylic acid amide

### Step1:

### CPY-catalyzed transpeptidation of hGH-Leu-Ala with N-((S)-5-Amino-5-(carbamoyl)pentyl)-3-(aminooxymethyl)benzamide

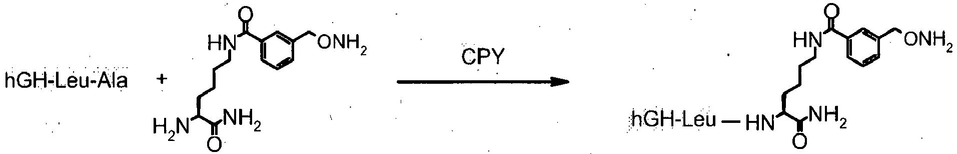

To a solution of hGH-Leu-Ala (30mg, 0.5mM final concentration) in H₂O:diisopropylamine (100:1 v/v, 0.9 ml), was added N-((S)-5-amino-5-(carbamoyl)pentyl)-3-(aminooxymethyl)nzamide (288 mg, 200mM final concentration) in solution in HEPES buffer 250mM pH 8.5 containing 5 mM EDTA (1.46 ml). The pH was adjusted to 8.2 by addition of sodium hydroxide (10 M in water). The reaction volume was adjusted to 2.65 ml by addition of HEPES buffer 250 mM pH8.5 containing 5mM EDTA. The reaction was started by addition of the enzyme (Fluka #21943) in solution in water (11 U/ml final concentration). The reaction mixture was incubated at 30°C.

The reaction was monitored by capillary electrophoresis, CE analysis method 1.

### CE analysis method 1

The capillary electrophoresis was performed using a Hewlett Packard 3D CE system equipped with a diode array detector.

The fused silica. capillary (Agilent) used had a total length of 64.5, an effective length of 56cm and an ID of 50µm. Samples were injected by pressure at 50mbar for 4s. Separations were carried out at 30°C, under a tension of +25kV, using phosphate buffer 50 mM pH 7 as electrolyte. The analysis was monitored at 200 nm. Between runs, an acidic and a basic wash were performed: the capillary was rinsed with water for 2 min, then with phosphoric acid 0.1 M for 2 min, then with water for 2 min; this was followed by rinsing with sodium hydroxyde 0.1 M for 3 min, and water for 2 min, before equilibrating the capillary with the electrolyte.

When the yield of transpeptidated product had reached 54% (after 1.5 h reaction time), the reaction was stopped by inactivating the enzyme with PMSF (100 mM in solution in dry isopropanol, final concentration 2 mM).

After addition to the reaction mixture of a buffer containing triethanolamine (45 mM), 3-methylthio-1 propanol (0.14 M) and PMSF (0.2 mM), the excess of reagent was eliminated by ultra filtration on Millipore Amicon Ultra cut off 10 kD (twice). The remaining protein solution was ultrafiltered again after addition of a solution containing 3-methylthio-1 propanol (0.14 M) (buffer B (Tris 50 mM pH 8.5, 0.2 M NaCl)) and PMSF (0.2 mM) (twice).

Finally, the protein solution obtained was run on desalting column (Amersham HiPrep 26/10 Desalting, eluent: Tris 50mM pH 8.5, 10 ml/min). A buffer shift to 3-methylthio-1 propanol (0.14 M) was then performed. The final protein concentration was 10 mg/ml (2.3 ml).

The solution obtained was used in the next step without further purification.

### Step 2:

### Oximation with ButyrALD-PEG-ButyrALD-3400:

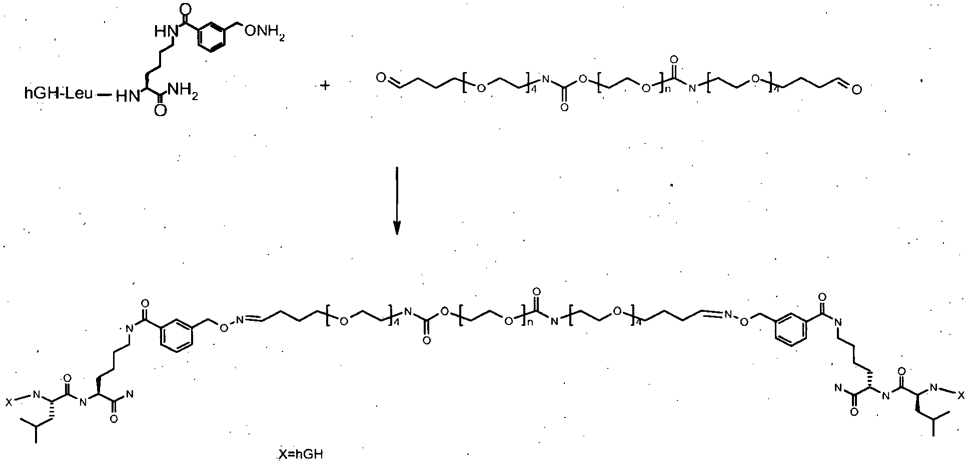

To the protein solution above (10 mg/ml protein, 2.1 ml) was added a solution of butyraldehyde-poly(ethyleneglycol)-butyraldehyde (average MW 3400) (catalog number 0808F02 from Nektar) (0.95 mg (0.45 equivalents) in 3-methylthio-1 propanol 0.14 M (0.4 ml)). The reaction mixture was incubated at 30°C. The disappearance of the hydroxylamine derivative obtained in step 1 was followed by capillary electrophoresis, method 2.

### CE analysis method 2:

The electrolyte was a phosphate buffer 50 mM pH 2.5. No acid wash of the capillary was performed in between runs. All other conditions were as for CE analysis 1 above.

An analysis on an Agilent 2100 bioanalyzer was run after 48 h and showed the formation of a product with the expected mass for the dimer with a yield of about 20%.

The product was purified on ion exchange (Amersham MonoQ 10/100 GL, A buffer: Tris 50 mM pH 8.5, buffer B: A+0.2 M NaCl, 0 to 30% B over 3 column volume, 30 to 80% B over 20 column volume, and 80 to 100% over 3 column volume. 4 ml/min).

The pooled fractions containing the product were diluted in water and subjected again to the same purification procedure. The dimer and the monomer were however not separated properly.

A further purification step was thus performed on a gel filtration column (Hiprep Superdex200, Tris 50mM pH8.5, 2.5 ml/min). The dimer could be isolated but contained some degradation products, which were partially removed by a last ion exchange purification step, similar to the first purification step. Yield: 0.11 mg of the product was obtained (about 3% overall yield).

### Example 8 (Reference example)

### (S)-6-(5-(Acetyl)2-(3-(4-((4-((S)-2-carbamoyl-2-((((((((glutamyl)aspartyl)asparaginyl)-glutamyl)phenylalanyl)phenylalanyl)leucyl)amino)ethyl)phenoxy)methyl)triazol-1-yl)propoxy)-benzoylamino)2-((hGHyl)leucylamino)hexanoic amide

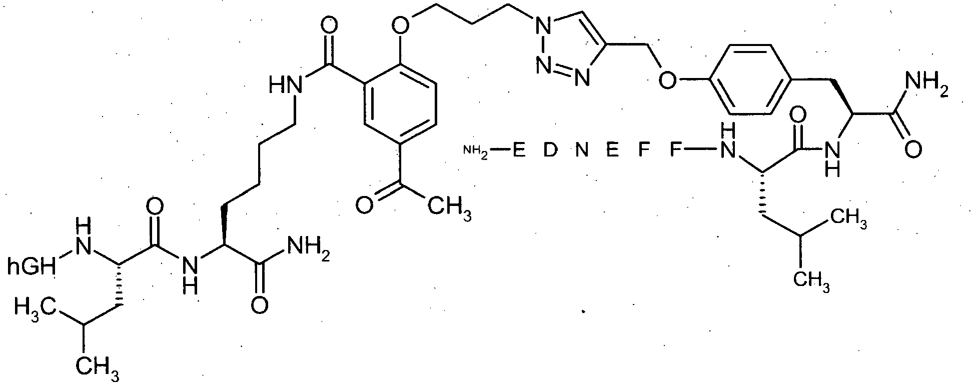

### Step 1:

### 5-Acetyl-2-(3-bromo-propoxy)benzoic acid methyl ester

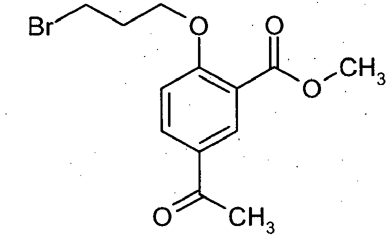

A mixture of methyl 5-acetylsalicylate (20 g, 103 mmol), potassium carbonate (42.7 g, 309 mmol), tetrabutylammonium iodide (1.90 g, 5.15 mmol), and N,N-dimethylformamide (150 ml) was added to a solution of 1,3-dibromopropane (42 ml, 412 mmol). The reaction mixture was heated to 60°C for 16 h. It was cooled to room temperature. Water was added until all potassium carbonate was dissolved. It was extracted with ethyl acetate (300 ml). The organic layer was washed with water (3 x 200 ml) and a saturated aqueous solution of sodium hydrogencarbonate (200 ml). It was dried over sodium sulphate. The solvent was removed in vacuo. The crude product was purified by flash chromatography on silica (400 g), using a mixture of ethyl acetate/heptane/dichloromethane (1:2:1) as eluent to give 10.77 g of 5-acetyl-2-(3-bromo-propoxy)benzoic acid methyl ester.
MS: m/z = 315 (M+1)
¹H-NMR (CDCl₃): δ 2.38 (quintet, 2 H); 2.59 (s, 3 H); 3.71 (t, 2 H); 3.91 (s, 3 H); 4.27 (t, 2 H); 7.05 (d, 1 H); 8.09 (d, 1 H); 8.42 (s, 1 H).

### Step 2:

### 5-Acetyl-2-(3-azidopropoxy)benzoic acid methyl ester

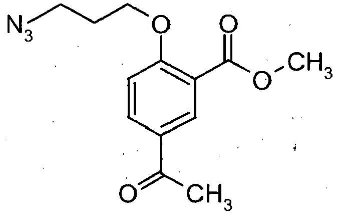

5-Acetyl-2-(3-bromo-propoxy)benzoic acid methyl ester (10.77 g, 34.2 mmol) was dissolved in *N,N*-dimethylformamide, (200 ml). Sodium azide (4.44 g, 68.3 mmol) and tetrabutylammonium iodide (0.126 g, 0.342 mmol) were added successively. The reaction mixture was stirred at 60°C for 16 h. It was cooled to room temperature and diluted with water (200 ml). It was extracted with ethyl acetate (300 ml). The organic layer was washed with water (3 x 200 ml) and dried over sodium sulphate. The solvent was removed in vacuo to yield the crude product. The aqueous phase was treated according to the literature (Lunn, G. Sansone E. B. Destruction of hazardous chemicals in the laboratory, 2nd edition, John Wiley & Sons, New York) in order to remove the excess azide. The crude product was purified by flash chromatography on silica (90 g), using a mixture of ethyl acetate/heptane/- dichloromethane (3:2:1) to dissolve the crude product and a mixture of ethyl acetate/heptane (3:2) as eluent to give 5.15 g of 5-acetyl-2-(3-azidopropoxy)benzoic acid methyl ester.
MS: m/z = 278 (M+1), 300 (M+Na), 249 (M-28).
¹H-NMR (CDCl₃): δ 2.12 (quintett, 2 H); 2.58 (s, 3 H); 3.62 (t, 2 H); 3.91 (s, 3 H); 4.21 (t, 2 H); 7.03 (d, 1H); 8.10 (d, 1H); 8.42 (s, 1H).

### Step 3:

### 5-Acetyl-2-(3-azidopropoxy)benzoic acid

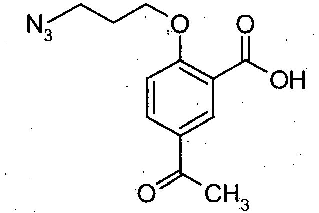

5-Acetyl-2-(3-azidopropoxy)benzoic acid methyl ester (5.15 g, 18.6 mmol) was dissolved in 1,4-dioxane (200 ml). A solution of lithium hydroxide (0.534 g, 22.3 mmol) in water (200 ml) was added. The reaction mixture was stirred for 4 days at room temperature. An 1 N aqueous solution of sodium hydroxide was added to obtain a solution with pH 13-14. It was washed with tert-butyl methyl ether (3 x 200 ml). The aqueous phase was acidified to pH 3 with a 10% aqueous solution of sodium hydrogensulphate. It was extracted with ethyl acetate (3 x 200 ml). The combined ethyl acetate layers were dried over sodium sulphate. The solvent was removed in vacuo to give crude 5-acetyl-2-(3-azidopropoxy)benzoic acid, which was used without further purification.
MS: m/z = 236 (M-28), 286 (M+23).
¹H-NMR (CDCl₃): δ 2.19 (quintet, 2 H); 2.63 (s, 3 H); 3.64 (t, 2 H); 4.37 (t, 2 H); 7.12 (d, 1 H); 8.22 (d, 1 H); 8.70 (s, 1 H); 10-12 (broad 1 H).
HPLC: Rₜ = 6.70 min (method 02-b4-4)

### Step 4:

### 5-Acetyl-2-(3-azidopropoxy)benzoic acid 2,5-dioxo-pyrrolidin-1-yl ester

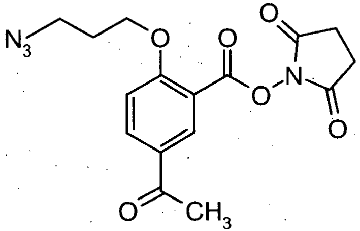

2-Succinimido-1,1,3,3-tetramethyluronium tetrafluoroborate (TSTU, 4.51 g, 15.0 mmol) was added to a solution of 5-acetyl-2-(3-azidopropoxy)benzoic acid (3.94, 15.0 mmol) and triethylamine (2.09 ml, 15.0 mmol) in *N,N*-dimethylformamide (100 ml). The reaction mixture was stirred for 24 h at room temperature. A 10% aqueous solution of sodium hydrogensulphate (400 ml) was added. The mixture was extracted with ethyl acetate (2 x 200 ml). The combined organic layers were washed with brine (200 ml) and dried over sodium sulphate. The solvent was removed in vacuo. It was treated with a mixture of ethyl acetate/heptane (2:1, 10 ml). The formed precipitation was isolated by filtration, washed with a mixture of ethyl acetate/heptane (2:1, 10 ml) and dried in vacuo to give 2.9 g of 5-acetyl-2-(3-azidopropoxy)benzoic acid 2,5-dioxo-pyrrolidin-1-yl ester.
MS: m/z = 383 (M+Na), 333 (M+1-N₂).
¹H-NMR (CDCl₃): δ 2.12 (quintet, 2 H); 2.61 (s, 3 H); 2.92 (broad, 4 H); 3.61 (t, 2 H); 4.24 (t, 2 H); 7.09 (d, 1 H); 8.23 (d, 1H); 8.59 (s, 1H).

### Step 5:

### {(S)-5-[5-Acetyl-2-(3-azidopropoxy)benzoylamino]-1-[carbamoyl]pentyl}carbamic acid tert-butyl ester

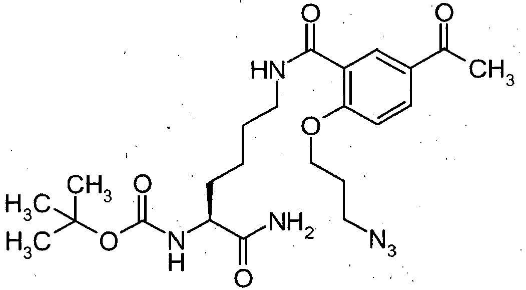

Crude (S)-5-amino-1-(carbamoyl)pentyl)carbamic acid tert-butyl ester (1.97 g, 8.05 mmol) was added to a solution of 5-acetyl-2-(3-azidopropoxy)benzoic acid 2,5-dioxo-pyrrolidin-1-yl ester (2.90 g, 8.05 mmol) and ethyldiisopropylamine (4.13 ml, 24.14 mmol) in *N,N*-dimethylformamide (200 ml). The reaction mixture was stirred for 16 h at room temperature. It was diluted with a 10% aqueous solution of sodium hydrogensulphate (400 ml) and extracted with ethyl acetate (2 x 300 ml). The combined organic layers were washed with a saturated aqueous solution of sodium hydrogencarbonate (300 ml) and dried over sodium sulphate. The solvent was removed in vacuo to give 3.01 g of crude {(S)-5-[5-acetyl-2-(3-azidopropoxy)benzoylamino]-1-[carbamoyl]pentyl}carbamic acid tert-butyl ester, which was used in the next step without further purification.
MS: m/z = 391 (M+1-Boc).
¹H-NMR (DMSO-d₆): δ 1.29-1.65 (m, 6 H); 1.36 (s, 9 H); 2.05 (quintet, 2 H); 2.55 (s, 3 H); 3.26 (q, 2 H); 3.55 (t, 2 H); 3.84 (q, 1 H); 4.23 (t, 2 H); 6.70 (d, 1 H); 6.93 (s, 1 H); 7.24 (m, 2 H); 8.02 (d, 1 H); 8.10 (t, 1 H); 8.18 (s, 1 H).
HPLC: Rt = 7.83 min (method 02-b4-4).

### Step 6:

### 5-Acetyl-N-((S)-5-amino-5-carbamoylpentyl)-2-(3-azidopropoxy)benzamide

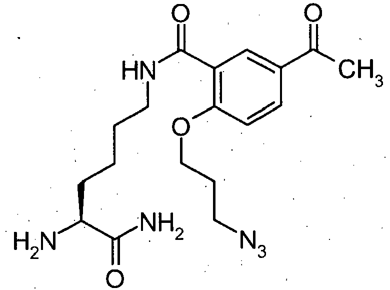

Trifluoroacetic acid (100 ml) was added to a mixture of {(S)-5-[5-acetyl-2-(3-azido-propoxy)benzoylamino]-1-[carbamoyl]pentyl}carbamic acid tert-butyl ester (3.01 g, 6 mmol) and dichloromethane (100 ml). The reaction mixture was stirred for 1.5 h at room temperature. The solvent was removed in vacuo. The residue was dissolved in dichloromethane (100 ml). The solvent was removed in vacuo. This latter procedure was repeated two times. The crude product was purified on a HPLC reversed phase C₁₈-column, using a gradient of 0-40% acetonitrile in water, which was buffered with 0.1 % trifluoroacetic acid, as eluent to give 1.40 g of the trifluoroacetate salt of 5-acetyl-N-((S)-5-amino-5-carbamoylpentyl)-2-(3-azidopropoxy)benzamide.
MS: m/z = 391 (M+1).
¹H-NMR (DMSO-d₆): δ 1.36 (m, 2 H); 1.52 (m, 2 H); 1.73 (m, 2 H); 2.05 (quintet, 2 H); 2.55 (s, 3 H); 3.27 (q, 2 H); 3.55 (t, 2 H); 3.69 (m, 1 H); 4.24 (t, 2 H); 7.25 (d, 1 H); 7.57 (s, 1 H); 7.83 (s, 1 H); 8.03 (m, 4 H); 8.18 (m, 2 H).
HPLC: Rt = 5.02 (method 02-b4-4).

### Step 7:

### hGH-Leu-Ala

A cloning strategy based on pNNC13, a pET11a derived vector already containing Zbasic2mt-D4K-hGH has been utilized. Using pNNC13 as template and a PCR primer set flanking the SacII and BamHI restriction sites, a 628 bp amplicon has been generated encoding two additional amino acids (leucine and alanine) in the C-terminal end of hGH. This PCR amplicon was then cloned back into pNNC13 using the existing SacII and BamHI sited to generate pNNC13.4 encoding Zbasic2mt-D4K-hGH-Leu-Ala. The integrity of the resulting clones was confirmed by DNA sequencing of the coding region.

*Escherichia coli* BL21 (DE3) was transformed with pET11a-Zbasic2mt-D4K-hGH-Leu-Ala. Single colony was inoculated into 100 ml LB media with 100 µg/ml Amp and grown at 37°C until OD600 reaches 0.6. The cell culture temperature was reduced to 20°C and the cells were induced with 1 mM IPTG for 6 hours at 20°C. The cells were harvested by centrifugation at 3000g for 15 minutes.

The cell pellet was re-suspended in cell lysis buffer (25 mM Na₂HPO₄, 25 mM NaH₂PO₄ pH 7, 5 mM EDTA, 0.1% Triton X-100), and the cells were disrupted by cell disruption at 30 kpsi (Constant Cell Disruption Systems). The lysate was clarified by centrifugation at 10,000g for 35 minutes and the supernatant was used for purification.

Zbasic2mt-D4K-hGH-Leu-Ala was purified on SP Sepharose FF using a step gradient elution (buffer A: 25 mM Na₂HPO₄ 25 mM NaH₂PO₄ pH 7; buffer B: 25 mM Na₂HPO₄ 25 mM NaH₂PO₄ pH 7, 1 M NaCl). The protein was subsequently cleaved using Enteropeptidase for the release of hGH-Leu-Ala. After digestion, hGH-Leu-Ala was further purified on a Butyl Sepharose 4FF column to separate the product from the Zbasic2mt-D4K domain and Enteropeptidase (buffer A: 100 mM HEPES pH 7.5, 2M NaCl; buffer B: 100 mM HEPES pH 7.5, a linear gradient was used).

As digestion was not complete, remaining Zbasic2mt-D4K-hGH-Leu-Ala had to be separated from hGH-Leu-Ala and this was done by loading the protein onto the SP Sepharose FF column again. Buffer exchange into a solution consisting of 25 mM Na₂HPO₄ and 25 mM NaH₂PO₄ (pH 7) is performed on a Sephadex G-25 Medium column before purification on the SP Sepharose FF column. Zbasic2mt-D4K-hGH-Leu-Ala binds to SP Sepharose FF whereas protein hGH-Leu-Ala was found in the flow through.

The buffer of the solution of the protein hGH-Leu-Ala was exchanged to 50 mM NH₄HCO₃. The solution was lyophilized.

### Step 8:

### (S)-6-(2-(3-Azidopropxoy)-5-acetylbenzoylamino)-2-(hGHylleucylamino)hexanoic amide

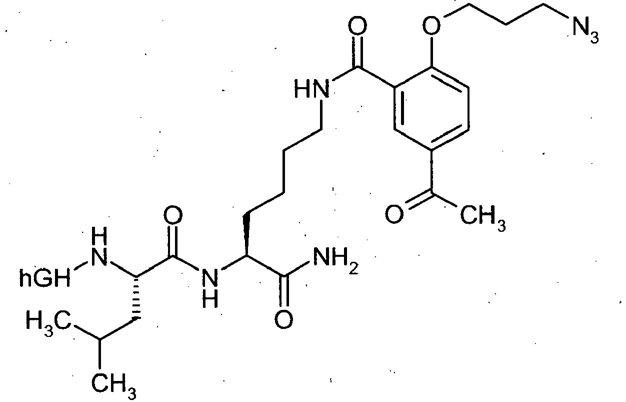

hGH-Leu-Ala (15 mg, 672 nmol) was dissolved in water (0.51 ml) and ethyldiisopropylamine (0.004 ml). A second solution was prepared by dissolving 5-acetyl-N-((S)-5-amino-5-carbamoylpentyl)-2-(3-azidopropoxy)berizamide (99 mg, 0.20 mmol) in a buffer consisting of 0.25 M HEPES and 5 mM EDTA which had been adjusted to pH 8.0. The two solutions were combined and were subsequently adjusted to pH 8.01 by addition of an aqueous 1 N solution of sodium hydroxide. A solution of CPY in water (200 U/ml, 0.075 ml) was added. The reaction mixture was shaken gently at room temperature for 16 h. A freshly prepared 100 mM solution of phenylmethanesulfonyl fluoride in isopropanol (0.0135 ml) was added. The reaction mixture was stirred gentyl for 0.5 h. It was filtered. The buffer was changed to a 50 mM ammonium hydrogencarbonate buffer by gel-chromatography on a HiPrep26/10 desalting column. A freshly prepared 100 mM solution of phenylmethane-sulfonyl fluoride in isopropanol (0.0045 ml) was added immediately to the fractions containing protein. These fractions were combined and subjected to a buffer-change chromatography using a HiPrep 26/10 desalting column and a buffer of 25 mM TRIS, which was adjusted to pH 8.5. The fractions containing protein were combined and subjected to a ion-exchange-chromatography on a MonoQ 10/100 GL column, using a gradient of 0-100% of a buffer consisting of 25 mM TRIS and 0.2 M sodium chloride at pH 8.5 in a buffer consisting of 25 mM TRIS at pH 8.5 over 120 column volumes at a flow of 4 ml/min. The fractions containing (*S*)-6-(2-(3-azidopropxoy)-5-acetylbenzoylamino)-2-(hGHylleucylamino)-hexanoic amide were identified by MALDI-MS. They were combined. The buffer was changed to a 50 mM ammonium hydrogencarbonate buffer by a gel-chromatography, using a HiPrep 26/10 desalting column to yield 3.91 mg of (*S*)-6-(2-(3-azidopropxoy)-5-acetyl-benzoylamino)-2-(hGHylleucylamino)hexanoic amide. The yield was determined by quantification at 280 nm, using an absorption coefficient of 0.731. An IEF-gel showed an expected change of the pKa of the protein compared to starting protein hGH-Leu-Ala. MALDI-TOF (CHCA (alpha-cyano-4-hydroxycinnamic acid), uncorrected): found: m/z = 22586 (M⁺), 11295 (M²⁺); required: 22727 (M⁺), 11363.5 (M²⁺).

### Step 9:

### [1-Carbamoyl-2-(4-hydroxyphenyl)ethyl]-carbamic acid tert-butyl ester

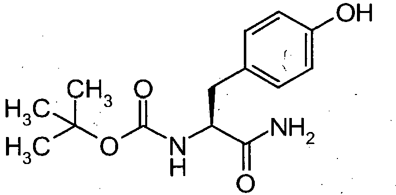

1-Hydroxybenzotriazole (5.44 g, 35.55 mmol) and 1-(3-dimethylaminopropyl)-3-ethylcarbodiimide hydrochloride (6.82 g, 35.55 mmol) were added successively to a solution of Boc-Tyr-OH (10 g, 35.55 mmol) in N,N-dimethylformamide (70 ml) and dichloromethane (70 ml). The reaction mixture was stirred for 20 min at room temperature. A 25% aqueous solution of ammonia (26.56 ml, 355 mmol) was added. The reaction mixture was stirred for 16 h at room temperature. It was diluted with ethyl acetate (300 ml) and washed with water (3 x 100 ml) and subsequently with a saturated aqueous solution of sodium hydrogen-carbonate (100 ml). The solvent was removed in vacuo to give 4.24 g of crude [1-Carbamoyl-2-(4-hydroxyphenyl)ethyl]carbamic acid tert-butyl ester.
¹H-NMR (DMSO-d₆): δ 1.31 (s 9 H); 2.80 (dd, 1 H); 2.83 (dd, 1 H); 4.00 (m, 1 H); 6.62 (d, 2 H); 6.70 (d, 1 H); 6.97 (br, 1 H); 7.03 (d, 2 H); 7.31 (br, 1 H); 9.14 (s, 1 H).

### Step 10:

### [(S)-1-Carbamoyl-2-(4-(prop-2-ynyloxy)phenyl)ethyl]carbamic acid tert-butyl ester

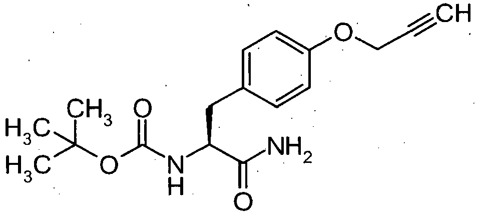

A mixture of of [(*S*)-1-carbamoyl-2-(4-hydroxyphenyl)ethyl]-carbamic acid tert-butyl ester (1.0 g, 3.57 mmol), tetrabutylammonium iodide (65 mg; 0.17 mmol), potassium carbonate (3.94 g, 29 mmol), propargyl bromide (0.38 ml, 4.28 mmol) and *N,N*-dimethylformamide (15 ml) was heated to 60°C for 16 h. It was cooled to room temperature, diluted with water (30 ml) and acidified with a 10% aqueous solution of sodium hydrogensulphate. The mixture was extracted with ethyl acetate (2 x 100 ml). The combined organic layers were washed with a saturated aqueous solution of sodium hydrogencarbonate (200 ml) and dried over magnesium sulphate. The solvent was removed in vacuo. The crude product was purified by flash chromatography on silica (100 g), using a mixture of dichloromethane/- methanol (10:1) as eluent, to give 998 mg of [(*S*)-1-crbamoyl-2-(4-(prop-2-ynyloxy)phenyl)-ethyl]carbamic acid tert-butyl ester.
MS: m/z = 341 (M + Na)⁺.
¹H-NMR (DMSO-d₆) δ 1.31 (s, 9 H); 2.50 (s, 1 H); 2.67 (dd, 1 H); 2.91 (dd, 1 H); 4.03 (m, 1 H); 4.74 (s, 2 H); 6.77 (d, 1 H); 6.86 (d, 2 H); 6.99 (s, 1 H), 7.17 (d, 2 H); 7.35 (s, 1 H).

### Step 11:

### (2S)-2-Amino-3-(4-(prop-2-ynyloxy)phenyl)propionamide

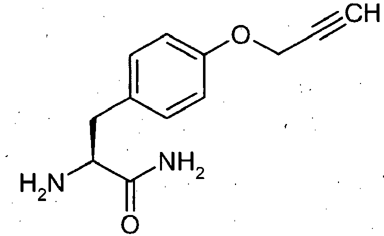

Trifluoroacetic acid (10 ml) was added to a solution of [(*S*)-1-carbamoyl-2-(4-(prop-2-ynyloxy)phenyl)ethyl]carbamic acid tert-butyl ester (998 mg, 3.13 mmol) in dichloromethane (10 ml). The reaction mixture was stirred for 1.5 h at room temperature. The solvent was removed. The residue was dissolved in dichloromethane (30 ml). The solvent was removed. The latter procedure was repeated twice to give 1.53 g of the trifluoroacetate salt of (2*S*)-2-amino-3-(4-(prop-2-ynyloxy)phenyl)propionamide.
MS: m/z = 219 (M + 1)⁺.
¹H-NMR (CDCl₃) δ 2.51 (s, 1 H); 3.02 (m, 2 H); 3.90 (m, 1 H); 4.78 (s, 2 H); 6.95 (d, 2 H); 7.20 (d, 2 H); 7.56 (s, 1 H); 7.87 (s, 1 H); 8.10 (br, 3 H).
HPLC (method 02-B4-4): R_{f} = 5.62 min.

### Step 12:

### (S)-2-(((((((Glutamyl)aspartyl)asparginyl)glutamyl)phenylalanyl)phenylalanyl)leucylamino)-3-(4-(propargyloxy)phenyl)propionic amide

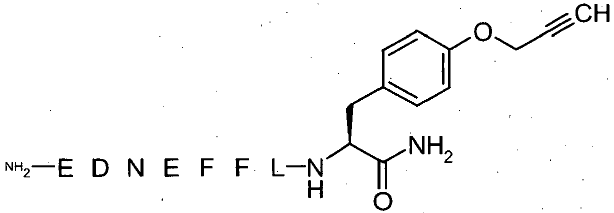

A solution of (((((((glutamyl)aspartyl)aspargyl)glutamyl)phenylalanyl)phenylalanyl)-leucyl)alanine (11 mg, 0.011 mmol) in a buffer consisting of 250 mM HEPES and 5 mM EDTA, which had been adjusted to pH 7.5 (4 ml), was added to a solution of the trifluoro-acetate salt of (2S)-2-amino-3-(4-(prop-2-ynyloxy)phenyl)propionamide (928 mg, 2.80 mmol) in a buffer consisting of 250 mM HEPES and 5 mM EDTA, which had been adjusted to pH 7.5 (3 ml). Beta-hydroxypropylcyclodextrin (220 mg) was added. The pH was adjusted to pH 8.09 by addition of an aqueous 1 N solution of sodium hydroxide (3.00 ml). A buffer consisting of 250 mM HEPES and 5 mM EDTA, which had been adjusted to pH 7.5 (0.96 ml) was added to obtain a total volume of 10.96 ml. The pH was found to be pH 8.06. A solution of CPY (200 U/ml, 0.22 ml) was added. The reaction mixture was gently shaken for 15 min. It was diluted with water (20 ml) and subjected to a HPLC-chromatography on a reversed phase C₁₈-column, using a gradient of 10-50% acetonitrile in water, which both were buffered with 0.1% trifluoroacetic acid. The fractions containing the desired product were combined and lyophilized to give 3.8 mg of (*S*)-2-(((((((glutamyl)aspartyl)asparginyl)-glutamyl)phenylalanyl)phenylalanyl)leucylamino)-3-(4-(propargyloxy)phenyl)propionic amide.
MS: m/z = 1114.
HPLC: Rₜ = 7.28 min (02-b4-4).

### Step 13:

A solution of (*S*)-2-(((((((glutamyl)aspartyl)asparginyl)glutamyl)phenylalanyl)-phenylalanyl)leucylamino)-3-(4-(propargyloxy)phenyl)propionic amide (0.192 mg, 173 nmol) in a 2% solution of 2,6-lutidine (0.10 ml) was added to a solution of (*S*)-6-(2-(3-azido-propxoy)-5-acetylbenzoylamino)-2-(hGHylleucylamino)hexanoic amide (1.95 mg, 86 nmol) in a 2% solution of 2,6-lutidine (0.250 ml). A solution of copper(I) was prepared by addition of a solution of copper(II) sulphate pentahydrate (0.216 mg, 863 nmol) in water (0.0215 ml) to a solution of ascorbic acid (0.760 mg, 4316 nmol) in a 2% solution of 2,6-lutidine in water (0.0215 ml). This copper(I) solution was left for 1 min and added to the above described solution of protein and peptide. The reaction mixture was gently shaken for 1 h at room temperature, The MALDI-TOF (uncorrected) showed a peak at m/z = 23724 in expectation for the desired product next to a smaller peak at m/z = 22582, corresponding to the starting protein. A SDS gel of the reaction mixture indicated as a product with a molecular weight of about 1 kDa higher than the starting material, in accordance with the expected result. A similar result was obtained in a Agilent Bioanalyzer experiment, which showed as the only protein present a product with a molecular weight approximately 1.2 kDa over the molecular weight of the starting material, as expected.

## Claims

1. A method for coupling two different polypeptides together at their respective C-terminus comprising
(a) an enzyme catalyzed modification of the C-termini of the polypeptides to be coupled by which a chemical group comprising reactive group W is introduced to the C-terminus of the first polypeptide to be coupled, and a chemical group comprising reactive group Z is introduced to the C-terminus of the second polypeptide to be coupled
(b) reacting
the reactive group Z with a reactive group Y of a spacer-molecule to form a chemical group X and reacting the reactive group W with a reactive group V of the spacer-molecule to form a chemical group U, wherein spacer between the two polypeptides comprises at least one ethylene glycol diradical, wherein X and U are independently selected from the group consisting of diradicals of oxime and triazole wherein V, W, Y and Z are independently selected from the group consisting of alkoxylamino, oxo, azido and alkynyl,

2. A method according to claim 1, wherein the enzyme is carboxypeptidase Y or a variant or a fragment thereof, which variant or fragment retains the ability to catalyse a reaction, by which the C-terminal amino acid of a protein or peptide is replaced by a different chemical moiety.

3. A method according to claim 2, wherein the enzyme is carboxypeptidase Y.

4. A method according to any one of claims 1 to 3, wherein at least one of the polypeptides is human growth hormone.

5. A method according to any one of claims 1 to 4, wherein at least one of the polypeptides is a derivative of human growth hormone.

6. A method according to any one of claims 1 to 5, wherein at least one of the polypeptides is a variant of human growth hormone or a derivative of a variant of human growth hormone.

7. A method according to any one of claims 1 to 6, wherein at least one of the polypeptides is hGH-Leu-Ala.

8. A method according to any one of claims 1 to 7, wherein the chemical group Z is introduced via a linker A.

9. A method according to claim 8, wherein linker A is selected from the group consisting of bi-radicals of straight, branched and/or cyclic C₁₋₁₀alkane, C₂₋₁₀alkene, C₂₋₁₀alkyne, C₁₋₁₀heteroalkane, C₂₋₁₀heteroalkene, C₂₋₁₀heteroalkyne, any of which may be substituted with oxo, wherein one or more homocyclic aromatic compound biradical(s) or heterocyclic aromatic compound biradical(s) may be inserted.

10. A method according to any one of claims 1 to 9, wherein the chemical group W is introduced via a linker B.

11. A method according to claim 10, wherein linker B is selected from the group consisting of bi-radicals of straight, branched and/or cyclic C₁₋₁₀alkane, C₂₋₁₀alkene, C₂₋₁₀alkyne, C₁₋₁₀heteroalkane, C₂₋₁₀heteroalkene, C₂₋₁₀heteroalkyne, which is optionally substituted with oxo, wherein one or more homocyclic aromatic compound biradical(s) or heterocyclic aromatic compound biradical(s) may be inserted.

12. A method according to any one of claims 8 to 11, wherein linker A and linker B are independently selected from the group consisting of 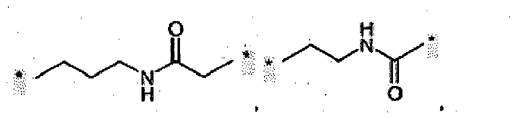 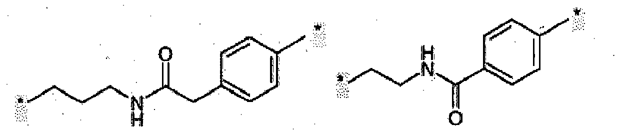 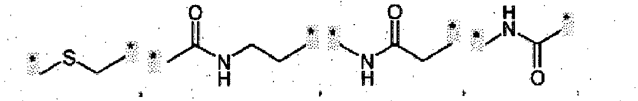 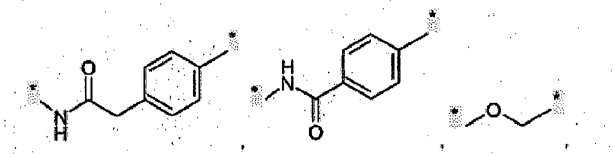 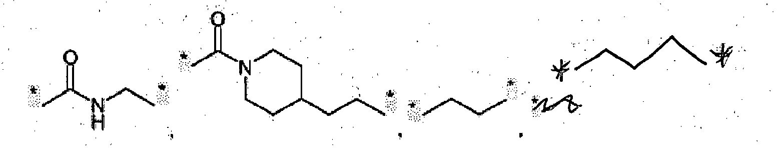 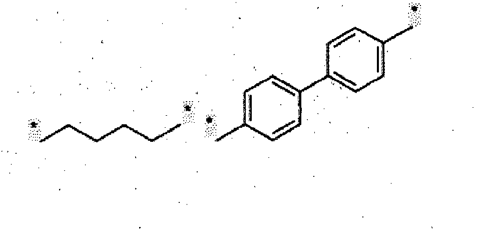 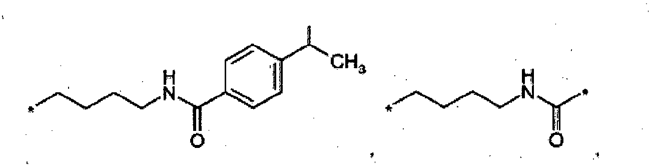 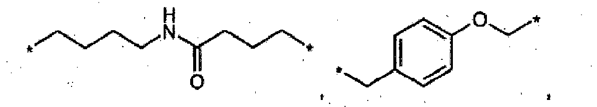 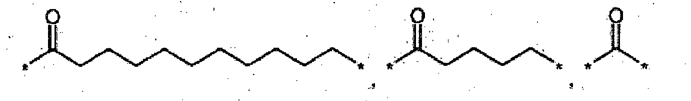 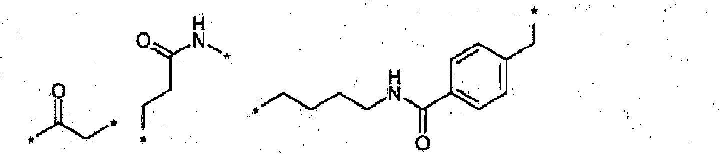 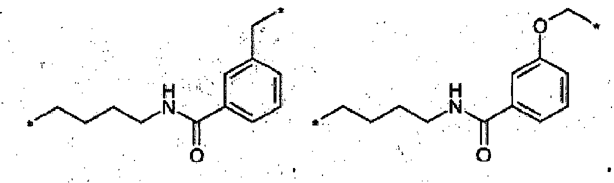 and

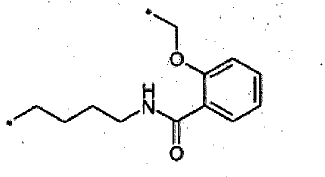

13. A method according to any of claims 1 to 12, wherein the moiety linking the two polypeptides in the end-product comprises a 1,2,3-triazole moiety.

## Patentansprüche

1. Verfahren zur Aneinanderkopplung zweier verschiedener Polypeptide an ihrem jeweiligen C-Terminus, umfassend
(a) eine enzymkatalysierte Modifikation der C-Termini der zu koppelnden Polypeptide, durch die eine die reaktive Gruppe W umfassende chemische Gruppe am C-Terminus des ersten zu koppelnden Polypeptids eingeführt wird und eine die reaktive Gruppe Z umfassende chemische Gruppe am C-Terminus des zweiten zu koppelnden Polypeptids eingeführt wird,
(b) Umsetzen
der reaktiven Gruppe Z mit einer reaktiven Gruppe Y eines Spacer-Moleküls unter Bildung einer chemischen Gruppe X und Umsetzen der reaktiven Gruppe W mit einer reaktiven Gruppe V des Spacer-Moleküls unter Bildung einer chemischen Gruppe U, wobei der Spacer zwischen den beiden Polypeptiden wenigstens ein Ethylenglykol-Diradikal umfasst, wobei X und U unabhängig aus der aus Diradikalen von Oxim und Triazol bestehenden Gruppe ausgewählt sind, wobei V, W, Y und Z unabhängig aus der aus Alkoxylamino, Oxo, Azido und Alkinyl bestehenden Gruppe ausgewählt sind.

2. Verfahren nach Anspruch 1 , wobei es sich bei dem Enzym um Carboxypeptidase Y oder eine Variant oder ein Fragment davon handelt, wobei in der Variante bzw. dem Fragment die Fähigkeit zur Katalyse einer Reaktion, durch die die C-terminale Aminosäure eines Proteins oder Peptids durch eine andere chemische Gruppierung ersetzt wird, erhalten bleibt.

3. Verfahren nach Anspruch 2, wobei es sich bei dem Enzym um Carboxypeptidase Y handelt.

4. Verfahren nach einem der Ansprüche 1 bis 3, wobei es sich bei wenigstens einem der Polypeptide um menschliches Wachstumshormon handelt.

5. Verfahren nach einem der Ansprüche 1 bis 4, wobei es sich bei wenigstens einem der Polypeptide um ein Derivat von menschlichem Wachstumshormon handelt.

6. Verfahren nach einem der Ansprüche 1 bis 5, wobei es sich bei wenigstens einem der Polypeptide um eine Variante von menschlichem Wachstumshormon oder ein Derivat einer Variante von menschlichem Wachstumshormon handelt.

7. Verfahren nach einem der Ansprüche 1 bis 6, wobei es sich bei wenigstens einem der Polypeptide um hGH-Leu-Ala handelt.

8. Verfahren nach einem der Ansprüche 1 bis 7, wobei die chemische Gruppe Z über einen Linker A eingeführt wird.

9. Verfahren nach Anspruch 8, wobei Linker A aus der aus Biradikalen von geradlinigem, verzweigtem und/oder cyclischem C₁₋₁ₒAlkan, C₂₋₁₀Alken, C₂₋₁₀Alkin, C₁₋₁₀Heteroalkan, C₂₋₁₀Heteroalken, C₂₋₁₀Heteroalkin, die jeweils mit Oxo substituiert sein können, bestehenden Gruppe ausgewählt ist, wobei ein oder mehrere Homocyclische-aromatische-Verbindung-Biradikal(e) oder Heterocyclischearomatische-Verbindung-Biradikal(e) eingefügt werden können.

10. Verfahren nach einem der Ansprüche 1 bis 9, wobei die chemische Gruppe W über einen Linker B eingeführt wird.

11. Verfahren nach Anspruch 10, wobei Linker B aus der aus Biradikalen von geradlinigem, verzweigtem und/oder cyclischem C₁₋₁ₒAlkan, C₂₋₁₀Alken, C₂₋₁₀-Alkin, C₁₋₁₀Heteroalkan, C₂₋₁₀Heteroalken, C₂₋₁₀-Heteroalkin, das gegebenenfalls mit Oxo substituiert ist, bestehenden Gruppe ausgewählt ist, wobei ein oder mehrere Homocyclische-aromatische-Verbindung-Biradikal(e) oder Heterocyclischearomatische-Verbindung-Biradikal(e) eingefügt werden können.

12. Verfahren nach einem der Ansprüche 8 bis 11, wobei Linker A und Linker B unabhängig aus der aus 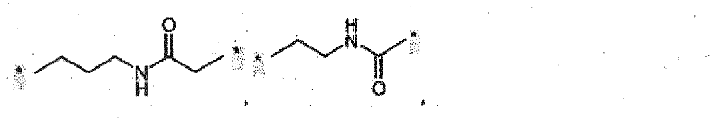 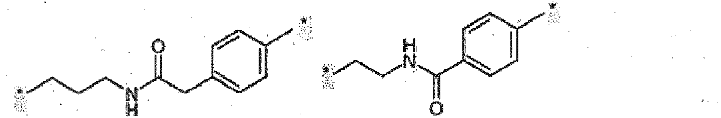 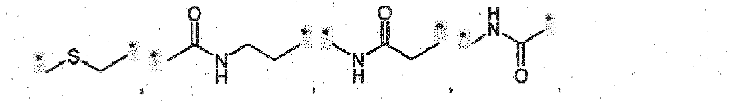 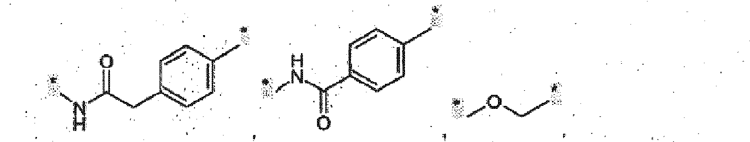 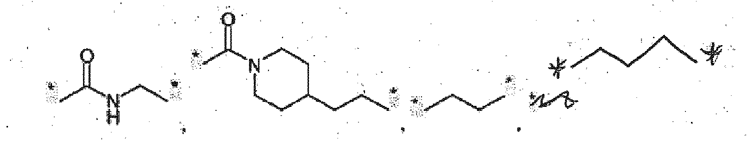 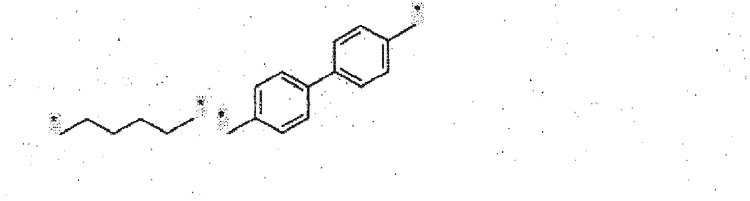 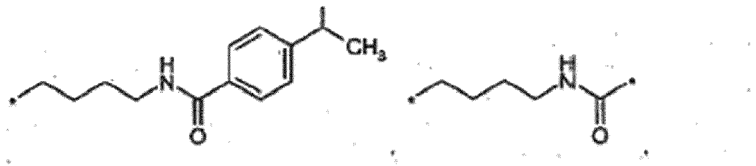 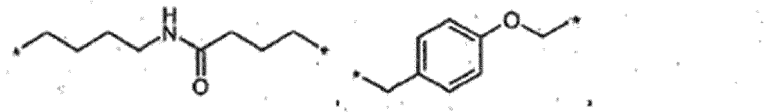 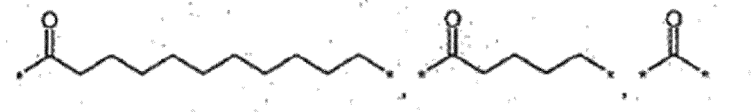 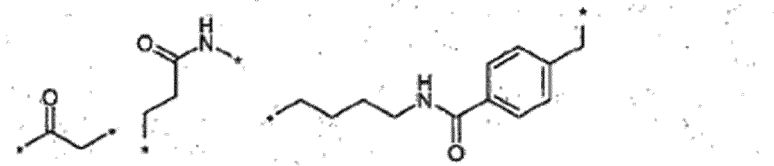 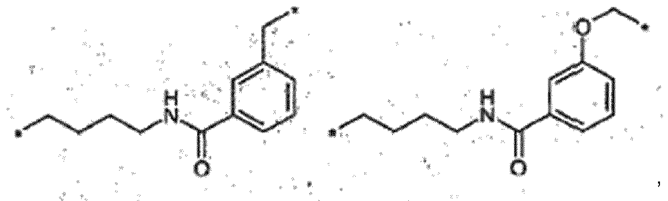 und 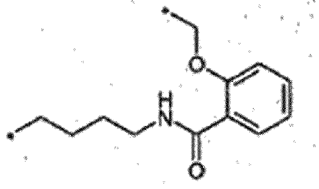 bestehenden Gruppe ausgewählt sind.

13. Verfahren nach einem der Ansprüche 1 bis 12, wobei die die beiden Polypeptide im Endprodukt verknüpfende Gruppierung eine 1,2,3-Triazolgruppierung umfasst.

## Revendications

1. Procédé de couplage de deux polypeptides différents conjointement à leurs extrémités C-terminales respectives comprenant
(a) une modification catalysée par une enzyme des extrémités C-terminales des polypeptides à coupler par laquelle un groupe chimique comprenant un groupe réactif W est introduit à l'extrémité C-terminale du premier polypeptide à coupler, et un groupe chimique comprenant le groupe réactif Z est introduit à l'extrémité C-terminale du deuxième polypeptide à coupler
(b) la réaction du groupe réactif Z avec un groupe réactif Y d'une molécule d'espaceur pour former un groupe chimique X est la réaction du groupe réactif W avec un groupe réactif V de la molécule d'espaceur pour former un groupe chimique U, l'espaceur entre les deux polypeptides comprend au moins un diradical d'éthylène glycol, X et U étant indépendamment choisis dans le groupe constitué de diradicaux d'oxime et de triazole où V, W, Y et Z sont indépendamment choisis dans le groupe constitué d'alcoxylamino, oxo, azido et alcynyle.

2. Procédé selon la revendication 1, dans lequel l'enzyme est la carboxypeptidase Y ou un variant ou un fragment de celle-ci, ledit variant ou fragment conservant la capacité à catalysée une réaction, par laquelle l'acide aminé C-terminal d'une protéine. ou un peptide est remplacé par un fragment chimique différent.

3. Procédé selon la revendication 2, dans lequel l'enzyme est la carboxypeptidase Y.

4. Procédé selon l'une quelconque des revendications 1 à 3, dans lequel au moins un des polypeptides est l'hormone de croissance humaine.

5. Procédé selon l'une quelconque des revendications 1 à 4, dans lequel au moins un des polypeptides est un dérivé d'hormone de croissance humaine.

6. Procédé selon l'une quelconque des revendications 1 à 5, dans lequel au moins un des polypeptides est un variant d'hormone de croissance humaine ou un dérivé d'un variant d'hormone de croissance humaine.

7. Procédé selon l'une quelconque des revendications 1 à 6, dans lequel au moins un des polypeptides est hGH-Leu-Ala.

8. Procédé selon l'une quelconque des revendications 1 à 7, dans lequel le groupe chimique Z est introduit via un lieur A.

9. Procédé selon la revendication 8, dans lequel le lieur A est choisi dans le groupe constitué de biradicaux d'alcane en C₁₋₁₀, alcène en C₂₋₁₀, alcyne en C₂₋₁₀, hétéroalcane en C₁₋₁₀, hétéroalcène en C₂₋₁₀, hétéroalcyne en C₂₋₁₀ dont l'un quelconque peut être substitué par oxo, dans lequel un ou plusieurs biradicaux de composé aromatique homocyclique ou biradicaux de composé aromatique hétérocyclique peuvent être insérés.

10. Procédé selon l'une quelconque des revendications 1 à 9, dans lequel le groupe chimique W est introduit via un lieur B.

11. Procédé selon la revendication 10, dans lequel le lieur B est choisi dans le groupe constitué de biradicaux d'alcane en C₁₋₁₀, alcène en C₂₋₁₀, alcyne en C₂₋₁₀, hétéroalcane en C₁₋₁₀, hétéroalcène en C₂₋₁₀, hétéroalcyne en C₂₋₁₀ linéaires, ramifiés et/ou cycliques, qui est facultativement substitué par oxo, dans lequel un ou plusieurs biradicaux de composé aromatique homocyclique ou biradicaux de composé aromatique hétérocyclique peuvent être insérés.

12. Procédé selon l'une quelconque des revendications 8 à 11, dans lequel le lieur A et le lieur B sont indépendamment choisis dans le groupe constitué de 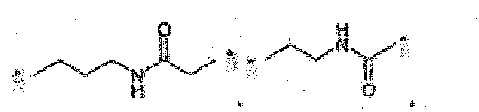 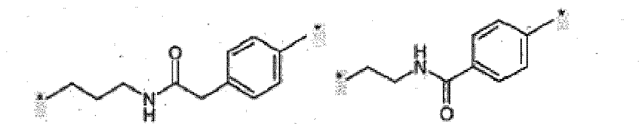 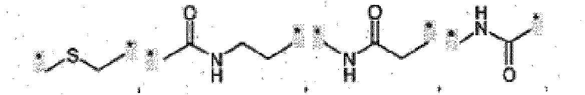 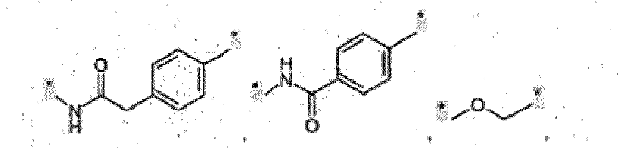 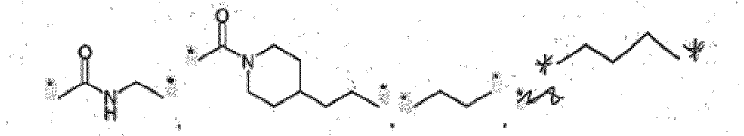 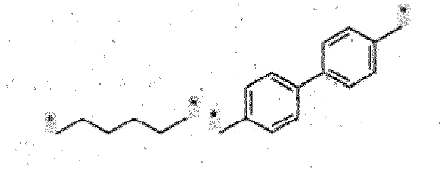 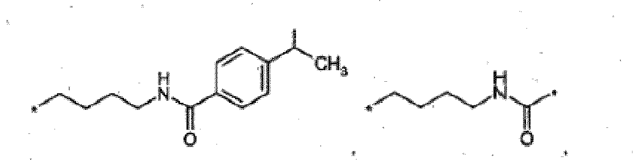 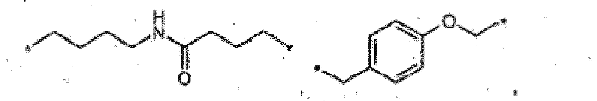 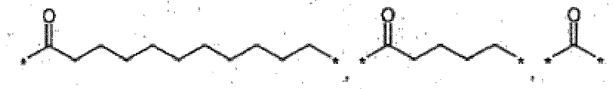 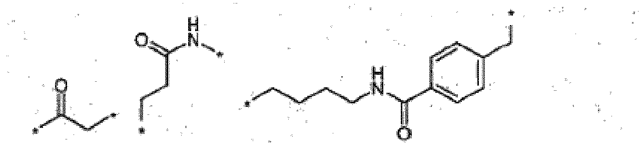 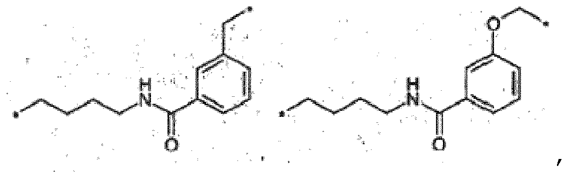 et

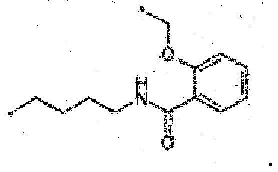

13. Procédé selon l'une quelconque des revendications 1 à 12, dans lequel le fragment reliant les deux polypeptides dans le produit final comprend un fragment 1,2,3-triazole.
